# EUROPEAN PATENT SPECIFICATION

(11) **EP 2 305 214 B1**
(45) Date of publication and mention of the grant of the patent: **03.01.2018**
(21) Application number: 09758388.4
(22) Date of filing: 04.06.2009
(51) Int. Cl.: A61K 9/10, A61K 9/127, A61K 9/19, A61K 9/51, A61K 47/34, A61K 47/42, A61K 49/00, A61K 51/06, A61K 51/12, A61P 35/00

(54) **NOVEL MOLECULAR ASSEMBLY, MOLECULAR PROBE FOR MOLECULAR IMAGING AND MOLECULAR PROBE FOR DRUG DELIVERY SYSTEM USING THE SAME, AND MOLECULAR IMAGING SYSTEM AND DRUG DELIVERY SYSTEM**
NEUE MOLEKULARE ANORDNUNG, MOLEKULARE SONDE ZUR MOLEKULAREN BILDGEBUNG UND MOLEKULARE SONDE FÜR EIN ARZNEIABGABESYSTEM DAMIT SOWIE MOLEKULARES BILDGEBUNGSSYSTEM UND ARZNEIABGABESYSTEM
NOUVEL ENSEMBLE MOLECULAIRE, SONDE MOLECULAIRE D IMAGERIE MOLECULAIRE ET SONDE MOLECULAIRE DE SYSTEME D ADMINISTRATION DE MEDICAMENT, SYSTEME D IMAGERIE MOLECULAIRE ET SYSTEME D ADMINISTRATION DE MEDICAMENT ASSOCIES

(30) Priority: 05.06.2008 JP 2008148521; 23.02.2009 JP 2009038871
(43) Date of publication of application: 06.04.2011
(73) Proprietor: Shimadzu Corporation, Kyoto 604-8511 (JP)
(72) Inventor: HARA, Isao, Kyoto-shi Kyoto 604-8511 (JP); YAMAHARA, Ryo, Kyoto-shi Kyoto 604-8511 (JP); OZEKI, Eiichi, Kyoto-shi Kyoto 604-8511 (JP); TAKEUCHI, Eri, Kyoto-shi Kyoto 604-8511 (JP); KIMURA, Shunsaku, Kyoto-shi Kyoto 615-8530 (JP); KONDOH, Shinae, Kyoto-shi Kyoto 606-8507 (JP); MAKINO, Akira, Kyoto-shi, Kyoto 615-8530 (JP); YAMAMOTO, Fumihiko, Kyoto-shi Kyoto 606-8507 (JP)
(74) Representative: Müller-Boré & Partner Patentanwälte PartG mbB
(86) International application number: PCT/JP2009/060253
(87) International publication number: WO 2009/148121

(56) References cited:
- JP-A- 2008 024 816
- US-A1- 2008 019 908
- AKIRA MAKINO ET AL: "Preparation of Novel Polymer Assemblies, Lactosome, Composed of Poly(L-lactic acid) and Poly(sarcosine)", CHEMISTRY LETTERS, CHEMICAL SOCIETY OF JAPAN, JAPAN, vol. 36, no. 10, 1 January 2007 (2007-01-01), pages 1220-1221, XP008162243, ISSN: 0366-7022, DOI: 10.1246/CL.2007.1220 [retrieved on 2007-09-08]
- HIROKI TANISAKA ET AL: "Near-Infrared Fluorescent Labeled Peptosome for Application to Cancer Imaging", BIOCONJUGATE CHEMISTRY, vol. 19, no. 1, 1 January 2008 (2008-01-01), pages 109-117, XP055047210, ISSN: 1043-1802, DOI: 10.1021/bc7001665
- TATSUYA KANZAKI ET AL.: 'Atarashii Nano-Carrier 'Peptosome' Jisedai DDS eno Kitai' KAGAKU TO SEIBUTSU vol. 45, no. 11, 2007, pages 779 - 784, XP008140608
- 'Abstracts of Annual Meeting of Pharmaceutical Society of Japan, 2007', vol. 127TH, article MASANAO SASATSU ET AL.: 'MeO-PEG amine-PLA block copolymer / PLA o Kizai to shita Nanoparticle -Ryushi Tokusei to Tainai Bunpu', page 125, XP008142938
- MASANAO SASATSU ET AL.: 'Hyoshikitai PLA-Pyrene no Gosei to PLA Yudotai Nanoparticle no Ryushi Tokusei' JOURNAL OF PHARMACEUTICAL SCIENCE AND TECHNOLOGY vol. 68, no. SUPP., April 2004, JAPAN, page 281, XP008142940
- NORASED N ET AL.: 'Multifunctional polymeric micelles as cancer- targeted, MRI-ultrasensitive drug delivery system' NANO LETT vol. 6, no. 11, 2006, pages 2427 - 2430, XP002540355
- 'Microfluidic system for studying the interaction of nanoparticles and microparticles with cells' ANAL CHEM vol. 77, no. 17, 2005, pages 5453 - 5459, XP008140565
- SHOUPING X ET AL.: 'Aggregates of amphiphilic fluorinated copolymers and their encapsulating and unloading homopolymer behaviors' J POLYM SCI PART B vol. 46, no. 11, 01 June 2008, pages 1000 - 1006, XP008140571
- RUXANDRA G ET AL.: 'Polyoxyethylene-coated nanospheres: effect of coating on zeta potential and phagocytosis' POLYM INT vol. 48, no. 4, 1999, pages 251 - 256, XP008140569
- SANG C L ET AL.: 'Synthesis and micellar characterization of amphiphilic diblock copolymers based on poly (2-ethyl-2-oxazoline) and aliphatic polyesters' MACROMOLECULES vol. 32, no. 6, 1999, pages 1847 - 1852, XP000823806

## Description

The present invention relates to a novel particulate molecular assembly made of a biocompatible amphiphilic substance, a molecular probe for molecular imaging comprising the assembly, a molecular probe for drug delivery comprising the assembly, and a method for preparing a particulate molecular assembly.

As described in JP-A-2005-172522 (Patent Document 1), in recent years, there has been a growing interest in nanotechnology, and new functional materials utilizing the characteristics inherent in nanosized substances have been developed. These new functional materials can be used in various fields such as energy, electronics, and medical and pharmaceutical fields. Among such various fields, nanotechnology has been attracting attention in detection of substances in biological samples and in-vivo imaging. Particularly, in medical and pharmaceutical fields, liposomes, which are nanoparticles composed of phospholipid, are used as carriers for drug delivery system (DDS).

In medical and pharmaceutical fields, as described in JP-A-2005-220045 (Patent Document 2), it is desired that changes in the form and function of organs or tissues caused by diseases in a living body are speedily and accurately detected by a simple method at the early stage of the diseases in the diagnosis and treatment of the diseases. Particularly, in order to early diagnose and treat cancer, it is essentially necessary to determine a small lesion site and to determine the size of the lesion site at an early stage in carcinogenesis . Examples of a method for early diagnosis include endoscopic biopsy and diagnostic imaging such as radiography, MRI, and ultrasonography. In a case where a radioactive indicator is used, the lifetime of the indicator is limited due to its half-life. In addition, a diagnostic apparatus is very expensive.

On the other hand, diagnostic imaging using a fluorescence indicator or a near-infrared indicator is also known. In the case of such diagnostic imaging, the lifetime of an indicator itself is not strictly limited, and a measuring apparatus for diagnose is not very expensive as compared to the apparatus using radiation. In addition, such optical diagnosis is noninvasive to a living body.

For example, autofluorescence observation via endoscope is in practical use, which utilizes the fact that the autofluorescence of tumor cells is weaker than that of normal cells (excitation: 450 nm, emission: 520 nm). In the case of using small animals, chemiluminescent diagnostic imaging of cancers is also used. Chemiluminescence is a phenomenon in which a luminescent substrate (luciferin) is oxidized by an enzyme (luciferase) to an unstable peroxide and then light is emitted in the process of decomposition of the peroxide.

Further, near-infrared fluorescence imaging has been also attracting attention, which is a technique for imaging a tumor site by allowing a near-infrared fluorochrome to accumulate in the tumor site. In the case of near-infrared fluorescence imaging, a compound that can emit fluorescence in the near-infrared region by irradiation with excitation light is administered as a contrast agent to a living body, and then the living body is externally irradiated with excitation light having a near-infrared wavelength to detect fluorescence emitted from the fluorescent contrast agent accumulating in a tumor site to determine a lesion site. As such a contrast agent, a nanoparticle such as a liposome having an indocyanine green derivative encapsulated therein has been reported (see JP-A-2005-220045 (Patent Document 2)).

On the other hand, peptide-type nanoparticles having higher biocompatibility have also been known (see Journal of Controlled Release 50 (1998) 205-214 (Non-Patent Document 1), Journal of Controlled Release 51 (1998) 241-248 (Non-Patent Document 2), Journal of Colloid Interface Science 280 (2004) 506-510 (Non-Patent Document 3), and Journal of American Chemical Society 2005, 127, 12423-12428 (Non-Patent Document 4)).

JP-A-2008-024816 (Patent Document 3) discloses a peptide-type nanoparticle using an amphiphilic block polymer having poly glutamic acid methyl ester as a hydrophobic block. This publication describes that the particle size of the nanoparticles can be controlled by changing the chain length of the amphiphilic block polymer and that accumulation of the nanoparticles in cancer tissue has been observed.

Further, Chemistry Letters, vol. 36, no. 10, 2007, pp. 1220-1221 (Non Patent Document 5) discloses that an amphiphilic block polymer composed of a polylactic acid chain and a polysarcosine chain is synthesized, and a molecular assembly having a particle size of 20 to 200 nm, which is applicable to a nanocarrier for DDS, is prepared by self-assembling of the amphiphilic block polymer.

It is to be noted that the above-described method for effective accumulation of a substance having a signal agent or a drug in cancer tissue utilizes EPR (enhanced permeability and retention) effect.

Cells proliferate faster in cancer tissue than in normal tissue, and therefore cancer tissue induces the neovessels to obtain oxygen and energy required for cell proliferation. It is known that these neovessels are fragile and therefore molecules leak from the vessels even when the molecules are somewhat large. Further, the excretory system of a substance in cancer tissue is underdeveloped, and therefore molecules leaking from the vessels are retained in cancer tissue for a certain period of time. This phenomenon is known as EPR effect.
Patent Document 1: JP-A-2005-172522
Patent Document 2: JP-A-2005-220045
Patent Document 3: JP-A-2008-024816
Non-Patent Document 1: Journal of Controlled Release, vol. 50, 1998, pp. 205-214
Non-Patent Document 2: Journal of Controlled Release, vol. 51, 1998, pp. 241-248
Non-Patent Document 3: Journal of Colloid Interface Science, vol. 280, 2004, pp. 506-510
Non-Patent Document 4 : Journal of American Chemical Society, 2005, vol. 127, pp. 12423-12428
Non-Patent Document 5: Chemistry Letters, vol. 36, no. 10, 2007, pp. 1220-1221
US 2008/0019908 A1 describes a novel amphiphilic substance, and a drug delivery system and a molecular imaging system using the same.

Endoscopic biopsy and diagnostic imaging such as radiography, MRI, and ultrasonography have their respective advantages, but they are invasive methods imposing psychological pressure, pain or suffering, and exposure to radiation on subjects. Further, in a case where a radioactive indicator is used, the lifetime of the indicator is limited due to its half-life. In addition, in this case, a diagnostic apparatus is very expensive.

On the other hand, as a noninvasive method, diagnostic imaging of cancers using fluorescence or chemiluminescence is known. However, especially the method using chemiluminescence needs genetic modification, and therefore cannot be applied to diagnosis in humans from the viewpoint of safety.

A liposome using near-infrared light, such as a liposome in the method described in JP-A-2005-220045 (Patent Document 2), is recognized by immune system cells, such as macrophages, in blood and eliminated, and is therefore captured by a reticuloendothelial system (RES), such as in liver and spleen, containing a large amount of macrophage-like cells. For this reason, such a liposome is not favorable for retentivity in blood. In order to improve the retentivity of a liposome, a liposome coated with polyethylene glycol (PEG) has also been reported (see U.S. Patent No. 5013556). It is believed that the PEG of the liposome has the function of improving the hydrophilicity of a liposome surface to prevent the liposome from being recognized as foreign matter by an immune system such as RES. However, there is no detailed report about the safety of such a PEG-coated liposome and its metabolite in a living body. In addition, there is a report that a commercially-available PEG-coated liposome, that is, Doxil® causes anaphylactoid reaction relatively frequently when administered to a human (see JOURNAL OF LIPOSOME RESEARCH, 10(4), 467-484 (2000)). Therefore, such a PEG-coated liposome has a problem in safety.

Further, such a liposome is limited in the composition of a hydrophobic part, and therefore also has a problem in that the control of its particle size is limited.

A nanoparticle described in JP-A-2008-024816 (Patent Document 3) uses a peptide-type amphiphilic block polymer (peptosome). Unlike the case of liposomes, a peptide-type amphiphilic block polymer is not dissolved in a solvent having low-boiling point such as chloroform, in production of nanoparticle. Therefore, in this case, nanoparticles need to be produced by a method comprising dissolving a peptide-type amphiphilic block polymer in trifluoroethanol (TFE) and then dispersing in water (i.e., by an injection method) . However, TFE itself is toxic, and therefore TFE used in an injection method needs to be strictly removed by gel filtration to administer nanoparticles prepared by such an injection method to a living body.

Further, the publication also describes that the peptide-type nanoparticles accumulate in cancer tissue by EPR (enhanced permeability and retention) effect. However, this evaluation has been made by fluorescence observation of only a region around cancer tissue. For example, although not described in the publication, when a mouse is observed from its abdomen side, accumulation of a drug in living tissue, such as liver and spleen, other than cancer is also observed. Therefore, when the peptide-type nanoparticles are used in fluorescence imaging, it is difficult to perform imaging of cancer tissue near the above-mentioned tissue, and when used in DDS, the delivery rate of a drug to a diseased site is lowered.

Further, the publication also describes that the particle size of the nanoparticles can be controlled by changing the chain length of the amphiphilic block polymer. However, in fact, the publication merely demonstrates that two types of nanoparticles mutually different in particle size can be obtained from two types of amphiphilic block polymers which are the same in block chain component (structural unit) but mutually different in chain length, and that several types of nanoparticles mutually different in particle size can be obtained from several types of amphiphilic block polymers mutually different in both block chain component (structural unit) and chain length. That is, the publication neither discloses nor suggests the correspondence relation between the physical amount of the amphiphilic block polymer and the particle size of nanoparticles. Therefore, the particle size of the nanoparticles cannot be continuously controlled by the teaching of the publication.

Chemistry Letters, vol. 36, no. 10, 2007, pp. 1220-1221 (Non-Patent Document 5) suggests that a polylactic acid chain-containing molecular assembly is applicable to a nanocarrier for DDS. However, there is no description about the administration of the molecular assemblies to a living body, and of course, there is no description about the dynamic behavior of the molecular assemblies in a living body.

Further, as in the case of the above-described publication 2, there is no description about the continuous control of the particle size of the molecular assemblies.

It is an object of the present invention to provide a molecular assembly which is less likely to accumulate in tissue other than cancer tissue, is highly safe for a living body, and can be prepared by a simple and safe method and whose particle size can be easily controlled. It is also an object of the present invention to provide a molecular probe useful for molecular imaging, and a molecular probe useful for drug delivery.

It is another object of the present invention to provide a method for preparing molecular assemblies, by which the particle size of molecular assemblies having a signal group or a drug can be arbitrarily controlled in order to allow the molecular assemblies to effectively accumulate in cancer tissue by utilizing EPR effect.

The present inventors have intensively studied, and as a result, have found that the above object of the present invention can be achieved by forming a particulate molecular assembly from a polylactic acid-based amphiphilic block polymer and a polylactic acid-based labeled polymer. This finding has led to the completion of the present invention.

The present inventors have further intensively studied, and as a result, have found that the above another object of the present invention can be achieved by preparing molecular assemblies by adding polylactic acids different in optical purity to a polylactic acid-based amphiphilic block polymer in various ratios. This finding has led to the completion of the present invention.

The subject matter of the present invention is characterized in the claims.

The molecular assembly according to the present invention is a particulate molecular assembly.

The following (1) to (24) relate to a molecular assembly.

The term "molecular assembly" basically refers to a structure formed by aggregation or self-assembling orientation and association of the molecules of an amphiphilic block polymer.

It is to be noted that a molecular assembly comprising an amphiphilic block polymer A1 containing a hydrophobic block chain basically composed of lactic acid units is sometimes referred to as a "lactosome". Therefore, the "molecular assembly" in the following (1) to (24) is also regarded as a lactosome in this specification.

### Al/B-Based Lactosome:

The following (1) is directed to a molecular assembly comprising an amphiphilic block polymer A1 and a labeled polymer B. In this specification, such a molecular assembly is sometimes referred to as an "Al/B-based lactosome".
(1) A molecular assembly comprising:
   an amphiphilic block polymer A1 comprising a hydrophilic block chain having 20 or more sarcosine units and a hydrophobic block chain having 10 or more lactic acid units ; and
   a labeled polymer B comprising at least 10 or more lactic acid units and a labeling group.

In the above (1), the term "sarcosine" refers to N-methylglycin.

The property, "hydrophilicity" of the hydrophilic block chain means that the hydrophilic block chain is relatively more hydrophilic than the hydrophobic block chain having 10 or more lactic acid units.

The property, "hydrophobicity" of the hydrophobic block chain means that the hydrophobic bock chain is relatively more hydrophobic than the hydrophilic block chain having 20 or more sarcosine units.

In the above (1), the "labeled polymer B" may be one or more labeled polymers selected from the group consisting of a labeled polylactic acid described in the following (2) and a labeled amphiphilic block polymer described in the following (3).
(2) The molecular assembly according to the above (1), wherein the labeled polymer B is a labeled polylactic acid comprising 10 or more continuous lactic acid units and a labeling group as constituent components.
(3) The molecular assembly according to the above (1), wherein the labeled polymer B is a labeled amphiphilic block polymer comprising a hydrophilic block chain, a hydrophobic block chain having 10 or more lactic acid units, and a labeling group.
(4) The molecular assembly according to any one of the above (1) to (3), which is prepared by a preparation method comprising the steps of:
   preparing a solution, in a container, containing the amphiphilic block polymer A1 and the labeled polymer B in an organic solvent;
   removing the organic solvent from the solution to obtain a film containing the amphiphilic block polymer A1 and the labeled polymer B on an inner wall of the container; and
   adding water or an aqueous solution into the container and ultrasonic treatment is performed to convert the film into particulate molecular assembly to obtain a dispersion liquid of the molecular assembly.
(5) The molecular assembly according to the above (4), wherein the preparation method further comprises, after the step of obtaining the dispersion liquid of the molecular assembly, the step in which the dispersion liquid of the molecular assembly is subjected to freeze-drying treatment.

### A1/A2-Based Lactosome:

The following (6) is directed to a molecular assembly comprising an amphiphilic block polymer A1 and a hydrophobic polymer A2. The molecular assembly described in the following (6) also comprises an amphiphilic block polymer A1 containing a hydrophobic block chain basically composed of lactic acid units, and is therefore regarded as a lactosome. In this specification, this type of lactosome is sometimes particularly referred to as an "A1/A2-based lactosome". Further, as will be described later, from the viewpoint that the hydrophobic polymer A2 may be basically composed of lactic acid units, one embodiment of such an A1/A2-based lactosome is sometimes particularly referred to as a "polylactic acid-blended lactosome".
(6) A molecular assembly comprising:
   an amphiphilic block polymer A1 comprising a hydrophilic block chain having 20 or more sarcosine units and a hydrophobic block chain having 10 or more lactic acid units; and
   a hydrophobic polymer A2 having 10 or more lactic acid units.

In the above (6), the term "sarcosine" refers to N-methylglycin.

The property, "hydrophilicity" of the hydrophilic block chain means that the hydrophilic block chain is relatively more hydrophilic than the hydrophobic block chain having 10 or more lactic acid units.

The property, "hydrophobicity" of the hydrophobic block chain means that the hydrophobic bock chain is relatively more hydrophobic than the hydrophilic block chain having 20 or more sarcosine units.

The property, "hydrophobicity" of the "hydrophobic polymer A2" means that the hydrophobic polymer A2 is relatively more hydrophobic than the hydrophilic block of the amphiphilic block polymer A1.
(7) The molecular assembly according to the above (6), wherein the hydrophobic polymer A2 is selected from the group consisting of:
   a hydrophobic polymer whose 10 or more lactic acid units are composed of L-lactic acid units;
   a hydrophobic polymer whose 10 or more lactic acid units are composed of D-lactic acid units; and
   a hydrophobic polymer whose 10 or more lactic acid units are composed of L-lactic acid units and D-lactic acid units.
(8) The molecular assembly according to the above (6) or (7), wherein the amphiphilic block polymer A1 and the hydrophobic polymer A2 are contained in a molar ratio of 10 : 1 to 1:10.
(9) The molecular assembly according to any one of the above (6) to (8), which is prepared by a preparation method comprising the steps of:
   preparing a solution, in a container, containing the amphiphilic block polymer A1 and the hydrophobic polymer A2 in an organic solvent;
   removing the organic solvent from the solution to obtain a film containing the amphiphilic block polymer A1 and the hydrophobic polymer A2 on an inner wall of the container; and
   adding water or an aqueous solution into the container and ultrasonic treatment is performed to convert the film into particulate molecular assembly to obtain a dispersion liquid of the molecular assembly.
(10) The molecular assembly according to the above (9), wherein the preparation method further comprises, after the step of obtaining the dispersion liquid of the molecular assembly, the step in which the dispersion liquid of the molecular assembly is subjected to freeze-drying treatment.

### A1/A2/B-Based Lactosome:

The following (11) is directed to a molecular assembly comprising an amphiphilic block polymer A1, a hydrophobic polymer A2, and a labeled polymer B. In this specification, such a molecular assembly is sometimes referred to as an A1/A2/B-based lactosome.
(11) The molecular assembly according to any one of the above (6) to (8), further comprising a labeled polymer B comprising at least 10 or more lactic acid units and a labeling group.

In the above (11), the "labeled polymer B" may be one or more labeled polymers selected from the group consisting of a labeled polylactic acid described in the following (12) and a labeled amphiphilic block polymer described in the following (13).

(12) The molecular assembly according to the above (11), wherein the labeled polymer B is a labeled polylactic acid comprising 10 or more continuous lactic acid units and a labeling group as constituent components.
(13) The molecular assembly according to the above (11), wherein the labeled polymer B is a labeled amphiphilic block polymer comprising a hydrophilic block chain, a hydrophobic block chain having 10 or more lactic acid units, and a labeling group.

(14) The molecular assembly according to any one of the above (11) to (13), which is prepared by a preparation method comprising the steps of:
   preparing a solution, in a container, containing the amphiphilic block polymer A1, the hydrophobic polymer A2, and the labeled polymer B in an organic solvent;
   removing the organic solvent from the solution to obtain a film containing the amphiphilic block polymer A1, the hydrophobic polymer A2, and the labeled polymer B on an inner wall of the container; and
   adding water or an aqueous solution into the container and ultrasonic treatment is performed to convert the film into particulate molecular assembly to obtain a dispersion liquid of the molecular assembly.
(15) The molecular assembly according to the above (14), wherein the preparation method further comprises, after the step of obtaining the dispersion liquid of the molecular assembly, the step in which the dispersion liquid of the molecular assembly is subjected to freeze-drying treatment.

In this specification, preparation method of the molecular assembly described in the above (4), (5), (9), (10), (14), and (15) is sometimes referred to as a "film method".

(16) The molecular assembly according to any one of the above (1) to (15), which is in the form of a micelle or a vesicle.

In the above (16), there is a case where a micelle is particularly useful for a molecular imaging system of cancer. For use as a drug delivery system, the molecular assembly may be in the form of either a micelle or a vesicle.
(17) The molecular assembly according to any one of the above (1) to (5) and (11) to (16), wherein the labeling group of the labeled polymer B is selected from the group consisting of a signal group and a ligand.

In the above (17), the "signal group" refers to a group having a property detectable for imaging, and includes fluorescent groups, radioactive element-containing groups, magnetic groups.

In the above (17), the "ligand" includes ligands for allowing the molecular assembly to specifically bind to a target site in an object when the molecular assembly is administered to the object and ligands for coordinating to a molecule or an atom of a drug or a signal agent to be delivered to a target site in an object when the molecular assembly is administered to the object.
(18) The molecular assembly according to the above (17), wherein the signal group is a near-infrared fluorescent group.

The molecular assembly according to the above (18) is useful as a molecular probe for fluorescence imaging.
(19) The molecular assembly according to the above (17), wherein the signal group is a radioactive element-containing group.
The molecular assembly according to the above (19) is useful as a molecular probe for positron emission tomography (PET) .

The following (20) to (23) are directed to the molecular assembly according to the above (6) comprising an amphiphilic block polymer A1 and a hydrophobic polymer A2, encapsulating a labeling agent therein.
(20) The molecular assembly according to any one of the above (6) to (10), further comprising a labeling agent.

The molecular assembly according to the above (20) is useful as a molecular probe for molecular imaging.

In a case where the molecular assembly according to the above (20) is in the form of a micelle, the labeling agent can be held inside the micelle. The labeling agent can be easily encapsulated in the molecular assembly in the form of a micelle comprising an amphiphilic block polymer A1 and a hydrophobic polymer A2, because the volume of a hydrophobic core of the micelle is increased by the hydrophobic polymer A2.

Also in a case where the molecular assembly according to the above (20) is in the form of a vesicle, the labeling agent can be held inside the vesicle.

(21) The molecular assembly according to the above (20), wherein the labeling agent is selected from the group consisting of a signal substance and a ligand substance.
(22) The molecular assembly according to the above (21), wherein the signal substance is a near-infrared fluorescent substance.
(23) The molecular assembly according to the above (21), wherein the signal substance is a radioactive element-containing substance.

The following (24) is directed to the molecular assembly according to any one of the above (1) to (23), holding a drug by encapsulation.
(24) The molecular assembly according to any one of the above (1) to (23), further comprising a drug.

The molecular assembly according to the above (24) is useful as a molecular probe for drug delivery system (DDS) .

In a case where the molecular assembly according to the above (24) is in the form of a vesicle, the drug can be held inside the vesicle.

Also in a case where the molecular assembly according to the above (24) is in the form of a micelle, the drug can be held inside the micelle. Particularly, in a case where the molecular assembly according to any one of the above (6) to (15) is in the form of a micelle, the drug can be easily encapsulated in the micelle, because the volume of a hydrophobic core of the micelle is increased by the hydrophobic polymer A2.

Further, in a case where the molecular assembly according to the above (24) is in the form of a micelle, the drug may be held by the micelle by allowing the constituent polymer itself (i.e., the above-described amphiphilic block polymer A1, hydrophobic polymer A2, and/or labeled polymer B) of the molecular assembly to have the drug. Examples of the molecular assembly whose constituent polymer itself has a drug include one whose constituent polymer is covalently bound to a drug, one whose constituent polymer is covalently bound to a ligand to coordinate a drug molecule to the ligand, and one having a drug molecule encapsulated in the micelle.

The following (25) to (28) relate to a molecular probe using the above molecular assembly.
(25) A molecular probe for molecular imaging comprising the molecular assembly according to any one of the above (1) to (5) and (11) to (24).
(26) The molecular probe for molecular imaging according to the above (25), which is a molecular probe for fluorescence imaging comprising the molecular assembly according to the above (22).
(27) The molecular probe for molecular imaging according to the above (25), which is a molecular probe for positron emission tomography comprising the molecular assembly according to the above (23).
(28) A molecular probe for drug delivery system comprising the molecular assembly according to the above (24) .

The following (29) is directed to a method for controlling a particle size of the molecular assembly according to any one of the above (6) to (14) comprising at least an amphiphilic block polymer A1 and a hydrophobic polymer A2 (i.e., A1/A2-based lactosome or A1/A2/B-based lactosome).
(29) A method for preparing molecular assembly comprising the step of:
   preparing, by using an amphiphilic block polymer A1 comprising a hydrophilic block chain having 20 or more sarcosine units and a hydrophobic block chain having 10 or more lactic acid units and a hydrophobic polymer A2 having 10 or more lactic acid units, molecular assembly comprising the amphiphilic block polymer A1 and the hydrophobic polymer A2; and
   controlling a particle size of the molecular assembly by adjusting an amount of the hydrophobic polymer A2 used with respect to an amount of the amphiphilic block polymer A1 used.

In the above (29), the "particle size" refers to a particle size occurring most frequently in particle size distribution, i.e., a medium particle diameter.

(30) The method for preparing molecular assembly according to the above (29), wherein the hydrophobic polymer A2 is selected from the group consisting of:
a hydrophobic polymer whose 10 or more lactic acid units are composed of L-lactic acid units;
a hydrophobic polymer whose 10 or more lactic acid units are composed of D-lactic acid units; and
a hydrophobic polymer whose 10 or more lactic acid units are composed of L-lactic acid units and D-lactic acid units.

(31) The method for preparing molecular assembly according to the above (29) or (30), wherein the amphiphilic block polymer A1 and the hydrophobic polymer A2 are used in a molar ratio of 10:1 to 1:10.

(32) The method for preparing molecular assembly according to any one of the above (29) to (31), further comprising using a labeled polymer B comprising at least 10 or more lactic acid units and a labeling group to prepare molecular assembly comprising the amphiphilic block polymer A1 and the hydrophobic polymer A2 and, in addition, the labeled polymer B.

The following (33) and (34) relate to a method using the above molecular probe.
(33) A molecular imaging system comprising administrating the molecular probe according to any one of the above (25) to (27) to a living body.

More specifically, the molecular imaging system includes a fluorescence imaging system using the molecular probe according to the above (26) and a positron emission tomography (PET) system using the molecular probe according to the above (27).

(34) A drug delivery system (DDS) comprising administering the molecular probe according to the above (28) to a living body.

According to the present invention, it is possible to provide a molecular assembly which is less likely to accumulate in tissue other than cancer tissue, is highly safe for a living body, and can be prepared by a simple and safe method and whose particle size can be easily controlled. Therefore, according to the present invention, it is possible to provide a molecular probe useful for molecular imaging and to provide a molecular probe useful for drug delivery.

More specifically, the molecular assembly according to the present invention uses a polylactic acid-type labeled polymer to reduce accumulation in tissue other than cancer tissue. This makes it possible to allow the molecular assembly to specifically accumulate in cancer tissue. Further, the molecular assembly according to the present invention is highly safe for a living body, can be easily applied to a molecular probe, can be prepared by a safe method, and has excellent biocompatibility and biodegradability. Further, the shape and size of the molecular assembly itself can be controlled by controlling the amount of lactic acid as a monomer in the process of its preparation.

The molecular assembly according to the present invention selectively accumulates in a cancer and enables imaging in a short period of time, and is therefore particularly useful for imaging of liver cancers and cancers of organs near the liver. Further, the molecular assembly is also useful as a molecular probe for PET or DDS targeting liver cancers or cancers of organs near the liver.

Further, when performing diagnostic imaging using chemiluminescence, the molecular assembly makes it possible to safely perform cancer diagnostic imaging without the need for genetic modification, and is therefore applicable to diagnostic imaging of tumors in human body.

Further, according to the present invention, it is possible to provide a method for preparing molecular assembly capable of arbitrarily controlling the particle size of molecular assembly. This makes it possible to allow molecular assembly having a signal group or a drug to effectively accumulate in cancer tissue by utilizing EPR effect.

### BRIEF DESCRIPTION OF THE DRAWINGS

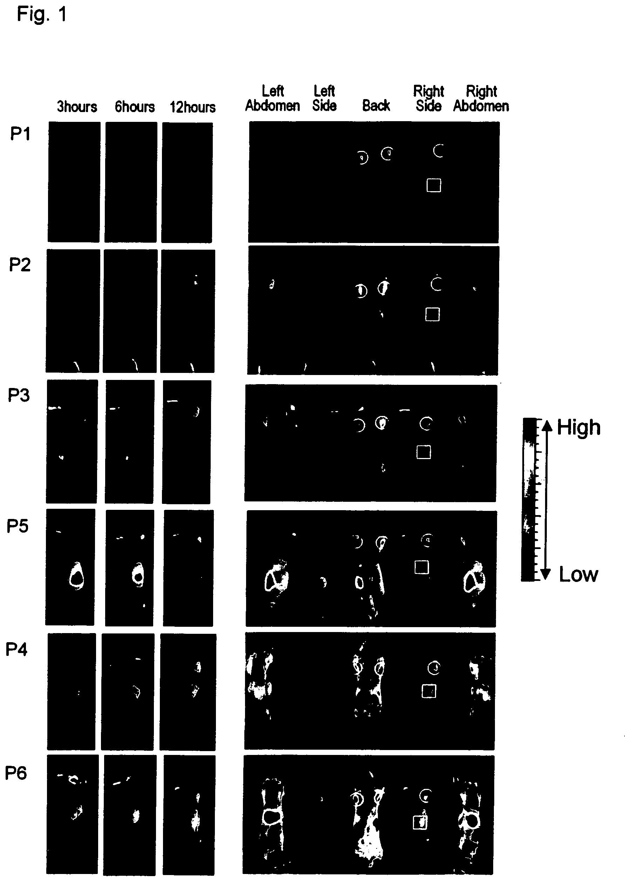

Fig. 1 shows the result of fluorescence imaging test of cancer-bearing mice, and more specifically shows, from above, the result of a fluorescence imaging test (Example 2) using molecular probes P1, P2, and P3 which are A1/B-based lactosomes nanoparticles, the result of a fluorescence imaging test (Comparative Example 4) using molecular probes P5 which is lactosome nanoparticles containing neither A2 nor B, and the result of a fluorescence imaging test (Comparative Example 5) using P4 and P6 which are peptosome nanoparticles, each of which includes, from left, images of a cancer-bearing mouse measured from the direction of its right side of the body after lapses of 3, 6, and 24 hours from tail vein injection of the nanoparticles and images of the mouse measured from 5 directions (i.e., from the directions of its left abdomen, left side of the body, back, right side of the body, and right abdomen) after a lapse of 24 hours from tail vein injection of the nanoparticles.

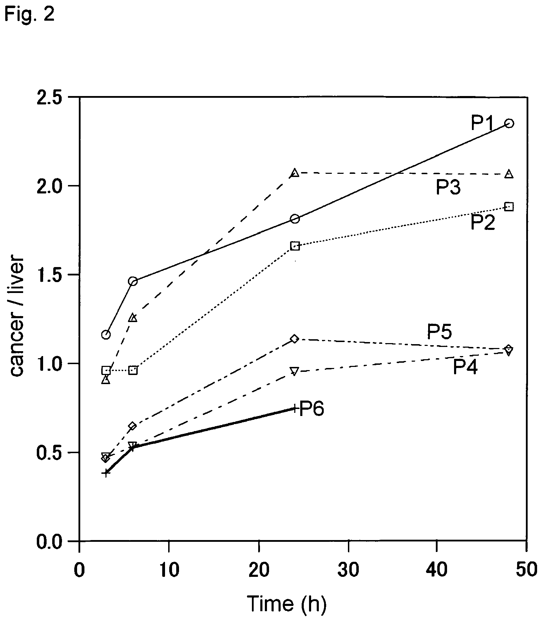

Fig. 2 is a graph showing a comparison of the ratio of fluorescence intensity of molecular probes accumulated in a cancer and liver, more specifically, in the case (Example 2) using the molecular probes P1, P2, and P3 which are A1/B-based lactosome nanoparticles, the case (Comparative Example 4) using that the molecular probes P5 which is a lactosome nanoparticle containing neither A2 nor B, and the case (Comparative Example 5) using P4 and P6 which are the peptosome nanoparticles, wherein the horizontal axis represents the time (h) that has elapsed from the tail vein injection of the nanoparticles and the vertical axis represents the ratio of fluorescence intensity at cancer to fluorescence intensity at liver.

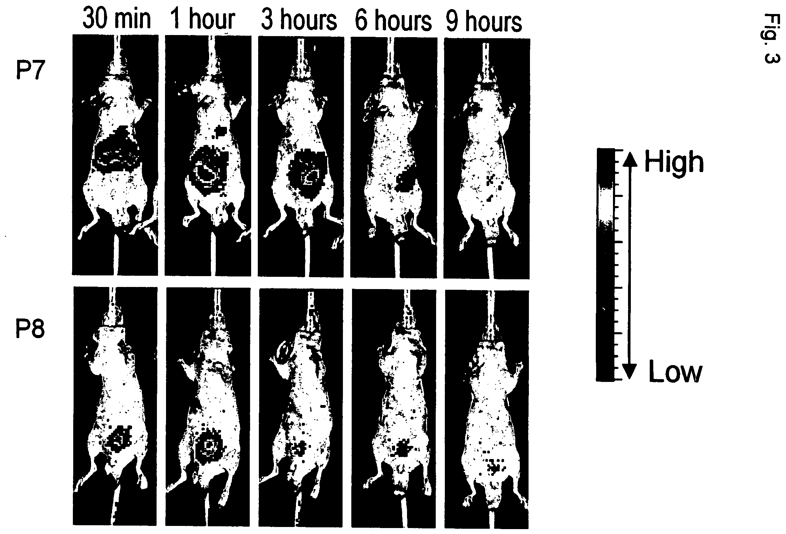

Fig. 3 shows the result of fluorescence imaging test of cancer-bearing mice, and more specifically shows, from above, the result of a fluorescence imaging test (Comparative Example 6) using molecular probes P7 which is a lactosome nanoparticle containing neither A2 nor B and the result of a fluorescence imaging test (Comparative Example 6) using molecular probes P8 which is a lactosome nanoparticle containing neither A2 nor B, each of which includes, from left, images of a cancer-bearing mouse measured from the direction of its abdomen after lapses of 30 minutes and 1, 3, 6, and 9 hours from tail vein injection of the nanoparticles.

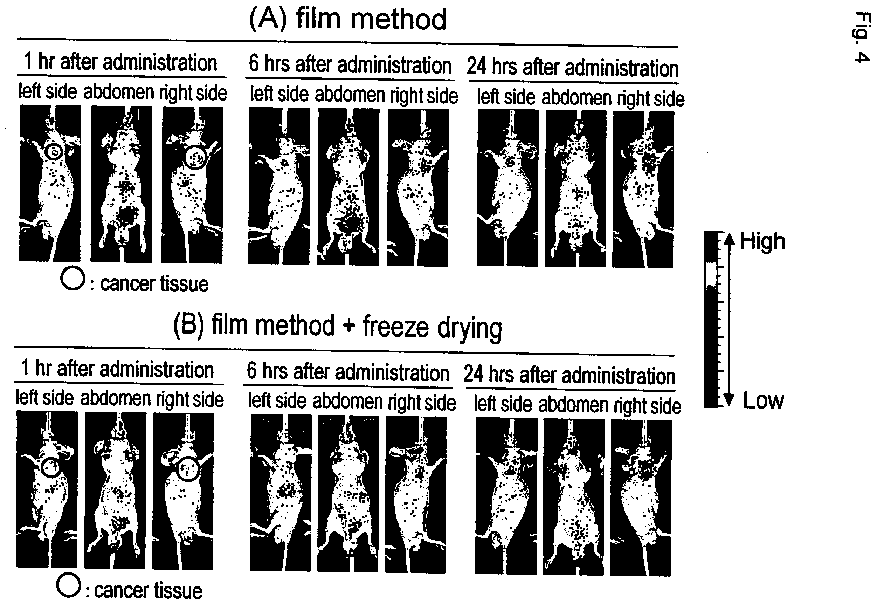

Fig. 4 shows the result of fluorescence imaging test of cancer-bearing mice, and more specifically shows, from above, the result (A) of a fluorescence imaging test (Example 2) using the molecular probes P1 which is a A1/B-based lactosome nanoparticle and the result (B) of a fluorescence imaging test (Example 3) using its freeze-dried product P1(FD), each of which includes, from left, images of a cancer-bearing mouse measured from the directions of its left side of the body, abdomen, and right side of the body after lapses of 1, 6, and 24 hours from tail vein injection of the nanoparticles.

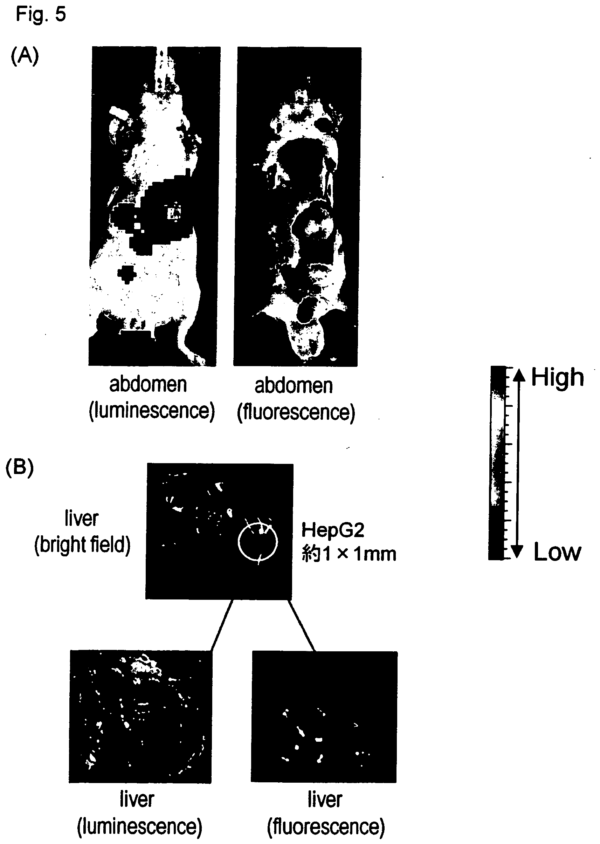

Fig. 5 shows (A) the result of luminescence and fluorescence imaging of the whole body (abdomen) of a cancer-bearing mouse prepared by orthotopic transplantation of human liver cancer cells (HepG2) after a lapse of 48 hours from administration of the molecular probes P2 which is A1/B-based lactosome nanoparticle, and (B) photograph of liver extirpated from the mouse and the result of luminescence and fluorescence imaging of the extirpated liver, obtained in Example 4.

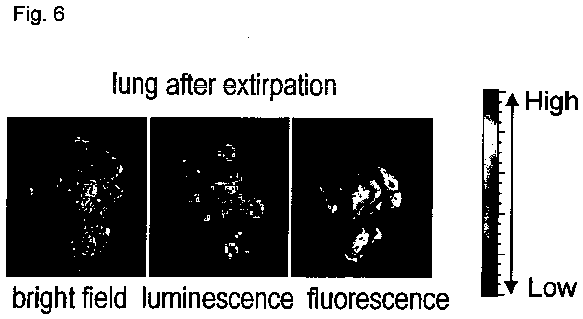

Fig. 6 shows the photograph of lung extirpated from a cancer-bearing mouse prepared by orthotopic transplantation of human lung cancer cells (H441) after a lapse of 48 hours from administration of the molecular probes P2 which is A1/B-based lactosome nanoparticle and the result of luminescence and fluorescence imaging of the extirpated lung, obtained in Example 5.

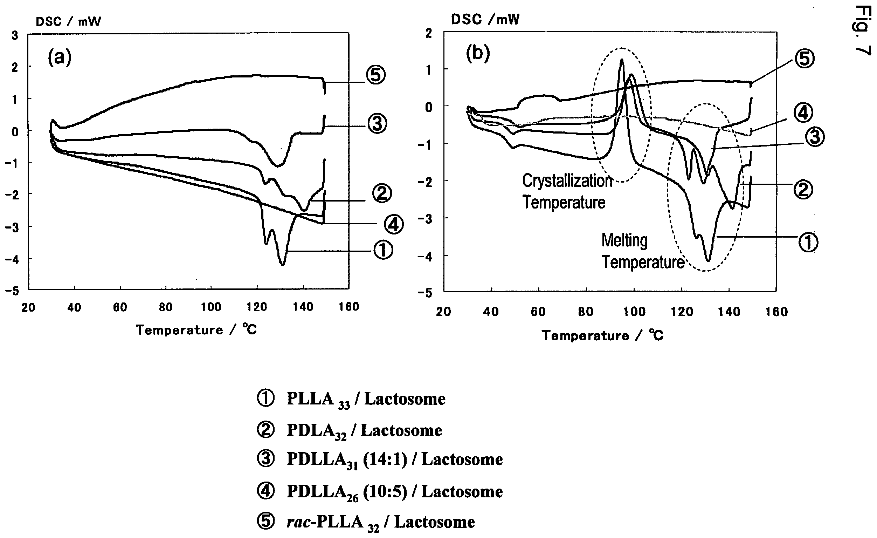

Fig. 7 shows the result of differential scanning calorimetry, obtained in Experimental Example 10, of polylactic acids different in optical purity wherein Fig. 7(a) shows the result of first heating and Fig. 7(b) shows the result of second heating.

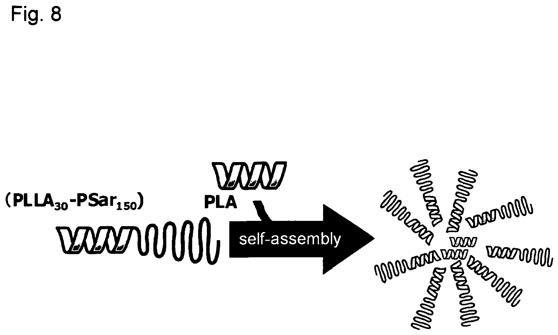

Fig. 8 is a schematic diagram showing that an A1/A2-based lactosome (polylactic acid-blended lactosome) is prepared by blending a polylactic acid A2 (PLA) with a polylactic acid-based amphiphilic polymer A1.

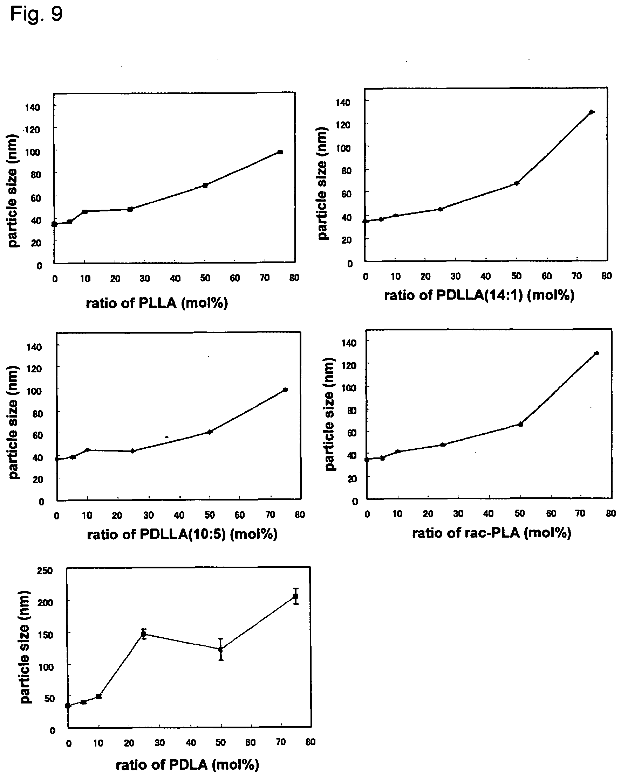

Fig. 9 shows the result of particle size measurement, obtained in Example 6, of five types of Al/A2-based lactosome nanoparticles prepared from a polylactic acid-based amphiphilic polymer A1 and five types of polylactic acids A2 different in optical purity.

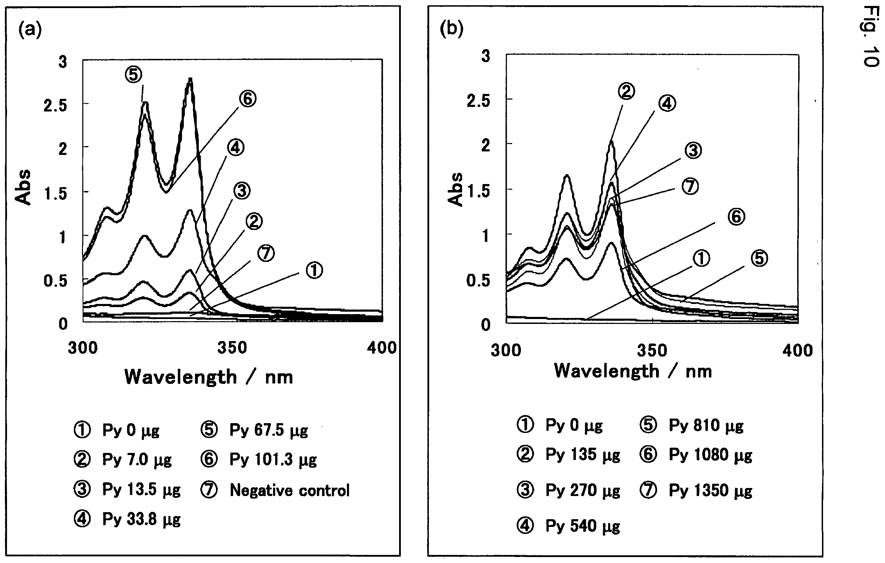

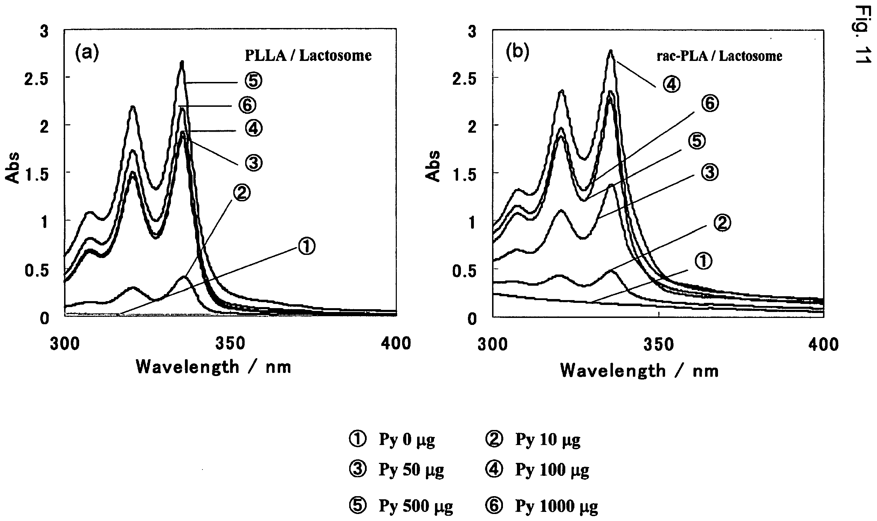

Fig. 10 shows absorption spectra, obtained in Comparative Example 7, of lactosome composed of a polylactic acid-based amphiphilic polymer A1, containing neither A2 nor B and having pyrene encapsulated therein, wherein Fig. 10 (a) shows the absorption spectra when the concentration of pyrene is 0 to 75 mol% and Fig. 10(b) shows the absorption spectra when the concentration of pyrene is 100 to 1000 mol%. Fig. 11 shows absorption spectra, obtained in Example 7, of Al/A2-based lactosomes composed of a polylactic acid-based amphiphilic polymer A1 and a polylactic acid A2 (polylactic acid-blended lactosomes) having pyrene encapsulated therein, wherein Fig. 11(a) shows absorption spectra of A1/A2-based lactosomes (PLLA/lactosomes) composed of a polylactic acid-based amphiphilic polymer A1 (PLLA₃₁-PSar₁₅₀) and a polylactic acid A2 (PLLA) and Fig. 11(b) shows absorption spectra of A1/A2-based lactosomes (rac-PLA/lactosomes) composed of a polylactic acid-based amphiphilic polymer A1 (PLLA₃₁-PSar₁₅₀) and a polylactic acid A2 (rac-PLA).

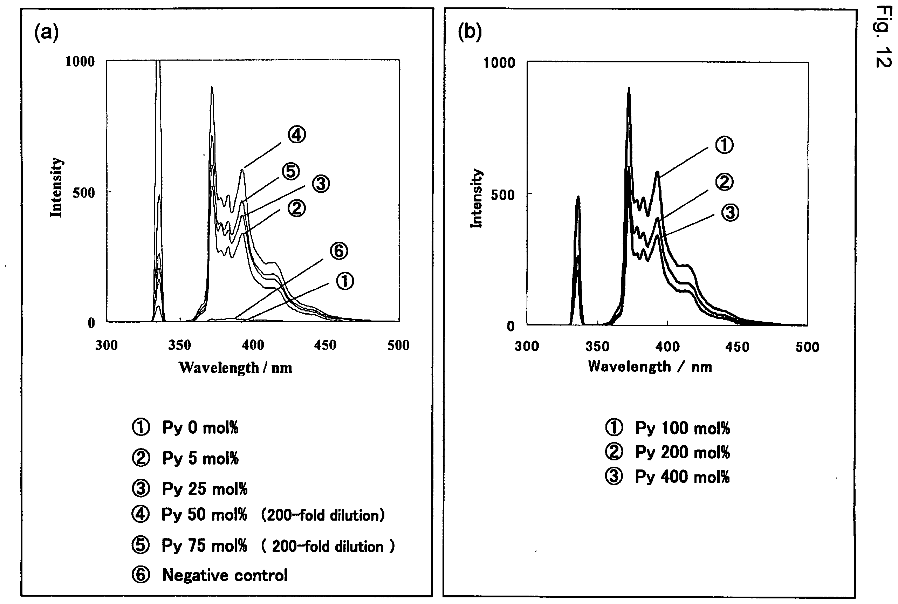

Fig. 12 shows fluorescence spectra, obtained in Comparative Example 7, of lactosomes composed of a polylactic acid-based amphiphilic polymer A1, containing neither A2 nor B) and having pyrene encapsulated therein, wherein Fig. 12 (a) shows the fluorescence spectra when the concentration of pyrene is 0 to 75 mol% and Fig. 12(b) shows the fluorescence spectra when the concentration of pyrene is 100 to 1000 mol%.

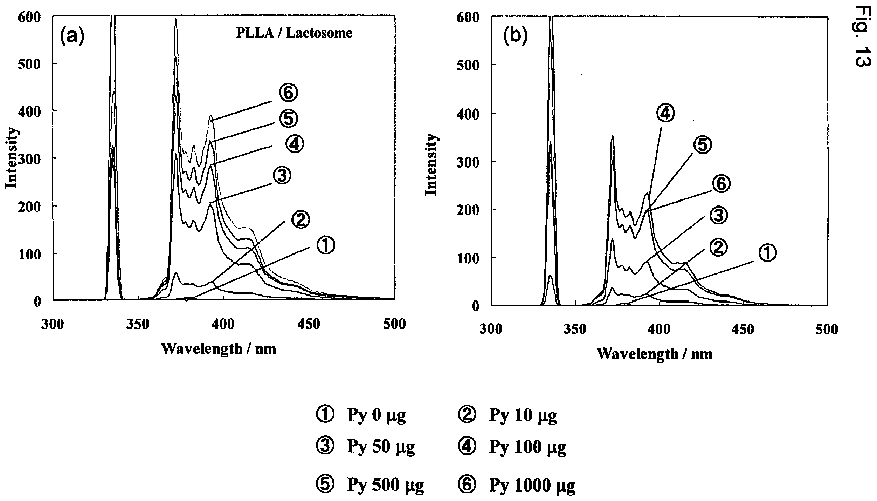

Fig. 13 shows fluorescence spectra, obtained in Example 7, of A1/A2-based lactosomes composed of a polylactic acid-based amphiphilic polymer A1 and a polylactic acid A2 (polylactic acid-blended lactosomes) having pyrene encapsulated therein, wherein Fig. 13(a) shows fluorescence spectra of A1/A2-based lactosomes (PLLA/lactosomes) composed of a polylactic acid-based amphiphilic polymer A1 (PLLA₃₁-PSar₁₅₀) and a polylactic acid A2 (PLLA), and Fig. 13(b) shows fluorescence spectra of A1/A2-based lactosomes (rac-PLA/lactosomes) composed of a polylactic acid-based amphiphilic polymer A1 (PLLA₃₁-PSar₁₅₀) and a polylactic acid A2 (rac-PLA).

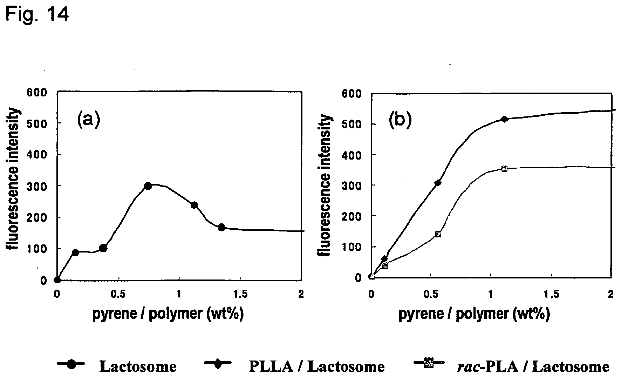

Fig. 14 is a graph showing the relationship between the concentration of pyrene and fluorescence intensity at 373 nm in the case of the lactosomes containing neither A2 nor B and having pyrene encapsulated therein (Fig. 14(a)) obtained in Comparative Example 7, and in the case of the A1/A2-based lactosomes (polylactic acid-blended lactosomes) having pyrene encapsulated therein (Fig. 14 (b)) obtained in Example 7.

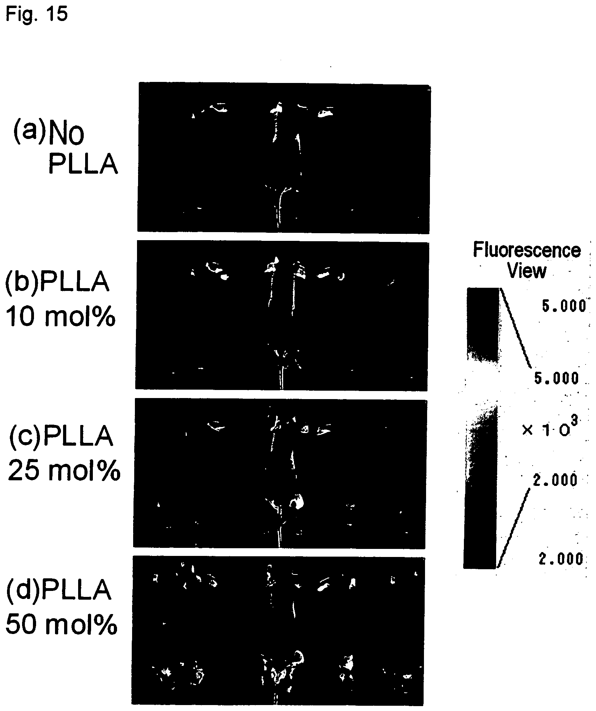

Fig. 15 shows the result, obtained in Example 8, of fluorescence imaging test of cancer-bearing mice, and more specifically shows the result (a) of a fluorescence imaging test using a molecular probe which is A1/B-based lactosome nanoparticle and the results (b), (c), and (d) of a fluorescence imaging test using a molecular probe which is A1/A2/B-based lactosome nanoparticle.

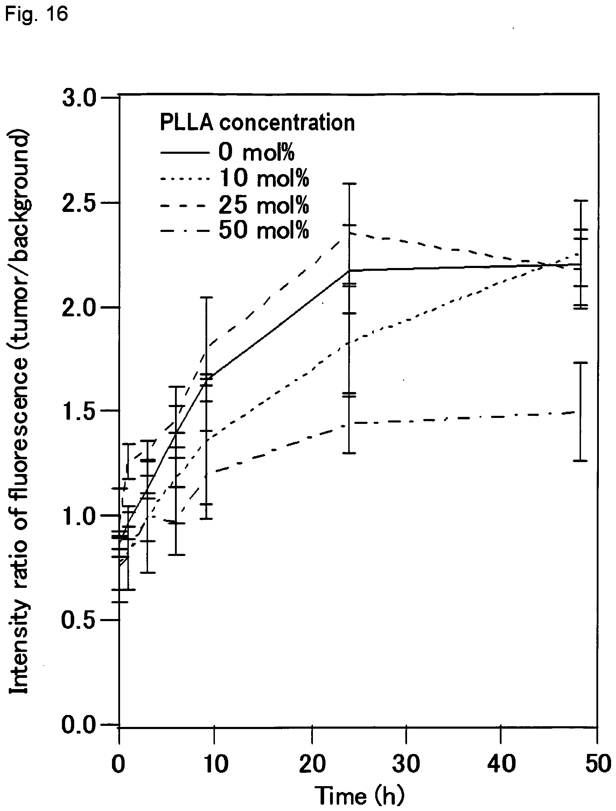

Fig. 16 is a graph, obtained in Example 8, showing changes in the ratio of fluorescence intensity at cancer site to background fluorescence intensity with time in a fluorescence imaging test of cancer-bearing mice.

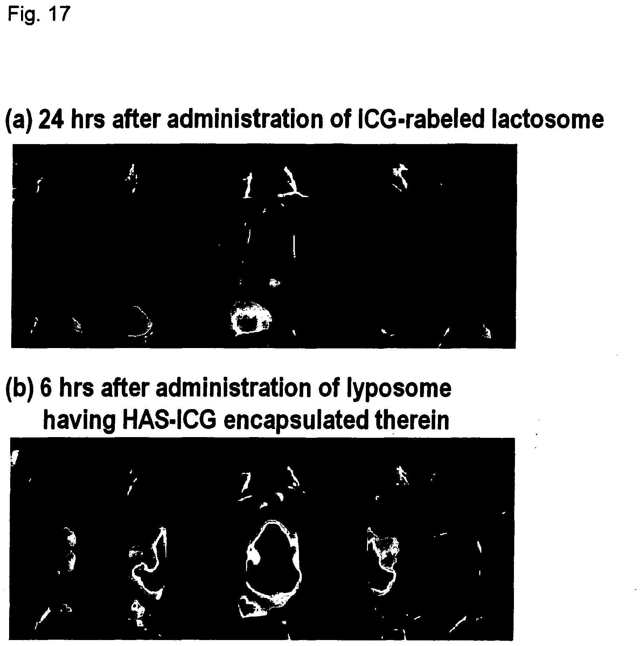

Fig. 17 shows a comparison between the result of fluorescence imaging test of a subcutaneous cancer using A1/B-based lactosome as a fluorescent probe (Example 9; (a)) and the result of fluorescence imaging test of a subcutaneous cancer using liposome as a fluorescent probe (Comparative Example 8; (b)).

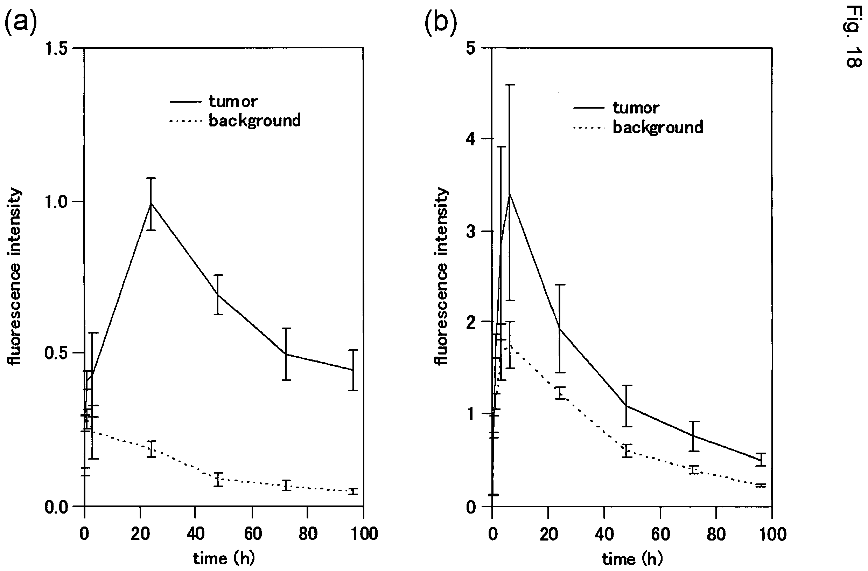

Fig. 18 shows a comparison of fluorescence intensity at subcutaneous cancer and background fluorescence intensity between the case of using A1/B-based lactosome as a fluorescent probe (Example 9; (a)) and the case of using liposome as a fluorescent probe (Comparative Example 8; (b)).

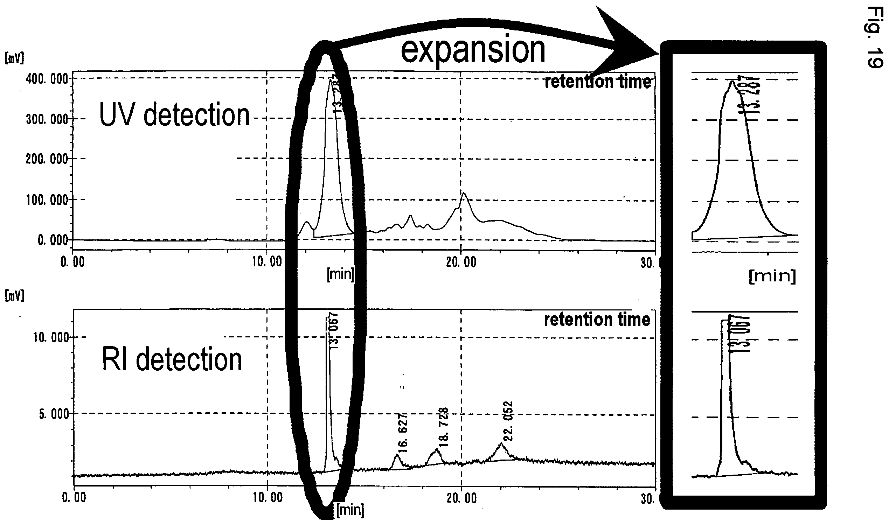

Fig. 19 shows the result, obtained in Experimental Example 13, of HPLC fractionation of ¹⁸F-PLLA which is a labeled polymer B.

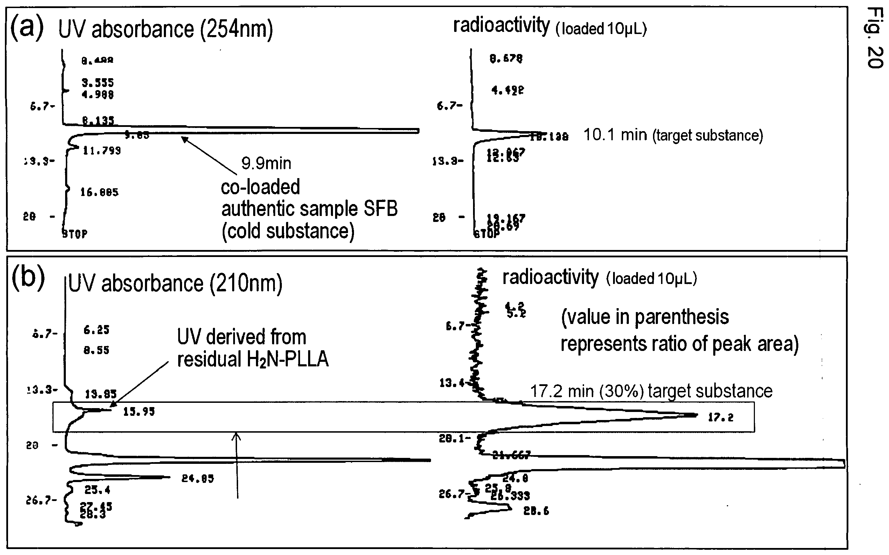

Fig. 20 shows the result (a) of HPLC fractionation of a precursor of ¹⁸F-BzPLLA as a labeled polymer B and the result (b) of HPLC fractionation of ¹⁸F-BzPLLA as a labeled polymer B, obtained in Experimental Example 14.

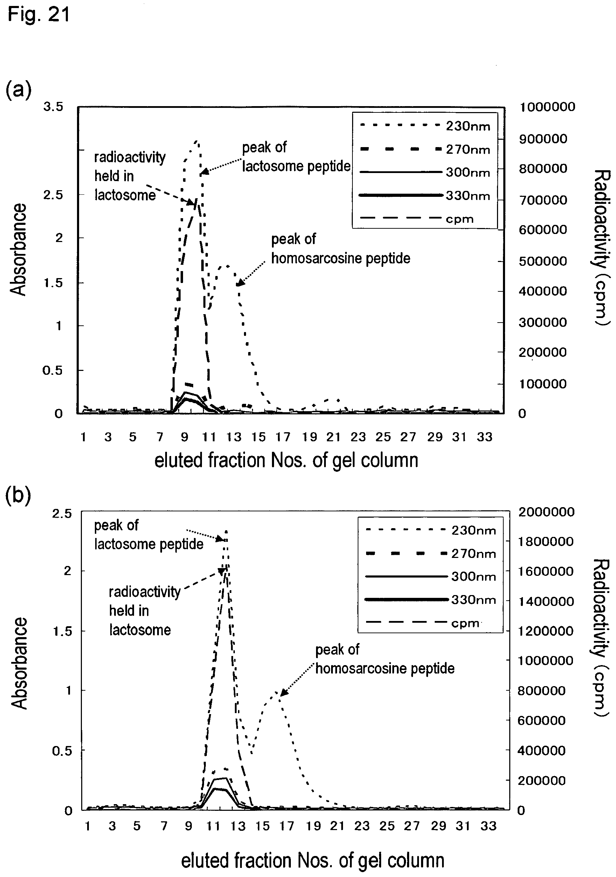

Fig. 21 shows the result of confirmation, obtained in Example 11, that A1/B-based lactosome nanoparticle has ¹⁸F-BzPLLA encapsulated therein as a labeled polymer B.

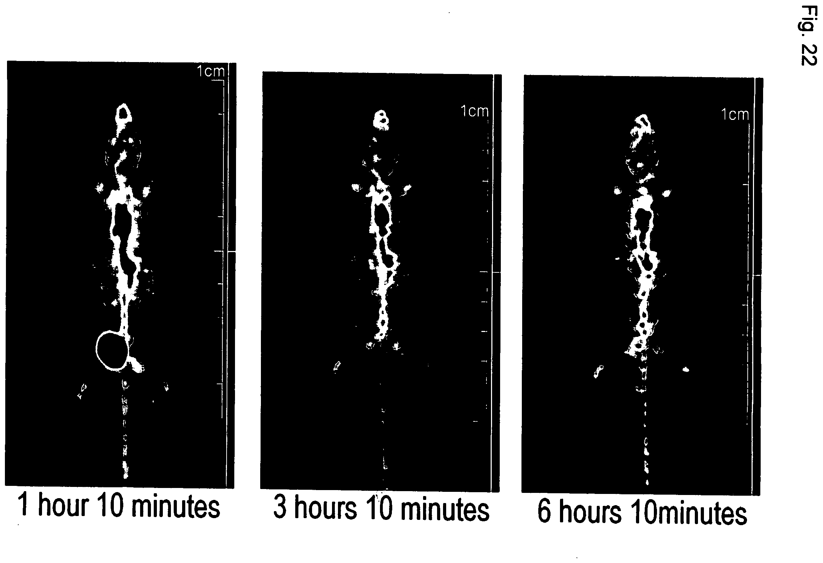

Fig. 22 shows the result, obtained in Example 12, of PET measurement test of a cancer-bearing mouse using A1/B-based lactosome nanoparticles, and more specifically shows signal intensities of a tumor-bearing mouse measured by PET after lapses of 1 hour and 10 minutes, 3 hours and 10 minutes, and 6 hours and 10 minutes from administration of ¹⁸F-lactosome nanoparticle which is A1/B-based lactosome nanoparticle as a PET probes.

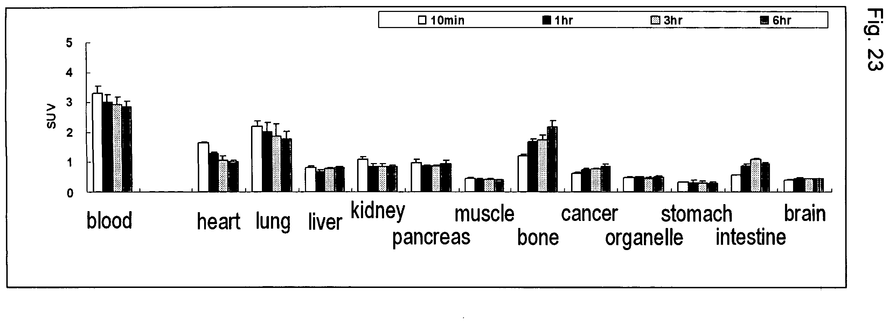

Fig. 23 shows the result, in Example 12, of confirmation of signal intensities in the body of a cancer-bearing mouse, to which A1/B-based lactosome nanoparticle has been administered, by autopsy.

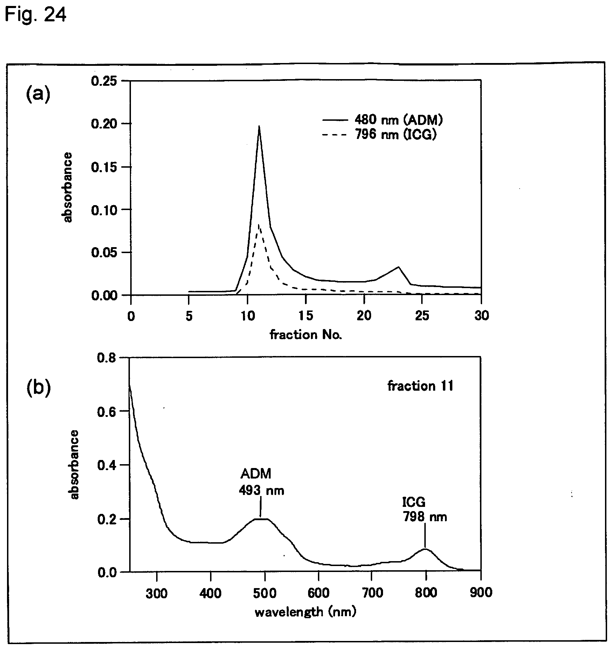

Fig. 24 shows the result, in Example 13, of confirmation that A1/A2-based lactosome nanoparticles have adriamycin as an anticancer agent encapsulated therein.

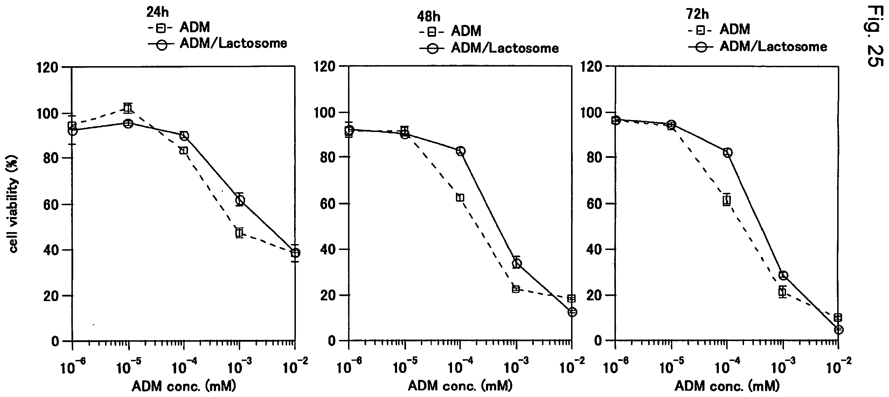

Fig. 25 shows the result, obtained in Example 13, of anticancer activity test of A1/A2-based lactosome nanoparticles having adriamycin encapsulated therein.

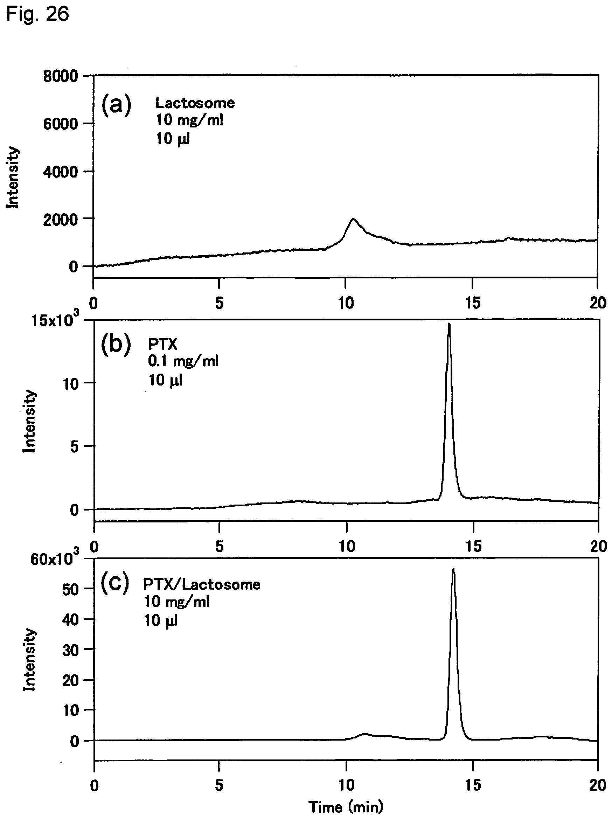

Fig. 26 shows the result in Example 14, and more specifically shows the result (c) of HPLC of A1/A2-based lactosome having paclitaxel encapsulated therein to determine the amount of paclitaxel contained in the A1/A2-based lactosome, which is compared to the result (a) of HPLC of lactosome containing neither A2 nor B and the result (b) of HPLC of a single substance, paclitaxel.

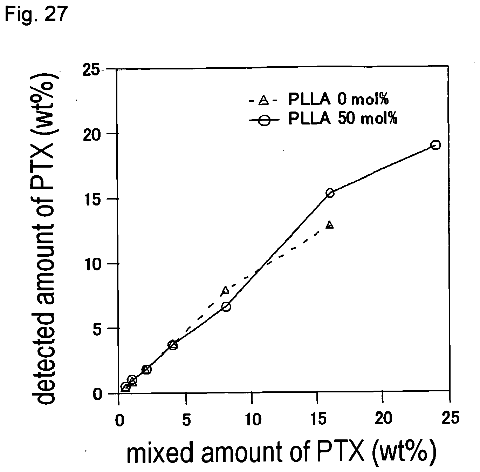

Fig. 27 is a graph, in Example 14, showing the amount of paclitaxel blended to A1/A2-based lactosomes (PLLA 50 mol%) and lactosomes containing neither A2 nor B (PLLA 0 mol%) and the amount of paclitaxel detected.

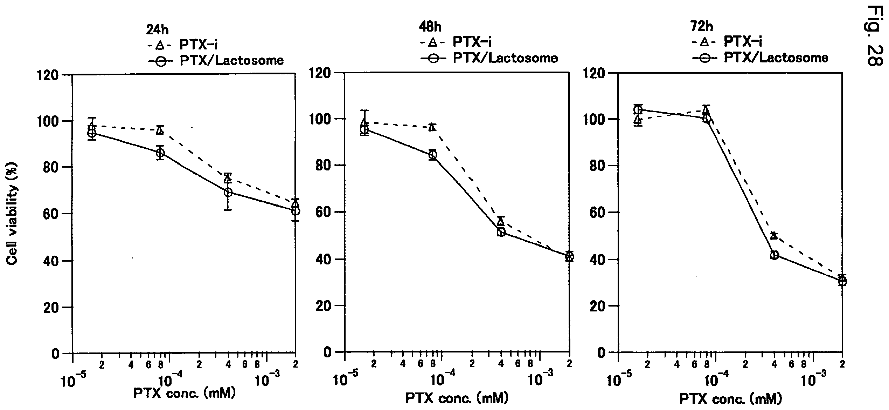

Fig. 28 shows the result, obtained in Example 15, of anticancer activity test of A1/A2-based lactosome nanoparticles having paclitaxel encapsulated therein.

Contents
1. Constituent Polymers of Lactosome
1-1. Amphiphilic Block Polymer A1
1-1-1. Structure of Amphiphilic Block Polymer A1
1-1-1-1. Hydrophilic Block Chain
1-1-1-2. Hydrophobic Block Chain
1-1-1-3. Others
1-1-2. Synthesis of Amphiphilic Block Polymer A1
1-1-2-1. Synthesis Method
1-1-2-2. Polymerization Degree Control and Its Influence on Shape etc. of Lactosome
1-2. Hydrophobic Polymer A2
1-3. Labelled Polymer B
1-3-1. Polymer Part
1-3-1-1. Polylactic Acid
1-3-1-2. Amphiphilic Block Polymer
1-3-2. Labeled Part
1-3-2-1. Signal Group
1-3-2-2. Ligand
1-3-2-3. Binding Form of Labeled Group
1-4. Other Groups
2. Lactosome
2-1. Shape of Lactosome
2-1-1. Micelle
2-1-2. Vesicle
2-1-3. Confirmation of Formation of Micelle or Vesicle
2-2. Size of Lactosome
2-2-1. Size of Particulate Lactosome
2-2-2. Measurement of Size of Lactosome
2-2-3. Control of Size of Lactosome
2-3. Ratio among Constituent Polymers of Lactosome
2-4. Formation of Lactosome
2-4-1. Film Method
2-4-2. Injection Method
3. Molecular Probe
3-1. Molecular Probe for Molecular Imaging
3-2. Molecular Probe for Drug Delivery
3-3. Control of Properties of Molecular Probe
4. Molecular Imaging and Drug Delivery
4-1. Administration of Molecular Probes
4-2. Target of Administration
4-3. Detection of Molecular Probes
4-4. Stability of Lactosome in Blood

### <1. Constituent Polymers of Lactosome>

A molecular assembly (lactosome) according to the present invention comprises, as constituent components, an amphiphilic block polymer A1 having a hydrophobic block basically composed of lactic acid units, a labeled polymer B basically composed of lactic acid units, and, optionally, a hydrophobic polymer A2 basically composed of lactic acid units.

### <1-1. Amphiphilic Block Polymer A1>

Hereinbelow, the structure and synthesis of the amphiphilic block polymer A1 in the present invention will be described.

### <1-1-1. Structure of Amphiphilic Block Polymer A1>

### <1-1-1-1. Hydrophilic Block Chain>

In the present invention, the specific degree of the physical property, "hydrophilicity" of a hydrophilic block chain is not particularly limited, but, at least, the hydrophilic block chain shall be relatively more hydrophilic than a hydrophobic block chain having 10 or more lactic acid units. Alternatively, the hydrophilic block chain shall be hydrophilic to such an extent that a copolymer composed of the hydrophilic block chain and the hydrophobic block chain can have amphiphilicity as a whole molecule of the copolymer. Alternatively, the hydrophilic block chain shall be hydrophilic to such an extent that the amphiphilic block polymer can self-assemble in a solvent to form a particulate self-assembly.

The kinds and ratio of structural units constituting the hydrophilic block chain are appropriately determined by those skilled in the art so that a resultant block chain can have such hydrophilicity as described above as a whole.

The hydrophilic block chain can be designed so that the upper limit of the number of structural units becomes, for example, 500. In the present invention, a hydrophilic block chain whose number of structural units is 50 to 300, preferably 100 to 200 may be often synthesized. If the number of structural units exceeds 500, when a molecular assembly is formed, the resultant molecular assembly tends to lack stability. On the other hand, if the number of structural units is less than 50, formation of a molecular assembly tends to be difficult per se.

As an example of the hydrophilic block chain, a polypeptide chain can be mentioned, and more specifically, a polypeptide chain having 20 or more sarcosine units can be mentioned. In the polypeptide chain, all the 20 or more sarcosine units may be either continuous or discontinuous. However, it is preferred that the polypeptide chain is molecularly-designed so that the basic characteristics thereof (which will be described later) are not impaired as a whole.

In a case where the hydrophilic block chain is a polypeptide chain having 20 or more sarcosine units and has another structural unit other than a sarcosine unit, such another structural unit is not particularly limited, but may be derived from amino acid (including hydrophilic amino acids and other amino acids). It is to be noted that in this specification, the term "amino acid" refers to natural amino acids, unnatural amino acids, and derivatives thereof by modification and/or chemical alteration, and further includes α-, β-, and γ-amino acids. Among them, α-amino acids are preferred, and examples of them include serine, threonine, lysine, aspartic acid, and glutamic acid.

As will be described later in <1-4>, the amphiphilic block polymer A1 may further contain a group such as a sugar chain or polyether. In this case, the amphiphilic block polymer A1 is preferably molecularly-designed so that the hydrophilic block contains a sugar chain or polyether.

Sarcosine (i.e., N-methylglycine) is highly water-soluble, and poly-sarcosine is highly flexible, because it has an N-substituted amide and therefore can be more easily cis-trans isomerized as compared to a normal amide group, and steric hindrance around the C^{α} carbon atom is low. The use of such a polypeptide as a constituent block chain is very useful in that the block chain can have both high hydrophilicity and high flexibility as its basic characteristics.

Any hydrophilic block chain other than the above specific example may be used in the present invention as long as it has such basic characteristics as described above.

### <1-1-1-2. Hydrophobic Block Chain>

In the present invention, the specific degree of the physical property, "hydrophobicity" of a hydrophobic block chain is not particularly limited, but, at least, the hydrophobic block chain shall be hydrophobic enough to be a region relatively more hydrophobic than the hydrophilic block chain so that a copolymer composed of the hydrophilic block chain and the hydrophobic block chain can have amphiphilicity as a whole molecule of the copolymer or so that the amphiphilic block polymer can self-assemble in a solvent to form a particulate self-assembly.

In the present invention, the hydrophobic block chain contains 10 or more lactic acid units (in this specification, the hydrophobic block chain basically composed of lactic acid units is sometimes simply referred to as "polylactic acid") . The hydrophobic block chain preferably contains 20 or more lactic acid units. In the hydrophobic block chain, all the lactic acid units may be either continuous or discontinuous. The kind and ratio of structural unit other than a lactic acid unit constituting the hydrophobic molecular chain are appropriately determined by those skilled in the art so that a resultant block chain can have such hydrophobicity as described above as a whole.

In a case where the hydrophobic block chain contains another structural unit other than a lactic acid unit, the kind and ratio of such another structural unit constituting the hydrophobic block chain are not particularly limited as long as a resultant block chain can have such hydrophobicity as described above as a whole. However, in this case, the hydrophobic block chain is preferably molecularly-designed so as to give various characteristics which will be described later.

In a case where the hydrophobic block chain contains another structural unit other than a lactic acid unit, such another structural unit may be selected from the group consisting of hydroxylic acids other than lactic acid and amino acids (including hydrophobic amino acids and other amino acids). Examples of the hydroxylic acids include glycolic acid and hydroxyisobutyric acid. Many of the hydrophobic amino acids have an aliphatic side chain and/or an aromatic side chain. Examples of natural amino acids include glycine, alanine, valine, leucine, isoleucine, proline, methionine, tyrosine, and tryptophan. Examples of unnatural amino acids include amino acid derivatives such as glutamic acid methyl ester, gluctamic acid benzyl ester, aspartic acid methyl ester, aspartic acid ethyl ester, and aspartic acid benzyl ester.

The upper limit of the number of structural units of the hydrophobic block chain is not particularly limited, but is preferably 100. In the present invention, a hydrophobic block chain whose number of structural units is 10 to 80, preferably 20 to 50 may be often synthesized. If the number of structural units exceeds 100, when a molecular assembly is formed, the resultant molecular assembly tends to lack stability. On the other hand, if the number of structural units is less than 10, formation of a molecular assembly is difficult per se.

Polylactic acid has excellent biocompatibility and stability. Therefore, a molecular assembly obtained from the amphiphilic material containing polylactic acid as a constituent block is very useful from the viewpoint of applicability to a living body, especially a human body.

Further, polylactic acid is rapidly metabolized due to its excellent biodegradability, and is therefore less likely to accumulate in tissue other than cancer tissue in a living body. Therefore, a molecular assembly obtained from the amphiphilic material containing polylactic acid as a constituent block is very useful from the viewpoint of specific accumulation in cancer tissue.

Further, polylactic acid is excellent in solubility in low-boiling point solvents. This makes it possible to avoid the use of a hazardous high-boiling point solvent when a molecular assembly is produced from the amphiphilic material containing polylactic acid as a constituent block. Therefore, such a molecular assembly is very useful from the viewpoint of safety for a living body.

Further, adjustment of the chain length of polylactic acid is preferred in that it contributes as one of factors to control the shape and the size of a molecular assembly produced from the amphiphilic material containing polylactic acid as a constituent block. Therefore, the use of polylactic acid as a constituent block is very useful in that a shape of the resultant molecular assembly can give an excellent versatility.

Therefore, also in a case where the hydrophobic block chain has a structural unit other than a lactic acid unit, the hydrophobic block chain is preferably molecularly-designed so as to give these various excellent characteristics.

From the viewpoint of optical purity, the hydrophobic block chain may include the following variations.

For example, the lactic acid units constituting the hydrophobic block chain may include only L-lactic acid units, or may include only D-lactic acid units, or may include both L-lactic acid units and D-lactic acid units. The hydrophobic block chain may be used singly or in combination of two or more of them selected from the above examples.

In a case where the lactic acid units include both L-lactic acid units and D-lactic acid units, the order of polymerization of L-lactic acid units and D-lactic acid units is not particularly limited. For example, L-lactic acid units and D-lactic acid units may be polymerized so that one or two L-lactic acid units and one or two D-lactic acid units are alternately arranged, or may be randomly polymerized, or may be block-polymerized.

Therefore, in a case where the lactic acid units include both L-lactic acid units and D-lactic acid units, the amount of each of the lactic acid units is not particularly limited. That is, the amount of L-lactic acid units contained in the hydrophobic block chain and the amount of D-lactic acid units contained in the hydrophobic block chain may be different from each other, or may be the same, and in this case the 10 or more lactic acid units may be a racemate having an optical purity of 0% as a whole.

Which of the variations of the hydrophobic block chain different in optical purity is used may be determined in consideration of the balance with the optical purity of the hydrophobic polymer A2 which will be described later. This will be described later in <1-2>.

### <1-1-1-3. Others>

In usual, the amphiphilic block polymer A1 does not have a labeling group. However, the amphiphilic block polymer A1 may further have a labeling group as long as it can be distinguished from other components (i.e., from the hydrophobic polymer A2 and the labeled polymer B which will be described later) when a molecular assembly is formed.

### <1-1-2. Synthesis of Amphiphilic Block Polymer A1>

### <1-1-2-1. Synthesis Method>

In the present invention, a method for synthesizing the amphiphilic block polymer A1 is not particularly limited, and any of well-known peptide synthesis method, polyester synthesis method, and/or depsipeptide synthesis method may be used.

Peptide synthesis may be performed by, for example, ring-opening polymerization of N-carboxyamino acid anhydride (amino acid NCA) using a base, such as an amine, as an initiator.

Polyester synthesis may be performed by, for example, ring-opening polymerization of lactide using a base, such as an amine, or a metal complex as an initiator. The type of lactide may be appropriately determined by those skilled in the art in consideration of a desired optical purity of a resultant block chain. For example, the type of lactide may be appropriately selected from among L-lactide, D-lactide, DL-lactide, and mesolactide, and the amount of lactide used may be determined by those skilled in the art so that a resultant block chain can have a desired optical purity.

Depsipeptide synthesis can be performed by, for example, the following method. Polylactic acid as a hydrophobic block is first synthesized, and then a polypeptide chain as a hydrophilic block is extended. Alternatively, a polypeptide chain as a hydrophilic block is first synthesized, and then polylactic acid as a hydrophobic block is extended.

It has been already confirmed by the present inventors that adjustment of the chain length of polylactic acid is one of means for more easily controlling the shape and size of the molecular assembly according to the present invention. Therefore, from the viewpoint of more flexibly controlling the chain length of polylactic acid, the amphiphilic block polymer A1 is preferably synthesized by first synthesizing polylactic acid as a hydrophobic block and then extending a polypeptide chain as a hydrophilic block chain.

### <1-1-2-2. Polymerization Degree Control and Its Effect on Shape etc. of Lactosome>

It has been already confirmed by the present inventors that polylactic acid as a hydrophobic block chain constituting the amphiphilic block polymer A1 enables to control the polymerization degree more easily and accurately than polysarcosine as a hydrophilic block chain. That is, the use of polylactic acid as a hydrophobic block chain makes it possible to more easily and accurately control the chain length. As described above, adjustment of the chain length of polylactic acid is one of means for controlling the shape and size of the molecular assembly according to the present invention. Therefore, accurate control of the chain length of polylactic acid is effective in that the shape and size of the molecular assembly can be easily controlled. For this reason, the use of the polylactic acid as a block chain in the amphiphilic block polymer for forming the molecular assembly makes it possible to accurately and easily form a desired molecular assembly.

It is to be noted that the molecular weight of the amphiphilic block polymer A1 in the present invention is set to such a value that the total number of structural units of the amphiphilic block polymer A1 does not become less than 20. If the total number of structural units of the amphiphilic block polymer A1 is less than 20, it strongly tends to occur the precipitation so as not to be dispersed in water.

In a case where a molecular assembly composed of the amphiphilic block polymer A1 and the labeled polymer B (which will be described later) is intended to be used as, for example, a molecular probe for molecular imaging or drug delivery, the molecular weight of the amphiphilic block polymer A1 is appropriately determined by those skilled in the art in consideration of the type of substance (i.e., a label or a drug) to be carried by the molecular probe, the effective concentration of the substance, and the duration of release of the substance.

### <1-2. Hydrophobic Polymer A2>

The hydrophobic polymer A2 is a hydrophobic polymer having 10 or more lactic acid units. Preferably, the hydrophobic polymer A2 has 15 or more lactic acid units . Here, the specific degree of "hydrophobicity" of the hydrophobic polymer A2 is not particularly limited, but at least, the hydrophobic polymer A2 is relatively more hydrophobic than the hydrophilic block of the amphiphilic polymer A1.

It is preferred that the hydrophobic polymer A2 is mainly composed of 10 or more lactic acid units. However, the hydrophobic polymer A2 may have another structural unit other than a lactic acid unit. All the lactic acid units may be either continuous or discontinuous.

As will be described later, the kind of structural unit and the chain length of the hydrophobic polymer A2 may be basically determined from the same point of view as in the case of the molecular design of the hydrophobic block chain of the amphiphilic block polymer A1 described above in <1-1-1-2>. This makes it possible to obtain the effect that the hydrophobic polymer A2 can have excellent affinity for the hydrophobic block chain of the amphiphilic block polymer A1 in a resultant molecular assembly.

In a case where the hydrophobic polymer A2 has another structural unit other than a lactic acid unit, the other structural unit is contained in the hydrophobic polymer A2 to the extent that the hydrophobic polymer A2 does not impinge on deviating from the above-defined "hydrophobicity" as a whole. Therefore, the other structural unit may be either more hydrophilic or more hydrophobic than a lactic acid unit. The kind and the ratio of the another structural units constituting the hydrophobic polymer A2 are appropriately determined by those skilled in the art so that the hydrophobic polymer A2 can have such hydrophobicity as described above as a whole. Examples of the other structural unit include those mentioned above in <1-1-1-2> as structural units other than a lactic acid unit.

The upper limit of the number of structural units of the hydrophobic polymer A2 is not particularly limited as long as the hydrophobic polymer A2 is not longer than the amphiphilic block polymer A1, but is preferably set so that the hydrophobic polymer A2 is not longer than twice the length of the hydrophobic block of the amphiphilic block polymer A1. Therefore, the upper limit of the number of structural units of the hydrophobic polymer A2 may be 200. In the present invention, a hydrophobic polymer A2 whose number of structural units is 10 to 160, preferably 20 to 100 may be often synthesized. If the number of structural units exceeds 200, when a molecular assembly is formed, the resultant molecular assembly tends to lack stability. On the other hand, if the number of structural units is less than 10, the hydrophobic core volume-increasing effect and particle size-controlling effect of the hydrophobic polymer A2 are lowered.

As has been described above in <1-1-1-2>, the molecular assembly according to the present invention preferably has various characteristics such as excellent biocompatibility, excellent stability, excellent biodegradability, and excellent solubility of its constituent polymers in low-boiling point solvents. Therefore, also in a case where the hydrophobic polymer A2 contains the other structural unit, the hydrophobic polymer A2 is preferably molecularly-designed so as to give these various excellent characteristics.

From the viewpoint of optical purity, the hydrophobic polymer A2 may further include the following variations.

For example, the lactic acid units constituting the hydrophobic polymer A2 may include only L-lactic acid units, or may include only D-lactic acid units, or may include both L-lactic acid units and D-lactic acid units. The hydrophobic polymer A2 may be used singly or in combination of two or more of them selected from the above examples.

In a case where the lactic acid units include both L-lactic acid units and D-lactic acid units, the order of polymerization of L-lactic acid units and D-lactic acid units is not particularly limited. For example, L-lactic acid units and D-lactic acid units may be polymerized so that one or two L-lactic acid units and one or two D-lactic acid units are alternately arranged, or may be randomly polymerized, or may be block-polymerized.

Therefore, in a case where the lactic acid units include both L-lactic acid units and D-lactic acid units, the amount of each of the lactic acid units is not particularly limited. That is, the amount of L-lactic acid units contained in the hydrophobic polymer A2 and the amount of D-lactic acid units contained in the hydrophobic polymer A2 may be different from each other, or may be the same, and in the case the 10 or more lactic acid units may be a racemate having an optical purity of 0% as a whole.

Which of the variations of the hydrophobic polymer A2 different in optical purity is used may be determined in consideration of the balance with the optical purity of the hydrophobic block chain of the above-described amphiphilic block polymer A1. From the viewpoint of controlling the particle size of molecular assembly, stably holding a label or a drug, the combination of the hydrophobic block chain of the above-described amphiphilic block polymer A1 and the hydrophobic polymer A2 is preferably selected so that the hydrophobic block chain and the hydrophobic polymer A2 are less likely to form a stereocomplex.

For example, in a case where the hydrophobic block chain of the amphiphilic block polymer A1 is composed of L-lactic acid units, the main chain of the hydrophobic polymer A2 is preferably composed of D-lactic acid units or of both L-lactic acid units and D-lactic acid units.

Further, for example, in a case where the hydrophobic block chain of the amphiphilic block polymer A1 is composed of D-lactic acid units, the main chain of the hydrophobic polymer A2 is preferably composed of L-lactic acid units or of both L-lactic acid units and D-lactic acid units.

In usual, the hydrophobic polymer A2 does not have a labeling group. However, the hydrophobic polymer A2 may further have a labeling group as long as it can be distinguished from other components (i.e., from the amphiphilic block polymer A1 and the labeled polymer B which will be described later) when a molecular assembly is formed.

### <1-3. Labeled Polymer B>

The labeled polymer B has at least 10 or more lactic acid units and a labeling group. Preferably, the labeled polymer B has 15 or more lactic acid units. For example, the labeled polymer B may be mainly composed of 10 or more lactic acid units and a labeling group (i.e., a labeled polylactic acid) or may have the 10 or more lactic acid units as a hydrophobic block and a hydrophilic block as a counterpart to the hydrophobic block (i.e., a labeled amphiphilic block polymer). These labeled polymers may be used singly or in combination of two or more of them. Therefore, for example, a labeled polylactic acid and a labeled amphiphilic block polymer may be used together.

### <1-3-1. Polymer Part>

As has been described above in <1-1-1-2>, the molecular assembly according to the present invention preferably has various properties such as excellent biocompatibility, excellent stability, excellent biodegradability, and excellent solubility of its constituent polymers in low-boiling point solvents. Therefore, a polymer part of the labeled polymer B is preferably molecularly-designed so as to give these various excellent properties.

### <1-3-1-1. Polylactic acid>

In a case where the labeled polymer B is a labeled polylactic acid, a polymer part thereof (i.e., a polylactic acid part) is mainly composed of 10 or more, preferably 15 or more lactic acid units . All the lactic acid units may be either continuous or discontinuous.

The kind of structural unit and the chain length of the polylactic acid part of the labeled polymer B may be basically determined from the same point of view as in the case of the molecular design of the hydrophobic block chain of the amphiphilic block polymer A1 described above in <1-1-1-2>. This makes it also possible to obtain the effect that the labeled polymer B can have excellent affinity for the hydrophobic block chain of the amphiphilic block polymer A1 in a resultant molecular assembly.

### <1-3-1-2. Amphiphilic Block Polymer>

In a case where the labeled polymer B is a labeled amphiphilic block polymer, a polymer part thereof (i.e., an amphiphilic block polymer part) may have a hydrophilic block chain and a hydrophobic block chain having 10 or more, preferably 15 or more lactic acid units . All the lactic acid units may be continuous or discontinuous.

The kinds of structural units and the chain length of the amphiphilic block polymer part of the labeled polymer B may be basically determined from the same point of view as in the case of the molecular design of the amphiphilic block polymer A1 described above in <1-1-1>. This makes it also possible to obtain the effect that the labeled polymer B can have excellent affinity for the hydrophobic block chain of the amphiphilic block polymer A1 in a resultant molecular assembly.

### <1-3-2. Labeled Part>

A labeled part is selected from the group consisting of a signal group and a ligand.

### <1-3-2-1. Signal Group>

A signal group is a group having a property detectable for imaging. According to the present invention, the signal group is selected from the group consisting of fluorescent groups, radioactive element-containing groups, and magnetic groups. Means for detecting these groups may be appropriately selected by those skilled in the art.

Examples of fluorescent groups include groups derived from fluorescein-based pigments, cyanine-based pigments such as indocyanine pigments, rhodamine-based pigments, and quantum dots.

In the present invention, near-infrared fluorescent groups (e.g., groups derived from cyanine-based pigments or quantum dots) are used as fluorescent groups.

Each substituent group having a hydrogen bond exhibits absorption in the near-infrared region (700 to 1300 nm), but the degree of absorption is relatively small. Therefore, near-infrared light easily penetrates through living tissue. It can be said that by utilizing such characteristics of near-infrared light, in-vivo information can be obtained without putting an unnecessary load on the body. Particularly, when a target to be measured is decided to a site close to the body surface of a small animal, near-infrared fluorescence can give useful information.

More specific examples of the near-infrared fluorescent groups include indocyanine pigments such as ICG (indocyanine green), Cy7, DY776, DY750, Alexa790, and Alexa 750. In a case where the molecular assembly according to the present invention is intended for use targeting, for example, cancer, an indocyanine pigment such as ICG or DY750 may be particularly preferably used from the viewpoint of accumulation in a cancer.

Examples of the radioactive element-containing groups include groups derived from saccharides, amino acids, or nucleic acids labeled with a radioisotope such as ¹⁸F. One specific example of a method for introducing a radioactive element-containing group includes a method comprising the step of polymerizing lactide using mono-Fmoc ethylenediamine, the step of protecting a terminal OH group by a silyl protecting group, the step of eliminating Fmoc by piperidine treatment, the step of polymerizing sarcosine-N-carboxyanhydride (SarNCA) and terminating the end of the polymer, the step of eliminating the silyl protecting group to perform conversion to a sulfonate ester (e.g., trifluoromethanesulfonate ester, p-toluenesulfonate ester), and the step of introducing a radioactive element-containing group. If necessary, this specific example may be modified by those skilled in the art.

Examples of the magnetic groups include groups having a magnetic substance such as ferrichrome and groups contained in ferrite nanoparticles and magnetic nanoparticles.

### <1-3-2-2. Ligand>

The ligand is selected from ligands for, when the molecular assemblies according to the present invention are administered, allowing the molecular assemblies to specifically bind to a target site and ligands for coordinating to a molecule or an atom of a drug or a signal agent to be delivered to a target site when the molecular assemblies according to the present invention are administered.

As a targeting ligand for allowing the molecular assembly to specifically bind to a target site, a ligand selected from the group consisting of antibodies and adhesion factors such as RGD (arginine-glycine-aspartic acid) is used according to the present invention.

As a ligand for coordinating to a molecule or an atom of a drug or a signal agent to be delivered to a target site, a tricarboxylic acid which can coordinate to a transition metal is used according to the present invention.

### <1-3-2-3. Binding Type of Labeling Group>

One labeled polymer may have one or more labeling groups . That is, one or more labeling groups may be bound to one polymer part. In this case, the term "bind" specifically refers to covalent binding and "binding" refers to direct binding to a specific site in the polymer part and indirect binding to a specific site in the polymer part via an appropriate spacer group. The spacer group is not particularly limited, and is appropriately selected by those skilled in the art. Examples of such a spacer group include alkyl groups, polysaccharides such as carboxymethylcellulose and amylose, and water-soluble polymers such as polyalkylene oxide chains, polyethylene glycol chains, and polyvinylalcohol chains.

The labeling group may be bound to any site in the polymer part.

In a case where the polymer part of the labeled polymer B is polylactic acid, the labeling group may be bound to a terminal structural unit of the polylactic acid or may be bound to an internal structural unit other than the terminal structural units. In either case, a molecular assembly formed from such a labeled polymer B (i.e., a labeled polylactic acid) and the amphiphilic block polymer A1 holds the lebeling group in its inside. More specifically, in a case where the molecular assembly is in the form of a micelle, the labeling group may be held in a hydrophobic part located inside the micelle, or may be held around the interface between a hydrophilic part and a hydrophobic part of the micelle. In a case where the molecular assembly is in the form of a vesicle, the labeling group may be held in a membrane tissue of the vesicle by embedding.

In a case where the polymer part of the labeled polymer B is an amphiphilic block polymer, the labeling group may be bound to, for example, a terminal structural unit of the amphiphilic block polymer. Particularly, the labeling group may be bound to a hydrophilic block-side terminal structural unit of the amphiphilic block polymer. A molecular assembly formed from such a labeled polymer B (i.e., a labeled amphiphilic block polymer) and the amphiphilic block polymer A1 may hold the labeling group on the surface thereof, that is, may be surface-modified with the labeling group.

On the other hand, in a case where the polymer part of the labeled polymer B is an amphiphilic block polymer, the labeling group may also be bound to an internal structural unit other than terminal structural units of the amphiphilic block polymer. A molecular assembly formed from such a labeled polymer B (i.e., a labeled amphiphilic block polymer) and the amphiphilic block polymer A1 may hold the labeling group in its inside. More specifically, in a case where the molecular assembly is in the form of a micelle, the labeling group may be held in the inside of the micelle, and in a case where the molecular assembly is in the form of a vesicle, the labeling group may be held in a membrane tissue of the vesicle by embedding.

### <1-4. Other Groups>

In the present invention, the amphiphilic block polymer A1, the labeled polymer B, and the optional hydrophobic polymer A2 may further have additional groups. Such groups are not particularly limited, and are appropriately selected by those skilled in the art. Examples of the groups include functional groups such as organic groups having an appropriate chain length. Such a group is appropriately selected by those skilled in the art, and may serve as a group allowing the molecular assembly according to the present invention to have a desired form and/or a desired function so that the molecular assembly becomes more useful as, for example, a molecular probe for molecular imaging or drug delivery. More specific examples of such a functional group include sugar chains and water-soluble polymers. Examples of the sugar chains include carboxymethylcellulose and amylose. Examples of the water-soluble polymers include polyether chains and polyvinyl alcohol chains. Specific examples of the polyether chains include polyalkylene oxide chains such as polyethyleneglycol chains.

### <2. Lactosome>

The molecular assembly (lactosome) according to the present invention is a structure formed by aggregation or self-assembling orientation and association of the amphiphilic block polymer A1 and the labeled polymer B, and optionally the hydrophobic polymer A2,. That is, the molecular assembly according to the present invention includes an A1/B-based lactosome composed of at least the amphiphilic block polymer A1 and the labeled polymer B; and an A1/A2/B-based lactosome composed of at least the amphiphilic block polymer A1, the hydrophobic polymer A2, and the labeled polymer B.

### <2-1. Shape of Lactosome>

As described above, the molecular assembly according to the present invention is a structure formed by aggregation or self-assembling orientation and association of the amphiphilic block polymer A1 and the labeled polymer B, and optionally the hydrophobic polymer A2, and therefore the shape thereof is not particularly limited. That is, examples of the shape of the molecular assembly according to the present invention include micelle, vesicle, rod, and any other forms of molecular aggregation. By controlling the molecular structure or interaction point of the amphiphilic block polymer, it is possible to produce molecular assemblies having various shapes.

Since the molecular assembly according to the present invention has a particulate shape such as a micelle or a vesicle, the molecular assembly is useful as a molecular probe for molecular imaging or drug delivery. Therefore, the shape of the molecular assembly is appropriately determined by those skilled in the art in consideration of the intended use of the molecular assembly and other factors.

### <2-1-1. Micelle>

Fig. 8 is a schematic view of one reference example of a micelle having the hydrophobic polymer A2. The micelle shown in Fig. 8 is formed by self-assembling of the amphiphilic block polymer A1 (represented as PLLA₃₀-PSar₁₅₀ in Fig. 8) and the hydrophobic polymer A2 (represented as PLA in Fig. 8).

As exemplified in Fig. 8, the amphiphilic block polymer A1 self-assemble so that their hydrophobic block chains form a core part. On the other hand, the hydrophobic polymer A2 are located in the hydrophobic core. At this time, the hydrophobic polymer A2 exhibits the function of increasing the volume of the hydrophobic core. In addition to that, the hydrophobic polymer A2 also exhibits the function of increasing the particle size of the micelle. As will be described later in <2-2-3>, the present inventors have found that the degree of these functions of the hydrophobic polymer A2 depends on the blending ratio of the hydrophobic polymer A2.

According to the present invention, the molecular assembly has the labeled polymer B. When the labeled polymer B is polylactic acid, the micelle may hold a labeling group in a hydrophobic part located in its inside or around the interface between a hydrophilic part and a hydrophobic part whichever structural unit of the polylactic acid the labeling group is bound to, as described above in <1-3-2-3>,.

When polymer part of the labeled polymer B is an amphiphilic block polymer and a labeling group is bound to, for example, a hydrophilic block-side terminal structural unit of the amphiphilic block polymer, the micelle may hold the labeling group on its surface, that is, the micelle may be surface-modified with the labeling group.

On the other hand, in a case where the polymer part of the labeled polymer B is an amphiphilic block polymer and a labeling group is bound to an internal structural unit other than terminal structural units of the amphiphilic block polymer, the micelle may hold the labeling group in its inside.

Unlike a vesicle which will be described later, a micelle does not have a hollow space in its inside. However, a desired substance can be further encapsulated in the inside of a micelle by allowing the substance to coexist in the process of forming the micelle. In this case, the substance may be appropriately determined by those skilled in the art depending on, for example, the intended use of the micelle. For example, the substance may be a drug molecule or a labeling agent molecule. Such a drug molecule and a labeling agent molecule are often hydrophobic compounds. In this case, as described above, the micelle preferably contains the hydrophobic polymer A2 because the hydrophobic core of the micelle is excellent in capacity for holding such a hydrophobic compound.

For example, when the molecular assembly according to the present invention is intended for use as a cancer-targeting molecular probe, there is a case where it is particularly preferably in the form of a micelle from the viewpoint of EPR (enhanced permeability and retention) effect.

### <2-1-2. Vesicle>

As described above in <1-3-2-3>, in a case where the molecular assembly has the labeled polymer B whose polymer part is polylactic acid, the vesicle may hold a labeling group in its membrane tissue by embedding whichever structural unit of the polylactic acid the labeling group is bound to.

In a case where the polymer part of the labeled polymer B is an amphiphilic block polymer and a labeling group is bound to, for example, a hydrophilic block-side terminal structural unit, the vesicle may hold the labeling group on its surface, that is, the vesicle may be surface-modified with the labeling group.

On the other hand, in a case where the polymer part of the labeled polymer B is an amphiphilic block polymer and a labeling group is bound to an internal structural unit other than terminal structural units of the amphiphilic block polymer, the vesicle may hold the labeling group in its membrane tissue by embedding.

A vesicle usually has a hollow space filled with an aqueous phase in its inside, and therefore it may allow the aqueous phase to contain a substance to be encapsulated in the vesicle. The substance may be appropriately determined by those skilled in the art depending on, for example, the intended use of the vesicle. For example, the substance may be a drug molecule or a labeling agent molecule. Such a substance may be encapsulated in the vesicle in the form of a solution or suspension isotonic with the external environment of the molecular assembly.

### <2-1-3. Confirmation of Formation of Micelle or Vesicle>

The shape of the molecular assembly nay be confirmed by observation with a TEM (Transmission Electron Microscope) .

Further, in a case where the molecular assembly has an internal aqueous phase, the formation of the molecular assembly may be confirmed using a water-soluble fluorescent agent. In this case, confirmation may be performed by preparing molecular assemblies so that a water-soluble fluorescent agent is encapsulated in their aqueous phases, and then allowing the molecular assemblies to a column treatment to measure the absorbance of each fraction, and then determining whether absorption by the molecular assembly and absorption by the fluorescent agent are both detected in the same fraction.

### <2-2. Size of Lactosome>

### <2-2-1. Size of Particulate Lactosome>

The molecular assembly according to the present invention has a particulate shape, the particle size thereof is, for example, 10 to 500 nm. The term "particle size" used herein refers to a particle size occurring most frequently in particle size distribution, that is, a mode particle size. A particulate molecular assembly having a particle size less than 10 nm is difficult to be produced, and on the other hand, a particulate molecular assembly having a particle size larger than 500 nm is not suitable as an injection product especially when administered to a living body by injection.

### <2-2-2. Measurement of Size of Lactosome>

A method for measuring the size of the molecular assembly according to the present invention is not particularly limited, and is appropriately selected by those skilled in the art. Examples of such a method include an observational method with a TEM (Transmission Electron Microscope) and a DLS (Dynamic Light Scattering) method. In the case of a DLS method, the translational diffusion coefficient of particles undergoing Brownian movement in a solution is measured.

### <2-2-3. Control of Size of Lactosome>

The size of the molecular assembly may be controlled by, for example, controlling the chain length of each constituent polymer. This technique is effective in roughly determining the size of the lactosome.

For example, as described above in <1-1-2-1> and <1-1-2-2>, adjustment of the degree of polymerization of the hydrophobic block (i.e., polylactic acid) of the amphiphilic block polymer A1 is effective in controlling the chain length of the amphiphilic block polymer A1. The same applies for controlling the chain length of the hydrophobic polymer A2 or the labeled polymer B.

Alternatively, the size of the molecular assembly may be controlled by controlling the amount of the hydrophobic polymer A2 (which will be described later) to be blended. This technique is preferred in that the size of the molecular assembly can be continuously controlled and therefore delicate adjustment of the size of the lactosome can be performed. That is, this technique is effective in that molecular assemblies having a desired size can be obtained by appropriately determining the amount of the hydrophobic polymer A2 to be blended.

### <2-3. Ratio between Constituent Polymers of Lactosome>

The molecular assembly according to the present invention contains at least the amphiphilic block polymer A1 and the labeled polymer B as constituent polymers (i.e., in a case where the molecular assembly is an A1/B-based lactosome or an A1/A2/B-based lactosome) . The ratio between the polymer A and the polymer B is not particularly limited, and is appropriately selected by those skilled in the art. For example, the ratio between the amphiphilic block polymer A1 and the labeled polymer B on molar basis is 1:1000 to 1000:1, preferably 1:100 to 100:1. If the ratio of the amphiphilic block polymer A1 exceeds the above range, the ratio of molecular assemblies not containing a labeling agent to all the resultant molecular assemblies tends to unnecessarily increase. On the other hand, if the ratio of the labeled polymer B exceeds the above range, the resultant molecular assemblies tend to be unstable.

In a case where a molecular assembly contains at least the amphiphilic block polymer A1 and the hydrophobic polymer A2 as constituent polymers (i.e., in a case where the molecular assembly is an A1/A2-based lactosome; or an A1/A2/B-based lactosome according to the present invention), the ratio between the polymer A1 and the polymer A2 is not particularly limited.

However, as described above, the volume of the hydrophobic core of the molecular assembly and the size of the molecular assembly can be controlled by controlling the amount of the hydrophobic polymer A2 to be blended. From such a viewpoint, the amphiphilic block polymer A1 and the hydrophobic polymer A2 may be used in a ratio of, for example, 10:1 to 1:10 on molar basis. If the ratio of the hydrophobic polymer A2 exceeds the above range, it tends to be difficult for the resultant molecular assemblies to maintain their shape. On the other hand, if the ratio of the hydrophobic polymer A2 is less than the above range, it tends to be difficult to obtain effects obtained by blending the hydrophobic polymer A2 (i.e., hydrophobic core volume-increasing effect and particle size-controlling effect).

By using the amphiphilic block polymer A1 and the hydrophobic polymer A2 in amounts satisfying the above range, molecular assemblies having a particle size of, for example, 10 to 500 nm can be prepared.

### <2-4. Formation of Lactosome>

A method for forming the molecular assembly is not particularly limited, and may be appropriately selected by those skilled in the art depending on, for example, the desired shape, size, and characteristics of the molecular assembly and the type, properties, and amount of a substance to be carried by the molecular assembly. If necessary, after being formed by a method which will be described later, molecular assemblies may be surface-modified by a known method.

It is to be noted that whether particles have been formed or not may be confirmed by observation with an electron microscope.

### <2-4-1. Film Method>

A film method is conventionally used for preparing liposomes . The amphiphilic block polymer A1 and the labeled polymer B, and optionally the hydrophobic polymer A2 used in the present invention are soluble in low-boiling point solvents, and therefore the molecular assembly according to the present invention can be prepared by the film method.

The film method includes the following steps of: preparing a solution, in a container(e.g. , a glass container), containing the amphiphilic block polymer A1 and the labeled polymer B, and optionally the hydrophobic polymer A2 in an organic solvent; removing the organic solvent from the solution to obtain a film containing the amphiphilic block polymer A1 and the labeled polymer B, and optionally the hydrophobic polymer A2 on an inner wall of the container; and adding water or an aqueous solution into the container and ultrasonic treatment is performed to convert the film into particulate molecular assemblies to obtain a dispersion liquid of the molecular assemblies. The film method may further include the step of freeze-drying the dispersion liquid of molecular assemblies.

The solution containing the amphiphilic block polymer A1 and the labeled polymer B, and optionally the hydrophobic polymer A2 in an organic solvent is appropriately prepared by those skilled in the art. For example, the solution may be prepared by blending at a time all the polymers A1 and B, and optionally A2 to be used, or by previously preparing a film containing part of the polymers A1 and B, and optionally A2 to be used (e.g. , the polymer A1) and then adding a solution containing the remaining components to be used (e.g., the polymer B and optionally the polymer A2) to the film. The previously-prepared film containing part of the polymers may be formed in accordance with a method which will be described later (i.e., a method for forming a film containing the polymers A1, A2, and/or B).

Preferred examples of the organic solvent used in the film method include low-boiling point solvents. In the present invention, the term "low-boiling point solvent" refers to one whose boiling point is 100°C or less, preferably 90°C or less at 1 atmospheric pressure. Specific examples of such a low-boiling point solvent include chloroform, diethyl ether, acetonitrile, ethanol, acetone, dichloromethane, tetrahydrofuran, hexane.

When such a low-boiling point solvent is used as a solvent for dissolving the polymers A1 and B, and optionally A2, it can be very easily removed. A solvent removal method is not particularly limited, and may be appropriately selected by those skilled in the art depending on, for example, the boiling point of an organic solvent to be used. For example, solvent removal may be performed under reduced pressure or by natural drying.

By removing the organic solvent, a film containing the amphiphilic block polymer A1 and the labeled polymer B, and optionally the hydrophobic polymer A2 is formed on an inner wall of the container. Then, water or an aqueous solution is added to the container having the film attached to the inner wall thereof. The water or aqueous solution is not particularly limited, and may be appropriately selected by those skilled in the art from biochemically or pharmaceutically acceptable ones such as distilled water for injection, normal saline, and buffer solutions.

After adding water or an aqueous solution, the container is subjected to sonication. As a result, molecular assemblies are formed in the process of peeling-off of the film from the inner wall of the container by ultrasound. The sonication may be performed under the conditions of, for example, a temperature of 20°C to 60°C and a treating time of 1 minute to 60 minutes. At the time of the completion of sonication, a dispersion liquid, in which molecular assemblies are dispersed in the above-mentioned water or aqueous solution, is prepared in the container.

This dispersion liquid can be directly administered to a living body. That is, it is not necessary to preserve the obtained molecular assemblies in a solvent-free state. Therefore, the dispersion liquid can be very effectively applied to, for example, the production of molecular probes for PET (Positron Emission Tomography) using a drug having a short half-life.

Further, in a case where the obtained dispersion liquid is subjected to freeze-drying, any known freeze-drying method may be used without limitation. For example, a freeze-dried product of molecular assemblies may be obtained by freezing, with liquid nitrogen, the dispersion liquid of molecular assemblies obtained in such a manner as described above and allowing sublimation to occur under a reduced pressure. That is, this makes it possible to preserve molecular assemblies as a freeze-dried product. If necessary, the freeze-dried product is mixed with water or an aqueous solution to obtain a dispersion liquid of the molecular assemblies, and the thus obtained dispersion liquid of molecular assemblies can be used. The water or aqueous solution is not particularly limited, and may be appropriately selected by those skilled in the art from biochemically or pharmaceutically acceptable ones such as distilled water for injection, normal saline, and buffer solutions.

It is to be noted that, before being subjected to freeze-drying, the dispersion liquid may contain, other than the molecular assemblies according to the present invention formed from the amphiphilic block polymer A1 and the labeled polymer B, and optionally the hydrophobic polymer A2, the molecules of the amphiphilic block polymer A1 and the labeled polymer B, and optionally the hydrophobic polymer A2, remaining without contributing to forming the molecular assemblies. When such a dispersion liquid is subjected to freeze-drying, molecular assemblies can be further formed, in the process of concentration of a solvent, from the molecules of the amphiphilic block polymer A1 and the labeled polymer B, and optionally the hydrophobic polymer A2 remaining without having contributed to forming the molecular assemblies according to the present invention. Therefore, this makes it possible to efficiently prepare the molecular assemblies according to the present invention.

Further, in a case where the labeled polymer B is used and a dispersion liquid of molecular assemblies is not subjected to freeze-drying, as described above, the dispersion liquid of molecular assemblies may contain remaining molecules of the labeled polymer B. Therefore, in a case where such a dispersion liquid is administered to a living body, both a signal derived from a molecular assembly formed from, at least, the amphiphilic block polymer A1 and the labeled polymer B and a signal derived from the labeled polymer B, which may remain in the dispersion liquid, may be detected. However, the signal derived from the labeled polymer B is unnecessary for imaging, and therefore imaging is performed after the labeled polymer B is metabolized.

On the other hand, in a case where the labeled polymer B is used and a dispersion liquid of molecular assemblies is prepared through freeze-drying process, such a dispersion liquid of molecular assembly may contain remaining molecules of the labeled polymer B, but the amount of remaining molecules of the labeled polymer B is smaller. Therefore, in a case where such a dispersion liquid is administered to a living body, even though both a signal derived from a molecular assembly formed from, at least, the amphiphilic block polymer A1 and the labeled polymer B and a signal derived from the labeled polymer B, which may remain in the dispersion liquid, may be detected, the absolute amount of the labeled polymer B is smaller, and therefore the labeled polymer B that provides an unnecessary signal is metabolized more rapidly. This is advantageous in that the efficiency and accuracy of imaging are improved.

### <2-4-2. Injection Method>

An injection method is used for preparing not only the molecular assemblies according to the present invention but also various other molecular assemblies. According to the injection method, molecular assemblies may be prepared in the following manner. The amphiphilic block polymer A1 and the labeled polymer B, and optionally the hydrophobic polymer A2 are dissolved in an organic solvent such as trifluoroethanol, ethanol, hexafluoroisopropanol, or dimethylsulfoxide to obtain a solution. Then, the solution is dispersed in a water-based solvent such as distilled water for injection, normal saline, or a buffer solution. Then, purification such as gel filtration chromatography, filtering, or ultracentrifugation is performed, and then the organic solvent is removed, to prepare the molecular assemblies. In a case where molecular assemblies to be administered to a living body are prepared in such a manner as described above using an organic solvent hazardous to a living body, removal of the organic solvent needs to be strictly performed.

In a case where the molecular assembly are to be prepared as an encapsulated-type vesicle, a substance to be encapsulated is dissolved or suspended in a water-based solvent such as distilled water for injection, normal saline or a buffer solution; and in the above obtained solution or suspension, a solution obtained by dissolving the amphiphilic block polymer A1 and the labeled polymer B, and optionally the hydrophobic polymer A2 in the above-mentioned organic solvent is preferably dispersed.

### <3. Molecular Probe>

The molecular assembly according to the present invention appropriately hold a desired molecule, and such a molecular assembly is suitable for use in molecular imaging and drug delivery. In this specification, the molecular assembly intended to be used in molecular imaging or drug delivery is also referred to as a "molecular probe" or "nanoparticle".

### <3-1. Molecular Probe for Molecular Imaging>

Since the molecular assembly according to the present invention has a labeling group and may have a labeling agent, the molecular assembly is suitable as a molecular probe for molecular imaging.

Examples of the labeling group include those mentioned above in <1-3-2>. These labeling groups may be used singly or in combination of two or more of them.

Examples of the labeling agent include molecules having the signal group described above in <1-3-2-1> and molecules having the ligand group described above in <1-3-2-2>. These molecules may be used singly or in combination of two or more of them.

The molecular probe for molecular imaging may be, for example, of a type having a labeling agent introduced thereinto via a covalent bond or of a type having a signal agent coordinated by a ligand.

In other cases, the molecular probe for molecular imaging in the form of a micelle may be of a type containing a labeling agent therein, and the molecular probe for molecular imaging in the form of a vesicle may be of a type having a labeling agent-containing aqueous phase therein.

The molecular probe for molecular imaging enables the above-described label to specifically accumulate in a lesion or diseased site, which makes it possible to perform imaging of the lesion or diseased site.

Specific examples of the molecular probe for molecular imaging include molecular probes for fluorescence imaging, molecular probes for positron emission tomography (PET), and molecular probes for nuclear magnetic resonance imaging (MRI) .

### <3-2. Molecular Probe for Drug Delivery>

In a case where the molecular assembly according to the present invention contains a ligand coordinating to a drug as a labeling group and/or a drug, such a molecular assembly is useful as a molecular probe for drug delivery.

The drug to be used is not particularly limited as long as it is suited to a target disease. Specific examples of such a drug include anticancer drugs, antibacterial agents, antiviral drugs, anti-inflammatory drugs, immunosuppressive drugs, steroid drugs, hormone drugs, and antiangiogenic agents. These drug molecules may be used singly or in combination of two or more of them.

More specific examples of the anticancer drugs include camptothecin, exatecan (camptothecine derivative), emcitabine, doxorubicin, irinotecan, SN-38 (irinotecan active metabolite), 5-FU, cisplatin, oxaliplatin, paclitaxel, and docetaxel.

The molecular probe for drug delivery may be, for example, of a type having, as a labeling group, a ligand introduced thereinto via a covalent bond and coordinating to a drug.

In other cases, the molecular probe for drug delivery in the form of a micelle may be of a type containing a drug therein, and the molecular probe for drug delivery in the form of a vesicle may be of a type having a drug-containing aqueous phase therein.

The molecular probe for drug delivery enables a drug to specifically accumulate in a lesion or diseased site, which makes it possible to allow the drug to act on cells in the site.

Further, the molecular assembly according to the present invention may have both a drug and a signal agent (or a signal group). In this case, the nanoparticle is useful as a molecular probe for both drug delivery and molecular imaging.

### <3-3. Control of Properties of Molecular Probe>

The introduction of such a group as described above in <1-4> into the amphiphilic block polymer in preparing the molecular assembly according to the present invention makes it possible to impart a function, which varies depending on the type of group introduced, to the molecular probe. Further, the change of chain length of each constituent polymer makes it possible to adjust the particle size, shape, tissue selectivity, in-vivo degradation rate, and sustained-releasability of an encapsulated drug or signal agent of the molecular probe. Further, the control of the amount of the hydrophobic polymer A2 to be blended makes it possible to continuously control the particle size of the molecular probe. On the other hand, the properties of the particle may be controlled by using amphiphilic peptides different in composition and molecular weight in combination.

### <4. Molecular Imaging and Drug Delivery >

Molecular imaging and drug delivery described herein include administration of the molecular probes described above to a living body. Molecular imaging and drug delivery as described herein are characterized by using the molecular probe described above, and other specific procedures may be appropriately determined by those skilled in the art based on the procedures of known molecular imaging and drug delivery.

### <4-1. Administration of Molecular Probes>

A method for administering the molecular probes to a living body is not particularly limited, and may be appropriately determined by those skilled in the art depending on, for example, the administration target and the intended use of the molecular probe. Therefore, the molecular probes may be administered either systemically or locally. More specifically, the molecular probes may be administered by any one of injection (needle injection or needleless injection), oral administration, and external administration.

### <4-2. Administration Target>

The administration target in the molecular imaging and drug delivery is not particularly limited. The molecular assembly according to the present invention is particularly excellent in specific accumulation in a cancer site. The molecular assembly according to the present invention accumulates in cancer tissue due to EPR (enhanced permeability and retention) effect, and therefore its ability to specifically accumulate in cancer tissue does not depend on the type of cancer. For this reason, the administration target of the molecular assembly according to the present invention is preferably a cancer. Examples of the cancer as the administration target include a wide variety of cancers such as liver cancers, pancreas cancers, lung cancers, uterine cervical cancers, breast cancers, and colon cancers.

The ability of the molecular assembly according to the present invention to specifically accumulate in a cancer site is mainly due to, particularly, realization of rapid metabolism in liver. Therefore, the molecular assembly according to the present invention is significantly effective when its administration target is a liver cancer or a cancer that may occur around the liver.

### <4-3. Detection of Molecular Probe>

The molecular imaging as described herein further includes the step of detecting administered molecular probes . By detecting the administered molecular probes, it is possible to observe the appearances of an administration target (especially, the position and size of cancer tissue) from outside the body.

The administered molecular probes may be detected by any means capable of visualizing them. The detection means may be appropriately determined by those skilled in the art depending on the type of signal group or signal agent of the molecular probe.

For example, in the case of fluorescence imaging, a living body, to which molecular probes have been administered, is irradiated with excitation light to detect a signal, such as fluorescence, derived from a signal group or a signal agent of the molecular probe present in the body.

Parameters such as excitation wavelength and fluorescence wavelength to be detected may be appropriately determined by those skilled in the art depending on the type of signal group or signal agent of the molecular probe administered and the type of administration target.

In the case of positron emission tomography (PET), annihilation γ-rays emitted from a signal group or a signal agent of the molecular probe present in the body can be detected by γ-ray detector. In the case of nuclear magnetic resonance imaging (MRI), a local magnetic field distortion produced by a magnetic material of a signal group or a signal agent of the molecular probe in the body is detected as a change in MRI signal by a receiver coil.

The time between administration and the start of detection may be appropriately determined by those skilled in the art depending on the type of signal group or signal agent of the molecular probe administered and the type of administration target. For example, in the case of fluorescence imaging, detection may be started after a lapse of 3 to 48 hours from administration, and in the case of PET or MRI, detection may be started after a lapse of 1 to 9 hours from administration. If the time between administration and the start of detection is less than the above range, a detected signal is too strong and therefore it tends to be difficult to clearly distinguish an administration target from other sites (background) . On the other hand, if the time between administration and the start of detection exceeds the above range, molecular probes tend to be excreted from an administration target.

From the viewpoint of accuracy, detection of administered molecular probes is preferably performed by measuring a living body not from one direction but from two or more directions. More specifically, a living body is preferably measured from at least three directions, more preferably from at least five directions. In the case of measurement from five directions, a living body may be measured from, for example, both right and left abdomen sides, both right and left sides of the body, and back side.

### <4-4. Stability of Lactosome in Blood>

The molecular probe according to the present invention exhibits excellent stability in blood.

More specifically, the blood retention of the molecular probe according to the present invention is at least the same as that of a nanoparticle modified by a water-soluble polymeric compound, polyethylene glycol (PEG), which is conventionally known as a nanoparticle having excellent properties. A method for measuring lactosomes in blood may be appropriately determined by those skilled in the art depending on the type of signal group or signal agent of the molecular probe.

### EXAMPLES

Hereinbelow, the present invention will be described in more detail with reference to the following examples. The examples disclosed in this specification are as follows.
Experimental Example 1: Synthesis of Precursor of Constituent Polymer of Nanoparticle
Experimental Example 2: Synthesis of Constituent Polymer of Nanoparticle - Amphiphilic Block Polymer A
Experimental Example 3: Synthesis of Constituent Polymer of Nanoparticle - Non-Lactic Acid-Based Amphiphilic Block Polymer
Experimental Example 4: Synthesis of Constituent Polymer of Nanoparticle - Labeled Polymer B
Experimental Example 5: Synthesis of Constituent Polymer of Nanoparticle - Labeled Polymer B
Experimental Example 6: Synthesis of Constituent Polymer of Nanoparticle - Labeled Polymer B
Experimental Example 7: Synthesis of Constituent Polymer of Nanoparticle - Labeled Polypeptide
Experimental Example 8: Synthesis of Constituent Polymer of Nanoparticle - Labeled Polysarcosine
Example 1: Production of Molecular Probe - A1/B-Based Lactosome
Comparative Example 1: Production of Molecular Probe - Lactosome Containing Neither A2 Nor B
Comparative Example 2: Production of Molecular Probe - Peptosome
Comparative Example 3: Production of Molecular Probe - Lactosome Containing Neither A2 Nor B
Example 2: Fluorescence Imaging - Using A1/B-Based Lactosome
Comparative Example 4: Fluorescence Imaging - Using Lactosome Containing Neither A2 nor B
Comparative Example 5: Fluorescence Imaging - Using Peptosome
Comparative Example 6: Fluorescence Imaging - Using Lactosome Containing Neither A2 Nor B
Example 3: Fluorescence Imaging - Using A1/B-Based Lactosome
Example 4: Fluorescence Imaging - Using A1/B-Based Lactosome
Example 5: Fluorescence Imaging - Using A1/B-Based Lactosome
Experimental Example 9: Synthesis of Constituent Polymer of Nanoparticle - Hydrophobic Polymer A2
Experimental Example 10: Examination of Heat Characteristics of Constituent Polymer of Nanoparticle - Hydrophobic Polymer A2
Example 6: Production of Nanoparticles - A1/A2-Based Lactosome
Comparative Example 7: Production of Molecular Probe - Lactosome Containing Neither A2 Nor B
Example 7: Production of Molecular Probe - A1/A2-Based Lactosome
Example 8: Fluorescence Imaging - Using A1/A2(/B)-Based Lactosome
Example 9: Fluorescence Imaging - Using A1/B-Based Lactosome
Comparative Example 8: Fluorescence Imaging - Using Liposome
Experimental Example 11: Synthesis of Precursor of Constituent Polymer of Nanoparticle
Experimental Example 12: Synthesis of Precursor of Constituent Polymer of Nanoparticle
Experimental Example 13: Synthesis of Constituent Polymer of Nanoparticle - Labeled Polymer B
Example 10: Production of Molecular Probe - A1/B-Based Lactosome
Experimental Example 14: Synthesis of Constituent Polymer of Nanoparticle - Labeled Polymer B
Example 11: Production of Molecular Probe - A1/B-Based Lactosome
Example 12: PET Imaging - Using A1/B-Based Lactosome Example 13: Preliminary Test of DDS - Using A1/A2-Based Lactosome
Example 14: Production of Molecular Probe - A1/A2-Based Lactosome (Comparison with Lactosome Containing Neither A2 Nor B)
Example 15: Preliminary Test of DDS - Using A1/A2-Based Lactosome
Examples 6, 7, and 13 to 15 represent Reference Examples.
Hereinbelow, each of examples will be described in detail.

### <Experimental Example 1: Synthesis of Aminated Poly-L-Lactic Acid (a-PLA)>

Tn this experimental example, aminated poly-L-lactic acid (a-PLA) was synthesized using L-lactide (compound 1) and N-carbobenzoxy-1,2-diaminoethane hydrochloride (compound 2) (Scheme 1).

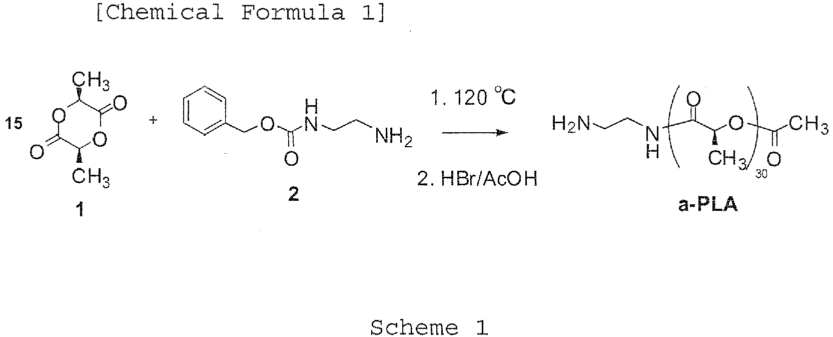

To N-carbobenzoxy-1,2-diaminoethane hydrochloride (compound 2) (310 mg, 1.60 mmol) served as a polymerization initiator, a dispersion liquid obtained by dispersing tin octanoate (6.91 mg) in toluene (1.0 mL) was added. The toluene was distilled away under reduced pressure, and then L-lactide (compound 1) (3.45 g, 24 mmol) was added to perform polymerization reaction at 120°C under Ar gas atmosphere. After 12 hours, the reaction container was air-cooled to room temperature to obtain a yellowish-white solid. The yellowish-white solid was dissolved in a small amount of chloroform (about 10 mL). The chloroform was dropped into cold methanol (100 mL) to obtain a white precipitate. The white precipitate was collected by centrifugation and dried under reduced pressure.

To a dichloromethane (1 mL) solution of the obtained white precipitate (500 mg), 25 v/v% hydrogen bromide/acetic acid (2.0 mL), and the mixture was stirred for 2 hours under dry air atmosphere in a shading environment. After the completion of reaction, a resultant reaction solution was dropped into cold methanol (100 mL) so that a precipitate was deposited. The precipitate was collected by centrifugation. The obtained white precipitate was dissolved in chloroform, washed with a saturated aqueous NaHCO₃ solution, and then dehydrated with anhydrous MgSO₄. Then, the MgSO₄ was removed by Celite® filtration, and the white precipitate was vacuum-dried to obtain white amorphous powder of a-PLA (440 mg) .

### <Experimental Example 2: Synthesis of Amphiphilic Block Polymer A1 (Polysarcosine-Poly-L-Lactic Acid; PSL1) >

In this experimental example, an amphiphilic substance, polysarcosine-poly-L-lactic acid (PSL1) was synthesized from sarcosine-NCA (Sar-NCA) and aminated poly-L-lactic acid (a-PLA) (Scheme 2).

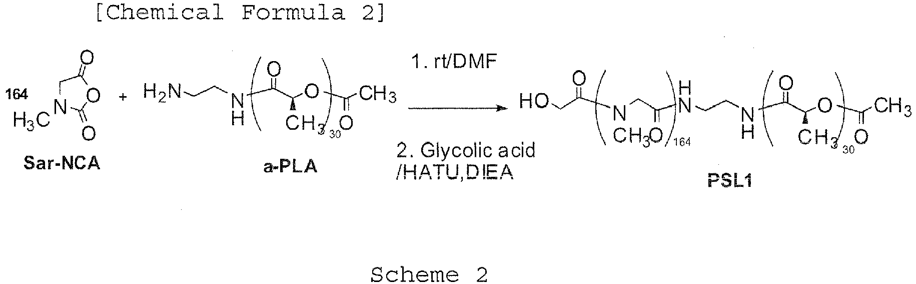

Dimethylformamide (DMF) (140 mL) was added to a-PLA (383 mg, 0.17 mmol) and sarcosine-NCA (Sar-NCA) (3.21 g, 27.9 mmol) under Ar gas atmosphere, and the mixture was stirred at room temperature for 12 hours to obtain a reaction solution. Then, the reaction solution was cooled to 0°C, and then glycolic acid (72 mg, 0.95 mmol), O-(benzotriazol-1-yl)-N,N,N',N'-tetramethyluronium hexafluorophosphate (HATU) (357 mg, 0.94 mmol), and N,N-diisopropylethylamine (DIEA) (245 µL, 1.4 mmol) were added thereto to perform reaction at room temperature for 18 hours.

The DMF was distilled away under reduced pressure using a rotary evaporator, and then purification was performed using an LH20 column. Fractions showing UV peak absorbance at 270 nm were collected and concentrated to obtain a concentrated solution. The concentrated solution was dropped into diethyl ether at 0°C to perform reprecipitation to obtain PLS1 (1.7 g) as a target substance.

### <Experimental Example 3: Synthesis of Sarcosine-Poly(Leucine-Aminoisobutyric Acid) (SLA)>

In this experimental example, an amphiphilic substance, sarcosine-poly(leucine-aminoisobutyric acid) (SLA) was synthesized from sarcosine-NCA (Sar-NCA) and poly(leucine-aminoisobutyric acid) (LAI) (Scheme 3).

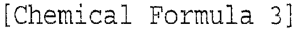

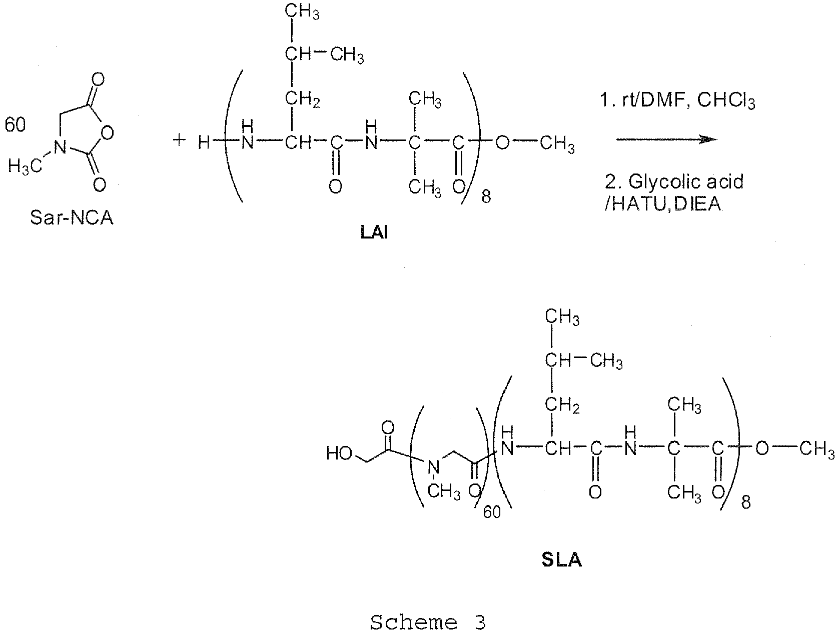

Boc-(Leu-Aib)₈-OMe (600 mg, 0.349 mmol) was added to a mixed solution of 6.0 mL of trifluoroacetic acid (TFA) and 0.6 mL of anisole to remove a Boc group to obtain a trifluoroacetate derivative. The trifluoroacetate derivative was washed with isopropyl ether and dried under vacuum for 2 hours to obtain a dry product. The dry product was dissolved in chloroform and neutralized with a 4 wt% aqueous sodium hydrogen carbonate solution to remove a TFA group. The chloroform solution was concentrated to obtain 420 mg (0.259 mmol) of poly(leucine-aminoisobutyric acid) (LAI) (H- (Leu-Aib)₈-OMe; LAI).

The thus obtained LAI was dissolved in 8.0 mL of a 1:1 (V/V) mixed solution of DMF and HCl₃, and the mixed solution was added to 6.0 mL of a 1:1 (v/v) mixed solution of DMF and HCl₃ containing Sar-NCA (1.11 g, 15.6 mmol) dissolved therein. After the Sar-NCA was consumed by reaction, a resultant reaction solution was cooled to 0°C, glycolic acid (98 mg, 1.30 mmol), HATU (492 mg, 1.30 mmol), and DIEA (338 µL, 1.94 mmol) was added thereto, and then stirring was performed at room temperature for 10 hours. Then, to a resultant reaction solution, glycolic acid (40 mg, 0.52 mmol), HATU (198 mg, 0.52 mmol), and DIEA (135 µL, 0.78 mmol) was added and stirring was performed for 12 hours . After the completion of reaction, a resultant reaction solution was concentrated and purified by gel filtration using Sephadex LH-20 to obtain SLA (186 mg) as a target substance.

### <Experimental Example 4 : Synthesis of Labeled Polymer B (ICG-Labeled Polysarcosine-Poly-L-Lactic Acid; PSL-ICG) (Fluorochrome Introduction Example 1)>

In this experimental example, polysarcosine-poly-L-lactic acid (PSL2) different in chain length from the polysarcosine-poly-L-lactic acid synthesized in Experimental Example 2 was synthesized, and the PLS2 was further labeled with ICG to obtain a labeled amphiphilic substance, polysarcosine-poly-L-lactic acid (PSL-ICG) (Scheme 4).

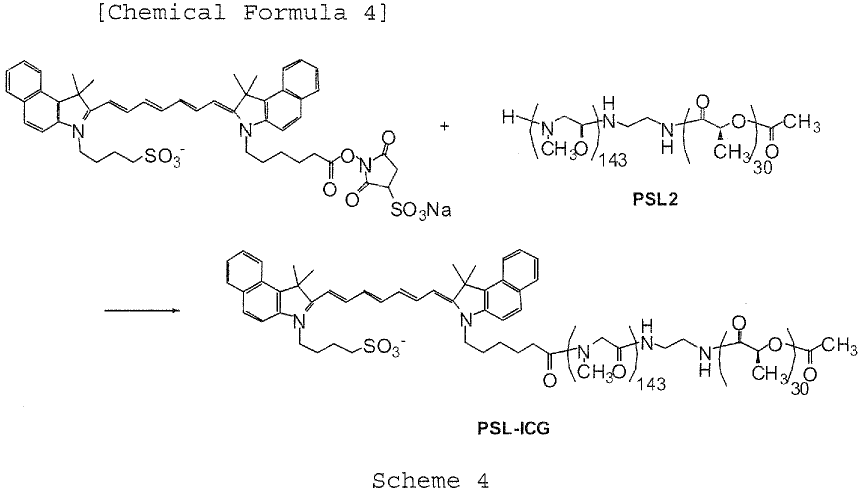

An amphiphilic substance, polysarcosine-poly-L-lactic acid (PLS2) was synthesized in the same manner as in Experimental Example 2 except that 143 equivalents of Sar-NCA with respect to a-PLA was used. To a DMF solution containing 10 mg (1.0 eq) of the PSL2, a DMF solution containing 1 mg (1.3 eq) of an indocyanine green derivative (ICG-sulfo-OSu) dissolved therein was added, and stirring was performed at room temperature for about 20 hours . Then, the solvent was distilled away under reduced pressure, and purification was performed using an LH20 column to obtain a compound PSL-ICG.

### <Experimental Example 5: Synthesis of Labeled Polymer B (ICG-Labeled Poly-L-Lactic Acid; PLA-ICG) (Fluorochrome Introduction Example 2)>

In this experimental example, an ICG-labeled-poly-L-lactic acid (PLA-ICG) was obtained by labeling the aminated poly-L-lactic acid (a-PLA) obtained in Experimental Example 1 with ICG (Scheme 5).

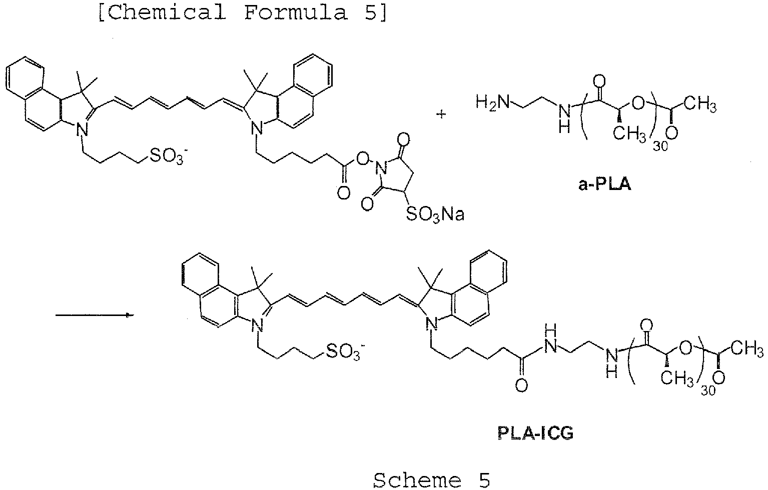

To a DMF solution containing 1.9 mg (1.0 eq) of the a-PLA obtained in Experimental Example 1, a DMF solution containing 1 mg (1.3 eq) of an indocyanine green derivative (ICG-sulfo-OSu) dissolved therein was added, and stirring was performed at room temperature for about 20 hours. Then, the solvent was distilled away under reduced pressure, and purification was performed using an LH20 column to obtain a

### compound PLA-ICG.

### <Experimental Example 6: Synthesis of Labeled Polymer B (DY750-Labeled Poly-L-Lactic Acid; PLA-DY750) (Fluorochrome Introduction Example 3)>

In this experimental example, DY750-labeled poly-L-lactic acid (PLA-DY750) was obtained by labeling the aminated poly-L-lactic acid (a-PLA) obtained in Experimental Example 1 with DY750 (Scheme 6).

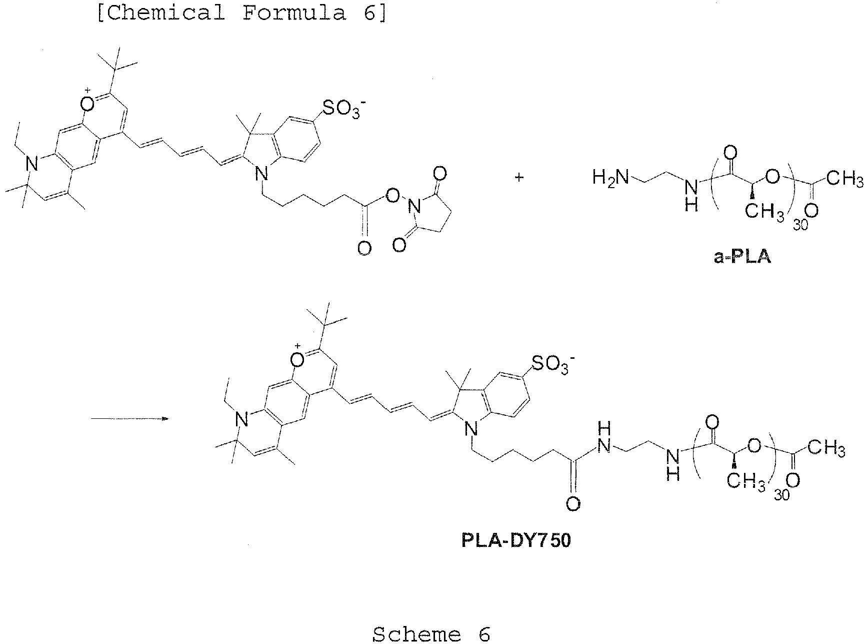

To a DMF solution containing 2.1 mg (1.0 eq) of the a-PLA obtained in Experimental Example 1, a DMF solution containing 1 mg (1.3 eq) of DY750 NHS-ester (manufactured by Dyomics) dissolved therein was added, and stirring was performed at room temperature for about 20 hours. Then, the solvent was distilled away under reduced pressure, and purification was performed using an LH20 column to obtain a compound PLA-ICG.

### <Experimental Example 7: Synthesis of DY776-Labeled Poly(Leucine-Aminoisobutyric Acid) (LAI-DY776) (Fluorochrome Introduction Example 4)>

In this experimental example, DY776-labeled poly(leucine-aminoisobutyric acid) (LAI-DY776) was obtained by labeling poly(leucine-aminoisobutyric acid) (LAI) with DY776 (Scheme 7).

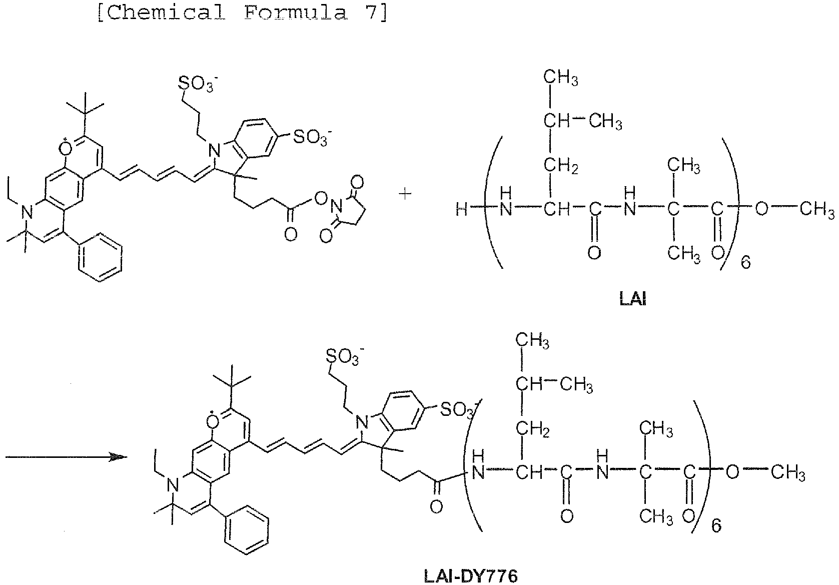

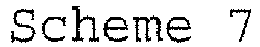

Poly(leucine-aminoisobutyric acid) (Leu-Aib)₆; LAI) was synthesized by a sequential synthesis method using N-t-butoxycarbonyl leucine (Boc-Leu) and aminoisobutyric acid methyl ester (Aib-OMe). To a DMF solution containing 20 mg (5 eq) of the thus obtained LAI, a DMF solution containing 2.5 mg (1 eq) of DY776 NHS-ester (manufactured by Dyomics) and 5.8 mg (5 eq) of HATU dissolved therein was added The reaction solution was cooled to 0°C, and then 10 µL of DIEA was added thereto, and further stirred at room temperature for about 12 hours. Then, the solvent was distilled away under reduced pressure, and purification was performed using an LH20 column to obtain a compound AIL-DY776.

Experimental Example 8: Synthesis of ICG-Labeled Polysarcosine (PS-ICG) (Fluorochrome Introduction Example 5)>

In this experimental example, ICG-labeled polysarcosine (PS-ICG) was obtained by labeling polysarcosine (PSar) with ICG (Scheme 8).

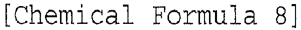

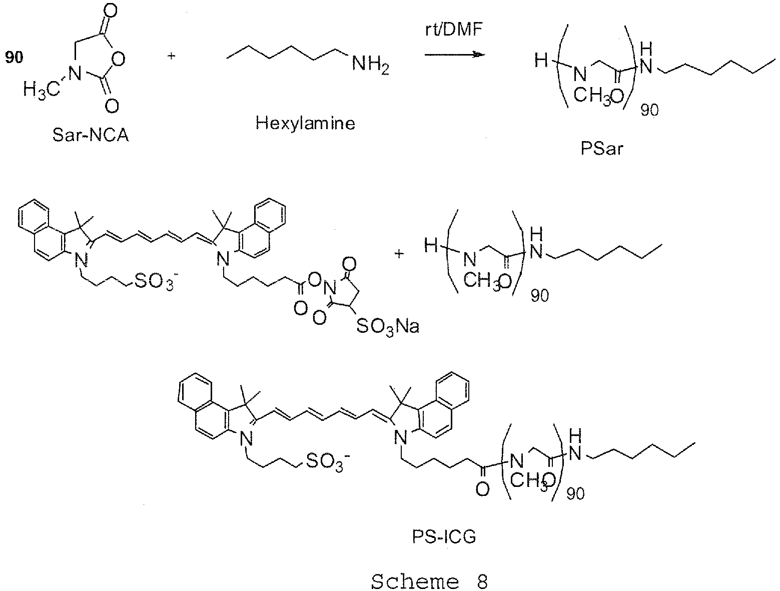

To Hexylamine (3.03 mg, 29.9 µmol) served as a polymerization initiator, Sar-NCA was added in an amount of 90 equivalents with respect to the hexylamine, and dissolved in DMF (15 mL) under Ar gas atmosphere to perform polymerization reaction at room temperature to obtain polysarcosine (Psar). After 24 hours, a resultant reaction solution was dropped into cold diethyl ether (150 mL) to obtain a white precipitate. The obtained white precipitate was collected by centrifugation and dried under reduced pressure. The obtained PSar was dissolved in DMF, mixed with a DMF solution containing 27.8 mg of ICG-sulfo-OSu dissolved therein was added thereto, and was then stirred at room temperature for about 20 hours. Then, the solvent was distilled away under reduced pressure, and purification was performed using an LH20 column to obtain a compound PS-ICG.

In the following Example 1, Comparative Example 1, Comparative Example 2, and Comparative Example 3, molecular assemblies (nanoparticles) that can be used as molecular probes for fluorescence imaging were prepared using a carrier agent (amphiphilic block polymer A1) and a labeling agent (labeled polymer B) shown in Table 1.

**[Table 1]**

| | Entry | Preparation Method | Carrier Agent | Labeling Agent |
|---|---|---|---|---|
| Example 1 | P1 | film method | PSL1 (polymer A1) | PSL-ICG (polymer B) |
| | P1(FD) | film method (*) | PSL1 (polymer A1) | PSL-ICG (polymer B) |
| | P2 | film method | PSL1 (polymer A1) | PLA-ICG (polymer B) |
| | P3 | film method | PSL1 (polymerA1) | PLA-DY750 (polymer B) |
| Comparative Example 1 | P5 | film method | PSL1 (polymer A1) | LAI-DY776 |
| Comparative Example 2 | P4 | injection method | SLA | PLA-ICG |
| | P6 | injection method | SLA | LAI-DY776 |
| Comparative Example 3 | P7 | film method | PSL1 (polymer A1) | ICG |
| | P8 | film method | PSL1 (polymer A1) | PS-ICG |

| | | | | |
|---|---|---|---|---|
| (*) with performing freeze-drying | | | | |

### <Example 1: Production of A1/B-Based Lactosome Nanoparticles (P1, P1(FD), P2, and P3>

In this example, as A1/B-based lactosome nanoparticles, lactosome nanoparticles P1, P1(FD), P2, and P3 were produced using a labeled polylactic acid (labeled polymer B) or a polylactic acid-based labeled amphiphilic block polymer (labeled polymer B).

Carrier agents (amphiphilic block polymer A1) and labeling agents (labeled polymers B) shown in Table 1 were each dissolved in chloroform to prepare their chloroform solutions (0.2 mM). These chloroform solutions were mixed together in a glass container so that the molar ratio between the carrier agent (polymer A1) and the labeling agent (polymer B) was 200 : 3. Then, the solvent was distilled away under reduced pressure to form a film containing the carrier agent (polymer A1) and the labeling agent (polymer B) on an inner wall surface of the glass container. Then, water or a buffer solution was added to the glass container having the film formed therein to disperse the film, and the sonication was performed at 60°C for 30 minutes to obtain a dispersion liquid of nanoparticles P1, P2, or P3.

Further, the dispersion liquid of nanoparticles P1 was frozen with liquid nitrogen, and allowed sublimation to occur under reduced pressure to obtain a freeze-dried product. Water was again added to the freeze-dried product to obtain P1(FD).

### <Comparative Example 1: Production of Lactosome Nanoparticles (P5) Containing Neither A2 Nor B>

In this comparative example, lactosome nanoparticles P5 using a labeled peptide were produced as lactosome nanoparticles containing neither the hydrophobic polymer A2 nor the labeled polymer B (lactosome nanoparticles containing neither A2 nor B). More specifically, a dispersion liquid of nanoparticles P5 was obtained in the same manner as in Example 1 except that the carrier agent (amphiphilic block polymer A1) and the labeling agent (labeled peptide) shown in Table 1 were used.

### <Comparative Example 2: Production of Peptosome Nanoparticles (P4 and P6)>

In this comparative example, peptosome nanoparticles P4 and P6 using a peptide-based amphiphilic polymer were produced as peptosome nanoparticles.

A chloroform solution (0.2mM) of a labeling agent shown in Table 1 was prepared, and a part of the chloroform solution containing 9 nmol of the labeling agent was transferred into a test tube, and the solvent was distilled away under reduced pressure. Then, 3.56 mg (600 nmol) of a carrier agent shown in Table 1 was added to the test tube, and then 90 µL of trifluoroethanol (TFE) was further dropped into the test tube to dissolve the carrier agent therein to obtain a TFE solution.

In another test tube, 1 mL of water (or a buffer solution) was placed, and stirred with a magnetic stirrer (600 rpm) in an ice bath. Then, 60 µL of the TFE solution was added to the water (or buffer solution) at once to be dispersed, and a stirring was performed for 30 minutes in an ice bath to obtain a dispersion liquid of nanoparticles P4 or P6.

The thus obtained dispersion liquid of nanoparticles was purified by column chromatography using Sephacryl S-100 .

### <Comparative Example 3: Production of Lactosome Nanoparticles (P7 and P8) Containing Neither A2 Nor B>

In this comparative example, lactosome nanoparticles P7 and P8 using a non-polymer-based labeled compound or a labeled polysarcosine were produced as lactosome nanoparticles containing neither the hydrophobic polymer A2 nor the labeled polymer B.

A chloroform solution (0.2 mM) of a carrier agent (amphiphilic block polymer A1) shown in Table 1 and a chloroform solution (0.2 mM) of ICG as a labeling agent were prepared. Then, these chloroform solutions were mixed together in a glass container so that the molar ratio between the carrier agent (polymer A1) and the labeling agent was 200:3. Then, the solvent was distilled away under reduced pressure to form a film containing the carrier agent (polymer A1) and the labeling agent on an inner wall surface of the glass container. Then, water or a buffer solution was added to the glass container having the film formed therein to disperse the film, and the sonication was performed at 60°C for 30 minutes to obtain a dispersion liquid of nanoparticles P7 .

Separately, a chloroform solution (0.2 mM) of a carrier agent shown in Table 1 was prepared. Then, the solvent was distilled away under reduced pressure to form a film containing the carrier agent on an inner wall surface of the glass container. Then, an aqueous PS-ICG solution (1 µM) was added to the glass container having the film formed therein so that the molar ratio between the carrier agent (polymer A1) and PS-ICG 200:3. Sonification was performed at 60°C for 30 minutes to obtain a dispersion liquid of nanoparticles P8.

Using the obtained nanoparticles P1 to P8 and P1(FD) as molecular probes, a fluorescence imaging test of cancer-bearing mice prepared by subcutaneous transplantation of human tumor cells into their shoulders was performed.

### <Example 2: Fluorescence Imaging Test 1 of Subcutaneous Cancer Using A1/B-Based Lactosome Nanoparticles (P1 to P3)>

Cancer-bearing mice were prepared by subcutaneous transplantation of human cancer cells in the following manner.

Seven-week-old Balb/c nu/nu mice (CLEA) were prepared as animals, and 5×10⁵ human cancer cells per 0.05 mL were subcutaneously transplanted into the left shoulder of each of the mice and 1×10⁶ human cancer cells per 0.1 mL were subcutaneously transplanted into the right shoulder of each of the mice. Cancer tissue was allowed to grow to a size of 3 to 7 mm for two weeks, and then these mice were subjected to an imaging test in the following manner.

Each of the tumor-bearing mice was anesthetized by isoflurane, and then 0.1 mL (0.1 nmol/body) of the dispersion liquid of nanoparticles P1, P2, or P3 was administered as a molecular probe from their tail vein. After the completion of administration of the probe dispersion liquid, fluorescence images of the whole body of the mouse were picked up with time. The images of the whole body were picked up from five directions, that is, from all the directions of the left abdomen, left side of the body, back, right side of the body, and right abdomen of the mouse. The fluorescent agent was excited by light having a wavelength of 785 nm, and fluorescence having a wavelength of around 845 nm was measured with time.

The results of the imaging test are shown in Fig. 1 and named as "P1", "P2", and "P3". Each of the results "P1" to "P3" in Fig. 1 includes, from left, the result of measurement of the mouse from the direction of its right side of the body after lapses of 3, 6, and 24 hours from the tail vein injection of the nanoparticles and the result of measurement of the mouse from the above-described five directions after a lapse of 24 hours. In Fig. 1, the difference in fluorescence intensity is represented by different color tones, and an area marked with a circle represents a cancer site, and an area marked with a square represents liver site.

### <Comparative Example 4: Fluorescence Imaging Test of Subcutaneous Cancer Using Lactosome Nanoparticles P5 Containing Neither A2 Nor B>

A fluorescence imaging test was performed in the same manner as in Example 2 except that P5 was used as a molecular probe. The thus obtained images are shown in Fig. 1 and named as "P5".

### <Comparative Example 5: Fluorescence Imaging Test of Subcutaneous Tumor Using Peptosome Nanoparticles P4 and P6>

A fluorescence imaging test was performed in the same manner as in Example 2 except that P5 was used as molecular probe. The thus obtained images are shown in Fig. 1 and named as "P4" and "P6".

### <Comparison of Result of Subcutaneous Cancer Fluorescence Imaging Test (A1/B-Based Lactosomes v.s. Lactosome Nanoparticles Containing Neither A2 Nor B, Peptosomes)>

A comparison was made among the results shown in Fig. 1 obtained by measuring the mice from the direction of their right side of the body after lapses of 3, 6, and 24 hours from the tail vein injection of the nanoparticles.

In the case of using the nanoparticles P1, P2, or P3, it was confirmed that fluorescence from a cancer and fluorescence from throughout the body were both detected after a lapse of 3 hours from the tail vein injection, but the intensity of the fluorescence from sites other than a cancer was rapidly reduced thereafter.

On the other hand, in the case of using the nanoparticles P4, it was confirmed that the nanoparticles (peptosomes) produced using a peptide-based amphiphilic polymer accumulated in liver and the fluorescent agent was transferred from liver to intestinal tract and then slowly excreted from the body.

In the case of using the nanoparticles P5 or P6, it was confirmed that fluorescence from a cancer and fluorescence from liver were observed after a lapse of 3 hours from the tail vein injection and then the intensity of the fluorescence from a cancer and the intensity of the fluorescence from liver were both increased.

A comparison was made among the results shown in Fig. 1 obtained by observing the mice from five directions after a lapse of 24 hours from the tail vein injection of the nanoparticles.

In the case of using the nanoparticles P1, P2, or P3, it was confirmed that the fluorescent agent hardly accumulated in sites other than cancers.

On the other hand, in the case of using the nanoparticles P4, it was confirmed that fluorescence from liver and fluorescence from intestinal tract were detected even after a lapse of 24 hours. The reason for this is considered as follows. The polylactic acid-modified fluorescent agent itself is easily metabolized in liver, but the polypeptide-based amphiphilic polymer constituting the nanoparticles is likely to accumulate in liver, which reduces the ratio of the nanoparticles accumulating in cancers.

In the case of using the nanoparticles P5, in addition to fluorescence from tumors, fluorescence from liver located at the center of the body was detected in all the images measured from five directions. The reason for this is considered as follows. The polypeptide modifying the fluorescent agent is not easily metabolized in liver and therefore accumulate in liver. In the case of using the nanoparticles P6, it can be considered that the nanoparticles P6 are more likely to accumulate in liver because both the nanoparticle and the fluorescent agent use a peptide-based polymer.

### <Comparison of Fluorescence Intensity between Subcutaneous Cancer and Background (A1/B-Based Lactosomes v.s. Lactosome Nanoparticles Containing Neither A2 Nor B, Peptosomes>

A comparison of fluorescence intensity at cancer measured from the direction of right side of the body of the mouse and fluorescence intensity at liver as a background was made among the cases of using the nanoparticles P1 to 6. The result is shown in Fig. 2. In Fig. 2, the horizontal axis represents the time (h) that has elapsed from the tail vein injection of the nanoparticles and the vertical axis represents the ratio of fluorescence intensity at cancer to fluorescence intensity at liver.

In the cases of using the nanoparticles P1, P2, and P3, the fluorescence intensity ratios after a lapse of 24 hours from the tail vein injection were 1.81, 1.66, and 2.07, respectively. On the other hand, in the cases of using the nanoparticles P4, P5, and P6, the fluorescence intensity ratios after a lapse of 24 hours from the tail vein injection were about 1.13, 0.95, and 0.75, respectively.

This result indicates that the use of a lactic acid-based polymer as both an amphiphilic polymer (carrier agent) and a polymer constituting a labeling agent constituting nanoparticles makes it possible to achieve a reduction in accumulation in liver and rapid metabolism of the polylactic acid-modified fluorescent agent. Therefore, according to the present invention, it is possible to perform imaging of cancer sites in a short period of time.

### <Comparative Example 6: Fluorescence Imaging Test of Subcutaneous Cancer Using Lactosome Nanoparticles P7 and P8 Containing Neither A2 Nor B>

Cancer-bearing mice were produced by subcutaneous transplantation of human cancer cells in the same manner as in Example 2.

Each of the cancer-bearing mice was anesthetized by isoflurane, and then 0.1 mL (0.1 nmol/body) of the dispersion liquid of nanoparticles P7 or P8 was administered as a molecular probe from its tail vein. After the completion of administration of the probe dispersion liquid, fluorescence images of the whole body of the mouse were picked up with time from the direction of its abdomen by IVIS200 (manufactured by Xenogen). The fluorescent agent was excited by light ranging from 710 to 760 nm and fluorescence ranging from 810 to 875 nm was measured with time.

The results of the imaging test are shown in Fig. 3 and named as "P7" and "P8". Each of the results "P7" and "P8" in Fig. 3 includes, from left, the result of measurement of the mouse after lapses of 30 minutes, 1 hour, 3 hours, 6 hours, and 9 hours from the tail vein injection of the nanoparticles . In Fig. 3, the difference in fluorescence intensity is represented by different color tones.

As can be seen from Fig. 3, neither the nanoparticles P7 nor the nanoparticles P8 accumulated in cancers. The reason for this is considered as follows. Neither ICG nor the PS-ICG has a polylactic acid chain, and therefore can form a stable molecular assembly with the nanoparticle.

In the case of using the nanoparticles P7, it was confirmed that fluorescence from liver was detected after a lapse of 30 minutes from the tail vein injection and then ICG was rapidly metabolized in intestinal tract and excreted from the body.

On the other hand, in the case of using the nanoparticles P8, it was confirmed that fluorescence from urinary bladder was detected after a lapse of 30 minutes from the tail vein injection and then the PS-ICG was rapidly excreted from the body.

ICG is conventionally used for humans as a contrast agent, and it is known that ICG is metabolized in intestinal tract after accumulation in liver. In the case of the nanoparticles P7, ICG is used alone, and therefore it can be considered that the interaction between ICG and the lactosome is weak and that ICG is not stably held by the lactosome in a living body and therefore exhibits the same behavior as when ICG is administered to a living body by itself.

In the case of the nanoparticles P8, ICG is modified with polysarcosine and is therefore improved in water solubility, thereby avoiding accumulation in liver. However, the water solubility of ICG modified with polysarcosine is too high, and therefore the interaction between ICG modified with polysarcosine and the lactosome is weak, which resulted in rapid excretion through urinary bladder to the outside of the body.

It can be considered from the results "P1" to "P3" and "P7" and "P8" that a labeling group needs to be modified with polylactic acid to allow the labeling group to be stably held by the lactosome.

### <Example 3 : Fluorescence Imaging Test of Subcutaneous Cancer Using Freeze-Dried A1/B-Based Lactosome Nanoparticles P1 (FD)>

Cancer-bearing mice were prepared by subcutaneous transplantation of human cancer cells in the same manner as in Example 2.

Each of the cancer-bearing mice was anesthetized by isoflurane, and then 0.1 mL (0.1nmol/body) of the dispersion liquid of nanoparticles P1 (FD) was administered as a molecular probe from its tail vein. After the completion of administration of the probe dispersion liquid, fluorescence images of the whole body of the mouse were picked up with time. The images of the whole body were picked up from the direction of abdomen of the mouse using IVIS200 (manufactured by Xenogen) . The fluorescent agent was excited by light ranging from 710 to 760 nm, and fluorescence ranging from 810 to 875 nm was measured with time.

The results of the imaging test are shown in Figs. 4 (A) and 4(B). Each of Figs. 4(A) and 4(B) includes, from left, result of measurement of the mouse after lapses of 1, 6, and 24 hours from the tail vein injection of the nanoparticles. In Fig. 4, the difference in fluorescence intensity is represented by different color tones.

As can be seen from Fig. 4, in the case of using the nanoparticles P1 (FD) once freeze-dried and then dispersed in water again, fluorescence was detected at a cancer tissue site (marked with a circle in Fig. 4) as in the case of using the nanoparticles P1, but fluorescence detected in urinary bladder after lapses of 1 hour and 6 hours from the tail vein injection was reduced as compared to the case of using the nanoparticles P1.

The reason for this is considered as follows. By freeze-drying, the carrier agent and the labeling agent dissolved singly in a solvent form molecular assemblies in the process of concentration of the solvent, and therefore the amount of the labeling agent dissolved singly in the solvent can be reduced.

From the result above, it can be considered that freeze-drying is more effective at allowing the labeling agent to be more effectively held by the lactosomes.

### <Example 4: Fluorescence Imaging Test of Liver Cancer Using Al/B-Based Lactosome Nanoparticles P2>

In this example, a fluorescence imaging test of cancer-bearing mice prepared by orthotopic transplantation of human liver tumor cells into their liver was performed using the nanoparticles P2.

The cancer-bearing mice were produced by orthotopic tansplantation of human liver tumor cells (HepG2) in the following manner.

Seven-week-old Balb/c nu/nu mice (CLEA) were prepared as animals, and 1 × 10⁶ HepG2 cells having a luciferase gene introduced therein per 0 .1 mL were tansplanted into the liver of each of the mice. Cancer tissue was allowed to grow to a size of 1 to 2 mm for one week, and then each of the mice was subjected to an imaging test in the following manner.

Each of the cancer-bearing mice was anesthetized by isoflurane, and then 0.1 mL (0.1 nmol/body) of the molecular probe dispersion liquid was administered from its tail vein. After the completion of administration of the probe dispersion liquid, fluorescence images of the whole body of the mouse were picked up with time. The fluorescent agent was excited by light having a wavelength of 785 nm, and fluorescence having a wavelength of around 845 nm was measured with time. Further, in order to determine the position of cancer tissue, 0.2 mL (2 mg/body) of luciferin was intraperitoneally administered to the mouse after a lapse of 48 hours from the beginning of measurement and luminescence derived from luciferase specifically expressed in cancer cells was measured using IVIS200 manufactured by Xenogen.

The results of the imaging test are shown in Figs. 5 (A) and (B). Fig. 5(A) includes the fluorescence and luminescence images of the whole body of the cancer-bearing mouse measured from the direction of its abdomen after a lapse of 48 hours from the administration of the probe dispersion liquid from its tail vein. As can be seen from the result shown in Fig. 5(A), signals were detected in the same area in liver in both cases of fluorescence measurement and luminescence measurement. Fig. 5(B) includes the photograph (bright-field image) of liver extirpated from the cancer-bearing mouse and the luminescence and fluorescence images of the extirpated liver. As a result of extirpation of liver, it was confirmed that cancer tissue (marked with a circle in Fig. 5B) having a size of about 1 × 1 mm was present in the surface thereof. Further, in the extirpated liver, an area where the strongest fluorescence was detected was the same as that where luminescence derived from cancer cells was detected.

The results above indicate that the molecular probe according to the present invention is less likely to accumulate in liver and is rapidly metabolized in liver and therefore can be used even in fluorescence imaging of tumors present in the surface of liver. A conventional method for diagnostic imaging using chemiluminescence needs genetic modification, but it has been confirmed that according to the present invention, liver cancers can be detected by the method of externally administering a near-infrared fluorescent agent, which enables diagnostic imaging of tumors to be performed without the need for genetic modification.

### <Example 5: Fluorescence Imaging Test of Lung Cancer Using A1/B-Based Lactosome Nanoparticles P2>

In this example, a fluorescence imaging test of cancer-bearing mice prepared by orthotopic transplantation of human lung cancer cells into their lung was performed.

The cancer-bearing mice were produced by orthotopic transplantation of human lung cancer cells (H441) in the following manner.

Six-week-old Balb/c nu/nu mice (CLEA) were produced as animals, and 1 × 10⁵ H441 cells, having a luciferase gene introduced therein, per 0.1 mL were transplanted into the lung of each of the mice. Cancer tissue was allowed to grow to a size of 5 to 10 mm for 11 days, and then each of the mice was subjected to an imaging test in the following manner.

Each of the cancer-bearing mice was anesthetized by isoflurane, and then 0.1 mL (0.1 nmol/body) of the molecular probe dispersion liquid was administered from its tail vein. After a lapse of 48 hours from the administration of the probe dispersion liquid, 0.2 mL (2 mg/body) of luciferin was intraperitoneally administered to the mouse. Thereafter, lung was extirpated from the mouse, and luminescence derived from luciferase specifically expressed in cancer cells and fluorescence were measured using IVIS200 manufactured by Xenogen. The fluorescent agent was excited by light having a wavelength of 785 nm and fluorescence having a wavelength of 845 nm was measured.

The results of the imaging test are shown in Fig. 6. Fig. 6 includes, from left, the bright-field image, luminescence image, and fluorescence image of the lung extirpated from the cancer-bearing mouse. As a result of extirpation of lung, it was confirmed that the cancers grew and occupied almost the half of the left lung. Further, in the extirpated lung, an area where fluorescence was detected was the same as that where luminescence derived from cancer cells was detected.

As can be seen from the results of Examples 4 and 5, the lactosome according to the present invention accumulates not only in a subcutaneously-transplanted cancer but also in an orthotopically-transplanted liver cancer or lung cancer, and therefore it can be considered that the lactosome according to the present invention is useful for drug delivery.

### <Experimental Example 9: Synthesis of Hydrophobic Polymers A2 (Polylactic Acids with Different Optical Activities)>

In this experimental example, five types of polylactic acid derivatives with different optical activities, PLLA, PDLA, rac-PLA, PDLLA (14:1), and PDLLA (10:5) (hereinafter, these polylactic acid derivatives are simply referred to as "polylactic acids") shown in Table 2 were synthesized by changing the blending ratio between optical isomers of lactide.

**[Table 2]**

| | | L-lactide | D-lactide | DL-lactide |
|---|---|---|---|---|
| Run | | mol ratio | | |
| 1 | PLLA | 100 | 0 | 0 |
| 2 | PDLA | 0 | 100 | 0 |
| 3 | *rac*-PLA | 0 | 0 | 100 |
| 4 | PDLLA | 14 | 0 | 1 |
| 5 | PDLLA | 10 | 0 | 5 |

### (1. Synthesis of PLLA)

Poly-L-lactic acid (PLLA) was synthesized using L-lactide (compound 1) and N-carbobenzoxy-1,2-diaminoethane hydrochloride (compound 2) (Scheme 9).

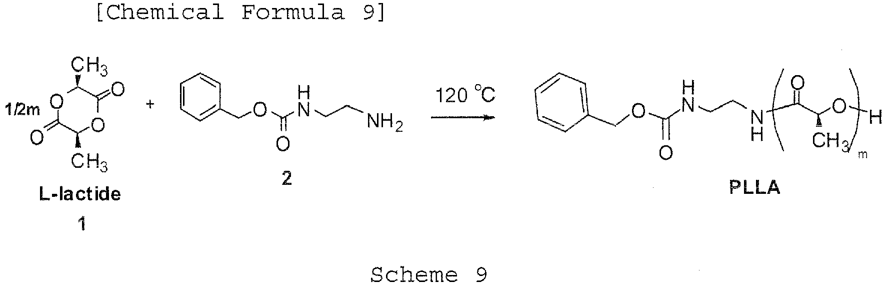

To N-carbobenzoxy-1,2-diaminoethane hydrochloride (compound 2) (400 mg, 2.06 mmol) served as a polymerization initiator, a dispersion liquid obtained by dispersing tin octanoate (22.25 mg) in toluene (1.0 mL) was added. The toluene was distilled away under reduced pressure, and then L-lactide (compound 1) (4.45 g, 30.9 mmol) was added to perform polymerization reaction at 120°C under Ar gas atmosphere. After a lapse of 8 hours, the reaction container was air-cooled to room temperature to obtain a yellowish-white solid. The yellowish-white solid was dissolved in a small amount of dimethylformamide (about 10 mL) and purified using an LH20 column. Then, fractions showing absorption at 270 nm were collected and concentrated to obtain a concentrated liquid, and the concentrated liquid was dissolved in chloroform. The resulting chloroform solution was dropped into cold methanol (100 mL) to obtain a white precipitate. The white precipitate was collected by centrifugation and dried under reduced pressure to obtain a compound PLLA.

### (2. Synthesis of PDLA)

A compound PDLA was obtained in the same manner as in the synthesis of PLLA described above in (1) except that the L-lactide (compound 1) was changed to D-lactide.

### (3. Synthesis of rac-PLA)

An oily compound rac-PLA was obtained in the same manner as in the above (1), except that the L-lactide (compound 1) was changed to DL-lactide, that includes collecting and concentrating fractions showing absorption at 270 nm to obtain a concentrate, azeotropically boiling the obtained concentrate with diethyl ether twice or three times and then performing drying under reduced pressure.

### (4. Synthesis of PDLLA (14:1))

A compound PDLLA (14 :1) was obtained in the same manner as in the above (1) except that the L-lactide (compound 1) was changed to a 14:1 (molar ratio) mixture of L-lactide and DL-lactide.

### (5. Synthesis of PDLLA (10:5))

An oily compound PDLLA (10:5) was obtained in the same manner as in the above (1), except that the L-lactide (compound 1) was changed to a 10:5 (molar ratio) mixture of L-lactide and DL-lactide, that includes collecting and concentrating fractions showing absorption at 270 nm to obtain a concentrate, azeotropically boiling the obtained concentrate with diethyl ether twice or three times and then performing dryiing under reduced pressure.

The average polymerization degree, molecular weight, and property of each of these synthesized polylactic acids are shown in Table 3. The average polymerization degree of each of the polylactic acids was determined from the result of ¹H NMR measurement by using a signal derived from a terminal benzene ring as a reference.

**[Table 3]**

| Run | | Average Polymerization Degree (m) | Molecular Weight (Mw) | Property |
|---|---|---|---|---|
| 1 | PLLA | 33 | 2572.21 | white solid |
| 2 | PDLA | 32 | 2500.15 | white solid |
| 3 | rac-PLA | 32 | 2500.15 | oil |
| 4 | PDLLA(L:DL=14:1) | 31 | 2428.09 | white solid |
| 5 | PDLLA(L:DL=10:5) | 26 | 2067.79 | oil |

### <Experimental Example 10: Heat Characteristics of Polylactic Acids>

In this experimental example, the five types of polylactic acids with different optical purities, PLLA, PDLA, rac-PLA, PDLLA (14:1), and PDLLA (10:5) synthesized in Experimental Example 9 (hereinafter, these five types of polylactic acids with different optical purities are sometimes collectively referred to as "PLAs") were analyzed by a differential scanning calorimeter (DSC) to determine the heat characteristics of these polylactic acids.

About 2 mg of a sample was weighed and placed in a standard aluminum sample container (alumina crimp cell), and the sample container was covered with a lid and the lid was crimped by a sealer/crimper (SSC-30) to hermetically seal the samplecontainer. Alumina was used as a reference substance. Measurement was performed using DSC-60 (manufactured by Shimadzu Corporation) at a temperature rise of 10°C/min in a temperature range of 30 to 150°C.

Fig. 7(a) shows the result of first heating and Fig. 7(b) shows the result of second heating. In Figs. 7(a) and 7(b), the horizontal axis represents temperature (°C) and the vertical axis represents heat flow (mW).

In Fig. 7(a), no exothermic peaks were observed in all the polylactic acids. After the first heating, each of the polylactic acids was rapidly cooled from 150°C, which was sufficiently higher than a crystallization temperature, to a temperature equal to or less than a glass transition point. As a result or second heating, as shown in Fig. 7(b), PLLA, PDLA, and PDLLA (14:1), each of which had been obtained as a white solid, exhibited a crystallization temperature as the peak temperature of an exothermic peak and a melting temperature as the peak temperature of an endothermic peak. From the result, these three types of polylactic acids were found to be crystalline polymers. On the other hand, in the cases of the oily compounds, rac-PLA and PDLLA (10: 5), neither an exothermic reaction nor an endothermic reaction was observed. From the result, these polylactic acids were found to be non-crystalline polymers.

### <Example 6: Particle Size Control of A1/A2-Based Lactosome Nanoparticles>

In this example, the polylactic acid (PLA) synthesized in Experimental Example 9 was used as the hydrophobic polymer A2 and blended with the polylactic acid-based amphiphilic polymer A1 to prepare polylactic acid-blended lactosomes , and the particle size of the lactosomes was controlled by changing the blending ratio of the polylactic acid A2 to the polylactic acid-based amphiphilic polymer A1. In this case, each of the five types of polylactic acids (PLAs) synthesized in Experimental Example 9, that is, PLLA, PDLA, rac-PLA, PDLLA (14:1), and PDLLA (10:5) with different optical purities was used as the hydrophobic polymer A2 to prepare five types of polylactic acid-blended lactosomes.

Fig. 8 is a schematic diagram indicating that a polylactic acid-blended lactosome is prepared by blending the polylactic acid A2 (PLA) to the polylactic acid-based amphiphilic polymer A1.

Further, in this example, in the preparation of each of the five types of polylactic acid-blended lactosomes, molecular assemblies respectively having different ratio between polylactic acid-based amphiphilic polymer A1 and the polylactic acid A2 were prepared. A change in the particle size of the obtained molecular assemblies was determined.

The polylactic acid-based amphiphilic polymer A1 PLLA₃₃-PSar₁₆₃) and the polylactic acid A2 (PLA) in blending ratios shown in Table 4 were prepared in test tubes so that the total amount of the polylactic acid-based amphiphilic polymer A1 and the polylactic acid A2 was 9 mg, and then 1.5 mL of chloroform was added to dissolve the polylactic acid-based amphiphilic polymer A1 and the polylactic acid A2 . Then, the chloroform was removed by reduced-pressure drying to form a polymer film on an inner wall of the test tube. Then, 3 mL of pure water or a buffer solution was added to the test tube, and sonication was performed at 55°C to convert the film into particles. In this way, A1/A2-based (polylactic acid-blended) lactsome nanoparticles were obtained.

It is to be noted that, for the purpose of reference, lactosome nanoparticles whose polylactic acid-based amphiphilic polymer A1 content was 100 % (lactosome nanoparticles containing neither A2 nor B) were prepared in the same manner as described above except that the polylactic acid A2 was not used.

**[Table 4]**

| | | | | | | |
|---|---|---|---|---|---|---|
| PLLA-Psar (mol%) | 100 | 95 | 90 | 75 | 50 | 25 |
| 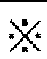 PLA (mol%) | 0 | 5 | 10 | 25 | 50 | 75 |

| | | | | | | |
|---|---|---|---|---|---|---|
| 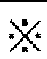 **PLA =PLLA , PDLA, rac-PLA , PDLLA (L:DL=14:1), PDLLA (L:DL=10:5)** | | | | | | |

The particle sizes of the lactosome nanoparticles different in the blending ratio of the polylactic acid A2 from each other were measured and examined by a DLS (Dynamic Light Scattering) method using a dynamic light scattering measuring device (Zetasizer Nano manufactured by Malvern Instruments) . As a result, it has been found that the particle size of the lactosome nanoparticles can be continuously controlled from 30 to 130 nm by changing the blending ratio of the polylactic acid A2 except when PDLA is used as the polylactic acid A2 and that such continuous particle size control can be achieved irrespective of optical purity (Fig. 9).

It is to be noted that in a case where PDLA was used as the polylactic acid A2, the measurement result was different from those of other cases. The reason for this is considered that it was difficult to form stable particles. One of factors responsible for this includes a balance with the optical purity of hydrophobic block of the polylactic acid-based amphiphilic polymer A1 used in this example. That is, it can be considered that the formation of a stereocomplex between an L-lactic acid block chain of the polylactic acid-based amphiphilic block polymer A1 and a D-lactic acid chain of the polylactic acid A2 is one of factors responsible for inhibition of formation of stable particles.

In the following Comparative Example 7 and Example 7, nanoparticles were prepared by allowing a compound to be encapsulated in lactosomes (lactosomes containing neither A2 nor B) or in polylactic acid-blended lactosomes (A1/A2-based lactosomes) as carriers by a film method, and the thus obtained nanoparticles were evaluated.

The compound encapsulated in the carrier was pyrene. Pyrene is common to substance often used as fluorescent agent to be encapsulated in molecular imaging probes or used as anticancer agents to be encapsulated in DDS probes, in that they are low-molecular aromatic hydrophobic compounds and are fluorescent materials. So pyrene is suitable as a model compound of such a substance.

### <Comparative Example 7: Encapsulation of Pyrene in Lactosome Nanoparticles Containing Neither A2 Nor B (Lactosome Nanoparticles Composed of Polylactic Acid-Based Amphiphilic Polymer A1)>

In this comparative example, a low-molecular weight hydrophobic compound (pyrene) was encapsulated in lactosome nanoparticles composed of the polylactic acid-based amphiphilic polymer A1.

Pyrene was added to 9 mg of the polylactic acid-based amphiphilic polymer A1 (PLLA₃₅-PSar₁₅₂) so that its blending ratio was 0 mol%, 5 mol% (6.8 µg), 10 mol% (13.5 µg), 25 mol% (33.8 µg), 50 mol% (67.5 µg), 75 mol% (101.3 µg), 100 mol% (135 µg), 200 mol% (270 µg), 400 mol % (540 µg), 600 mol% (810 µg), 800 mol% (1080 µg), or 1000 mol% (1350 µg), and a resultant mixture was dissolved in 1.5 mL of chloroform. The chloroform was distilled away under reduced pressure to form a thin transparent film on an inner wall of a test tube. Then, 3 mL of ultrapure water was added to the test tube, and the sonication was performed at 55°C for 30 minutes. Then, centrifugation at 2600 g for 15 minutes was performed to obtain a supernatant. The supernatant was filtered through a 0.20 µm filter (Millex®-LG manufactured by Nihon Millipore K.K.) to obtain a dispersion liquid of lactosome nanoparticles having pyrene encapsulated therein.

### <Example 7: Encapsulation of Pyrene in A1/A2-Based Lactosome Nanoparticles (Polylactic Acid-Blended Lactosome Nanoparticles Composed of Polylactic Acid-Based Amphiphilic Polymer A1 and Polylactic Acid A2)>

A1/A2-based lactosomes (PLLA/lactosomes) were prepared by blending the polylactic acid-based amphiphilic polymer A1 (PLLA₃₁-PSar₁₅₀) and the polylactic acid A2 (PLLA) in a molar ratio of 1:1 (total amount: 9 mg) and A1/A2-based lactosomes (rac-PLA/lactosomes) were prepared by blending the polylactic acid-based amphiphilic polymer A1 (PLLA₃₁-PSar₁₅₀) and the polylactic acid A2 (rac-PLA) in a molar ratio of 1:1 (total amount: 9 mg). Dispersion liquids of A1/A2-based (polylactic acid-blended) lactosome nanoparticles having pyrene encapsulated therein were obtained in the same manner as in Comparative Example 7 except that pyrene was added in amounts respectively of 0 µg, 10 µg, 50 µg, 100 µg, 500 µg, and 1000 µg.

The absorption spectra and fluorescence spectra of the lactosomes containing neither A2 nor B and having pyrene encapsulated therein obtained in Comparative Example 7 and the A1/A2-based (polylactic acid-blended) lactosomes having pyrene encapsulated therein obtained in Example 7 were measured in the following manner.

### <Comparison of Absorption Spectra between Lactosomes Containing Neither A2 Nor B and Having Pyrene Encapsulated Therein and A1/A2-Based Lactosomes Having Pyrene Encapsulated Therein>

The absorption spectrum of each of the lactosomes obtained in Comparative Example 7 and Example 7 was measured to determine whether pyrene was encapsulated respectively therein.

Measurement of absorption spectra was performed by an ultraviolet-visible spectrophotometer (UVmini-1240 manufactured by shimadzu Corporation).

### (In the case of Comparative Example 7)

The absorption spectra of the lactosomes containing neither A2 nor B (lactosomes composed of polylactic acid amphiphilic polymer A1) having pyrene encapsulated therein, obtained in Comparative Example 7, were measured. In this case, as a control test, a solution containing only pyrene (9 mg) was produced in the same manner and the absorption spectrum of the solution was measured.

The absorption spectra of the lactosome nanoparticles containing neither A2 nor B and having pyrene encapsulated therein are shown in Fig. 10. In Fig. 10, the horizontal axis represents wavelength (nm) and the vertical axis has arbitrary unit (Abs) (the same applies for Fig. 11 shown later).

Fig. 10(a) shows measurement results when the concentration of pyrene was in the range of 0 to 75 mol%, and Fig. 10(b) shows measurement results when the concentration of pyrene was in the range of 100 to 1000 mol%. As shown in Fig. 10, pyrene-derived absorption was hardly observed in the case of the control solution prepared by using only pyrene, but pyrene-derived absorption was observed only in the cases where pyrene was blended with the polylactic acid-based amphiphilic polymer A1. From the result, it was confirmed that pyrene had been encapsulated in the lactosome nanoparticles containing neither A2 nor B. That is, pyrene, which is a low-molecular weight hydrophobic compound not dissolved in H₂O by itself (solubility [H₂O] : 7.2 × 10⁻⁴ mmol/l), could be dispersed in H₂O by blending with the lactosomes containing neither A2 nor B.

### (In the case of Example 7)

The absorption spectra of the A1/A2-based lactosomes (polylactic acid-blended lactosomes composed of the polylactic acid-based amphiphilic polymer A1 and the polylactic acid A2) having pyrene encapsulated therein obtained in Example 7 were measured.

The absorption spectra of the A1/A2-based (polylactic acid-blended) lactosome nanoparticles having pyrene encapsulated therein are shown in Fig. 11. Fig. 11(a) shows the absorption spectra of the A1/A2-based lactosomes (PLLA/lactosomes) composed of the polylactic acid-based amphiphilic polymer A1 (PLLA₃₁-PSar₁₅₀) and the polylactic acid A2 (PLLA) and Fig. 11(b) shows the absorption spectra of the A1/A2-based lactosomes (rac-PLA/lactosomes) composed of the polylactic acid-based amphiphilic polymer A1 (PLLA₃₁-PSar₁₅₀) and the polylactic acid A2 (rac-PLA). As a result, it has been confirmed that pyrene can be encapsulated also in the A1/A2-based (polylactic acid-blended) lactosome nanoparticles.

### <Comparison of Fluorescence Spectra between Lactosomes Containing Neither A2 Nor B and Having Pyrene Encapsulated Therein and A1/A2-Based Lactosomes containing Pyrene Encapsulated Therein>

First, fluorescence spectra of pyrene encapsulated in the lactosomes were measured in order to compare, among the lactosomes, the influence of difference in the crystallinity of a hydrophobic core on interaction with pyrene.

Fluorescence measurement was performed by a spectrophotometer (RF-5300PC manufactured by Shimadzu Corporation) under conditions where the scanning range was 300 nm to 500 nm, the excitation wavelength (λex) was 336 nm, the excitation slit width was 3.0 nm or 1.5 nm, and the emission slit width was 3.0 nm or 1.5 nm.

### (In the case of Comparative Example 7)

The fluorescence spectra of the lactosomes (lactosomes composed of the polylactic acid-based amphiphilic polymer A1) containing neither A2 nor B and having pyrene encapsulated therein obtained in Comparative Example 7 were measured.

More specifically, the fluorescence spectra of the lactosomes containing neither A2 nor B and having pyrene encapsulated therein obtained in Comparative Example 7 in ratios of 0 mol%, 10 mol% (13.5µg), 25 mol% (33.8 µg), 50 mol% (67.5 µg), 75 mol% (101.3 µg), 100 mol% (135 µg), 200 mol% (270 µg), and 400 mol% (540 µg) were respectively measured. At this time, in the case of the lactosomes having 50 mol% of pyrene and lactosomes having 70 mol% of pyrene 200-fold dilution was performed, and in the case of the other lactosomes 100-fold dilution was performed before measurement of fluorescence spectrum.

The fluorescence spectra of the lactosome nanoparticles containing neither A2 nor B and having pyrene encapsulated therein are shown in Fig. 12. In Fig. 12, the horizontal axis represents wavelength (nm) and the vertical axis represents the intensity of fluorescence (the same applies for Fig. 13 described later).

Fig. 12(a) shows measurement results when the concentration of pyrene was in the range of 0 to 75 mol%, and Fig. 12(b) shows measurement results when the concentration of pyrene was in the range of 100 to 1000 mol%.

### (In the case of Example 7)

The fluorescence spectra of the A1/A2-based lactosomes (polylactic acid-blended lactosomes composed of the polylactic acid-based amphiphilic polymer A1 and the polylactic acid A2) having pyrene encapsulated therein obtained in Example 7 were measured.

More specifically, in the case of all A1/A2-based (polylactic acid-blended) lactosomes having pyrene encapsulated therein obtained in Example 7 600-fold dilution was performed before measurement of fluorescence spectrum.

The fluorescence spectra of the A1/A2-based (polylactic acid-blended) lactosomes having pyrene encapsulated therein are shown in Fig. 13. Fig. 13(a) shows measurement results of the A1/A2-based lactosomes (PLLA/lactosomes) composed of the polylactic acid-based amphiphilic polymer A1 (PLLA₃₁-PSar₁₅₀) and the polylactic acid A2 (PLLA), and Fig. 13(b) shows measurement results of the A1/A2-based lactosomes (rac-PLA/lactosomes) composed of the polylactic acid-based amphiphilic polymer A1 (PLLA₃₁-PSar₁₅₀) and the polylactic acid A2 (rac-PLA).

Then, the relation between the pyrene concentration and the fluorescence intensity at 373 nm was compared between Example 7 and Comparative Example 7 based on respective measured fluorescence spectra of the lactosomes, and the comparison results are shown in Fig. 14. In Fig. 14, the horizontal axis represents the percentage of the weight of pyrene with respect to the total weight of the polymer(s) constituting the lactosome and the vertical axis represents the intensity of fluorescence. Fig. 14(a) shows the relationship between the concentration of pyrene and the intensity of fluorescence of the lactosome containing neither A2 nor B and having pyrene encapsulated therein obtained in Comparative Example 7, and Fig. 14 (b) shows the relationships between the concentrations of pyrene and the intensities of fluorescence of the A1/A2-based (polylactic acid-blended) lactosomes having pyrene encapsulated therein obtained in Example 7.

As can be seen from Fig. 14(a), in the cases of the lactosomes containing neither A2 nor B obtained in Comparative Example 7, it is indicated that formation of particles is inhibited due to an increase in the concentration of pyrene. That is, this result indicates that the lactosomes containing neither A2 nor B obtained in Comparative Example 7 cannot contain pyrene in high concentration.

On the other hand, in Fig. 14(b), the intensity of fluorescence is increased as the pyrene content is increased. That is, this result indicates that in the cases of the A1/A2-based (polylactic acid-blended) lactosomes obtained in Example 7, particles are stably formed even when the concentration of pyrene is increased. The reason for this is considered that the polylactic acid A2 blended in the lactosomes in Example 7 increases the volume of an interacting region with a hydrophobic compound, pyrene, that is, the volume of a hydrophobic core, and therefore a larger amount of a low-molecular weight hydrophobic compound can be encapsulated than in the lactosomes of Comparative Example 7 containing neither A2 nor B.

Further, as can be seen from Fig. 14(b), there is a difference depending on the optical purity of the polylactic acid contained. More specifically, the fluorescence intensities of the A1/A2-based (polylactic acid-blended) lactosomes containing PLLA are higher than those of the A1/A2-based (polylactic acid-blended) lactosomes containing rac-PLA. This indicates that a difference in the crystallinity of a hydrophobic core has an influence on the interaction between a hydrophobic core and pyrene.

However, it has been found that, in either case, the A1/A2-based (polylactic acid-blended) lactosome obtained in Example 7 can contain pyrene in high concentration, which makes it possible to stably disperse pyrene in water at high concentration.

The above results indicate that the A1/A2-based (polylactic acid-blended) lactosomes described herein may also be used as probes for molecular imaging by encapsulating another signal agent instead of the above-mentioned pyrene therein or as probes for DDS by encapsulating another drug instead of the above-mentioned pyrene therein. The specific examples of such probes in which adriamycin for an anticancer drug and paclitaxel for an anticancer drug were respectively used will be described later in Examples 13 and 15.

### <Example 8: Fluorescence Imaging Test of Subcutaneous Cancer Using A1/B-Based Lactosome Nanoparticles and A1/A2/B-Based Lactosome Nanoparticles>

In this Example, a fluorescence imaging test of cancer-bearing mice prepared by subcutaneous transplantation of human cancer cells was performed using, as molecular probes for fluorescence imaging, A1/B-based lactosome nanoparticles containing the amphiphilic block polymer A1 (PSL1) and the labeled polymer B (PLA-ICG) as constituent polymers and A1/A2/B-based lactosome nanoparticles further containing the hydrophobic polymer A2 (PLLA) as a constituent polymer.

The A1/B-based lactosome nanoparticles and the Al/A2/B-based lactosome nanoparticles were produced in the following manner.

A chloroform solution (0.2 mM) containing the amphiphilic block polymer A1 (PSL1) and the label ed polymer B (PLA-ICG) in a molar ratio of 200:3 was prepared, and the dispersion liquid containing the hydrophobic polymer A2 (PLLA) in concentrations of 0, 10, 25, and 50 mol% in the chloroform solution was prepared. Then, the solvent was distilled away under reduced pressure to form a film on an inner wall of each of the glass containers. Then, water or a buffer solution was added to the glass container having the film formed therein to disperse the film, and sonication was performed at 60°C for 30 minutes to obtain a dispersion liquid of nanoparticles.

The cancer-bearing mice were produced by subcutaneous transplantation of human cancer cells in the following manner.

Seven-week-old Balb/c nu/nu mice (CLEA) were prepared as animals, and 1×10⁶ human cancer cells per 0.05 mL were subcutaneously transplanted into the right thigh of the mice. Czncer tissue was allowed to grow to a size of about 10 mm for two weeks, and then these mice were subjected to an imaging test in the following manner.

Each of the cancer-bearing mice was anesthetized by isoflurane, and then 0.05 mL (0.1 nmol/body) of the dispersion liquid of nanoparticles as a molecular probe was administered from its tail vein. After the completion of administration of the probe dispersion liquid, fluorescence images of the whole body of the mouse were picked up with time. The images of the whole body of were picked up from five directions, that is, from all the directions of the left abdomen, left side of the body, back, right side of the body, and right abdomen of the mouse. The fluorescent agent was excited by light having a wavelength of 785 nm, and fluorescence having a wavelength of about 845 nm was measured with time.

The thus obtained fluorescence images measured after a lapse of 24 hours are shown in Figs. 15(a) to 15(d). In Fig. 15, the difference in fluorescence intensity is represented by different color tones. Fig. 16 is a graph showing changes in the ratio of fluorescence intensity between cancer site (right thigh) and background (left thigh) (Intensity ratio of fluorescence (tumor/background)) with respect to time (Time (h)) till 48 hours after administration.

It was confirmed from the results shown in Fig. 15 that in both cases where the hydrophobic polymer A2 (polylactic acid) was not blended and where the hydrophobic polymer A2 (poly-L-lactic acid) was blended to change a particle size, the lactosome nanoparticles accumulated in a cander site. Further, as can be seen from the results shown in Fig. 16, the ratio of fluorescence intensity between cancer site and background exceeds 1 after a lapse of 24 hours even when 50 mol% of hydrophobic polymer A2 (polylactic acid) was blended, and therefore it can be said that the administered probes accumulated in acancer. Further, the ratio of fluorescence intensity between cancer site and background was higher when the hydrophobic polymer A2 (polylactic acid) was blended in a concentration of 25 mol% than when the hydrophobic polymer A2 (polylactic acid) was not blended. This indicates that there is a possibility that the ability of the nanoparticles to accumulate in a cancer site can be enhanced by changing the particle size of the nanoparticles.

### <Example 9: Fluorescence Imaging Test 2 of Subcutaneous Cancer Using A1/B-Based Lactosome Nanoparticles (P2)>

The cancer-bearing mice were prepared by subcutaneous transplantation of human cancer cells in the following manner.

Six-week-old Balb/c nu/nu mice (CLEA) were prepared as animals, and 1×10⁶ mouse ascites cancer cells per 0.05 mL were subcutaneously transplanted into the right thigh of the mice. Cancer tissue was allowed to grow to a size of about 15 mm for two weeks, and then these mice were subjected to an imaging test in the following manner.

Each of the Cancer-bearing mice was anesthetized by isoflurane, and then the dispersion liquid of the A1/B-based lactosome nanoparticles P2 (0.1 nmol (ICG)/body) as a molecular probe was administered from its tail vein. After the completion of administration of the probe dispersion liquid, fluorescence images of the whole body of the mouse were picked up with time. The images of the whole body were picked up from five directions, that is, from all the directions of the right back, right side of the body, abdomen, left side of the body, and left back of the mouse. The fluorescent agent was excited by light having a wavelength of 785 nm, and fluorescence having a wavelength of about 845 nm was measured with time.

The thus obtained fluorescence images measured after a lapse of 24 hours from administration of the A1/B-based lactosomes are shown in Fig. 17(a). In Fig. 17, the difference in fluorescence intensity is represented by different color tones.

### <Comparative Example 8: Fluorescence Imaging Test of Subcutaneous Cancer Using Liposome Nanoparticles>

The same procedure was performed in the same manner as in Example 9 except that a dispersion liquid of liposome nanoparticles having ICG-labeled human serum albumin (HSA-ICG) encapsulated therein as a molecular probe was used in an amount of 0.05 mL (6 nmol(ICG)/body).

These liposome nanoparticles were produced in the following manner.

First, 20 mg of dipalmitoyl phosphatidylcholine (DPPC), 10 mg of cholesterol, 2 mg of diacetyl phosphate, and 32 mg of sodium cholate were dissolved in 3 mL of a 1:1 mixed solution of chloroform and methanol, and then the solvent was distilled away under reduced pressure to obtain a lipid membrane . Then, 3 mL of a TAPS buffer solution (pH 8.4) was added to the lipid membrane, and sonicatation was performed to prepare a micelle solution (solution 1). Then, 20 mg of human serum albumin (HSA) was mixed with 1 mg of ICG-Sulfo-OSu, and 3 mL of a TAPS buffer solution (pH 8.4) was added thereto to perform reaction at 37°C for 3 hours to obtain a reaction. After the completion of the reaction, unreacted ICG-Sulfo-OSu was removed from the reaction solution by a centrifugal concentrator to prepare ICG-labeled human serum albumin (HSA-ICG) (solution 2). The solution 1 and the solution 2 were mixed together, and the resulting mixture was subjected to solvent replacement with a TAPS buffer solution (pH 8.4) using a centrifugal concentrator to prepare liposomes having HSA-ICG encapsulated therein.

The thus obtained fluorescence images measured after a lapse of 6 hours from administration of the liposomes are shown in Fig. 17(b).

### (Comparison of Results of Fluorescence Imaging Test of Subcutaneous CancerS (A1/B-Based Lactosomes v.s. Liposomes))

A comparison was made between the fluorescence images shown in Fig. 17(a) measured after 24 hours from administration of the A1/B-based lactosome nanoparticles (Example 9) and the fluorescence images shown in Fig. 17(b) measured after 6 hours from administration of the liposome nanoparticles (Comparative Example 8). As can be seen from Fig. 17, in the case of the liposomes, a large amount of the fluorescent agent accumulated in liver and abdomen other than in a cancer, but in the case of lactosomes, the fluorescent agent hardly accumulated in organs other than a cancer.

### (Comparison of Fluorescence Intensity between Subcutaneous Cancer and Background (A1/B-Based Lactosomes v.s. Liposomes))

Fig. 18 is a graph showing changes in fluorescence intensity at right thigh cancer ("tumor") site and changes in fluorescence intensity at left thigh ("background") with respect to time in 96 hours after administration of the nanoparticles. Fig. 18(a) shows the result of Example 9 in which the A1/B-based lactosome nanoparticles were administered, and Fig. 18(b) shows the result of Comparative Example 8 in which the peptosome nanoparticles were administered. In Fig. 18, the horizontal axis represents the time (h) that has elapsed after administration of the nanoparticles and the vertical axis represents fluorescence intensity.

As a result, it was found that in the case of the liposomes, a large amount of the fluorescent agent accumulated in liver or abdomen other than a cancer and the ratio of fluorescence intensity between cancer site and background was about 1.95, but in the case of the lactosomes, the fluorescent agent hardly accumulated in organs other than a cancer and the ratio of fluorescence intensity between cancer site and background was about 5.40 which was significantly higher than that measured using the liposomes.

Then, ¹⁸F-labeled poly-L-lactic acids (¹⁸F-PLLA, ¹⁸F-BzPLLA) were synthesized to prepare molecular probes for positron emission tomography.

### <Experimental Example 11: Synthesis of Polylactic Acid Labeled with Tosyl Group>

Bu-PLLA-OH (Bu=n-butyl-) having an average polymerization degree of 30.4 was reacted with 6 molar equivalents of p-toluenesulfonyl chloride (Ts-Cl) in the presence of 0.5 molar equivalent of dimethylaminopyridine (DMAP) . As a result, a target substance labeled with a tosyl group, Bu-PLLA-OTs was obtained in a yield of 90.3% (Scheme 10) .

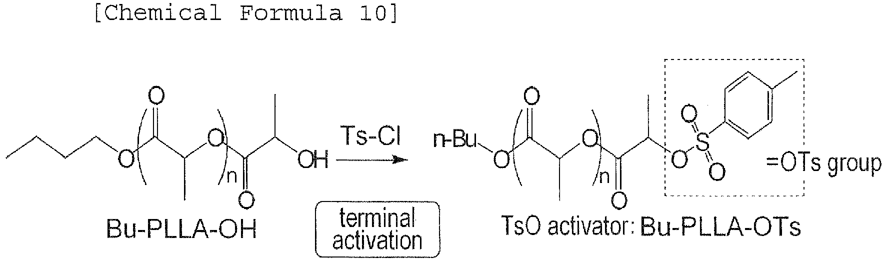

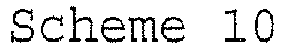

### <Experimental Example 12: Synthesis of Polylactic Acid Labeled with Triflate>

Bu-PLLA-OH (Bu=n-butyl) having an average polymerization degree of 30.4 was reacted with 2 molar equivalents of trifluoromethanesulfonic anhydride (TfO anhydride) in the presence of 2 molar equivalents of pyridine. As a result, a target substance labeled with triflate, Bu-PLLA-OTf was obtained in a yield of 89.0% (Scheme 11).

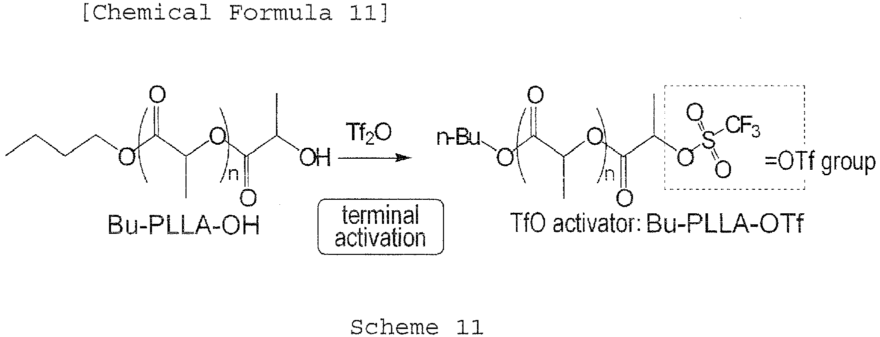

### <Experimental Example 13: Synthesis of Labeled Polymer B (¹⁸F-Labeled Poly-L-Lactic Acid (¹⁸F-PLLA)) >

The Bu-PLLA-OTf obtained in Experimental Example 12 was subjected to ¹⁸F labeling reaction (scheme 12). As a result, the generation of polylactic acid labeled with ¹⁸F, ¹⁸F-PLLA was confirmed by an HPLC equipped with a gamma detector and an absorption spectrometer. Fig. 19 shows the result of HPLC fractionation of ¹⁸F-PLLA.

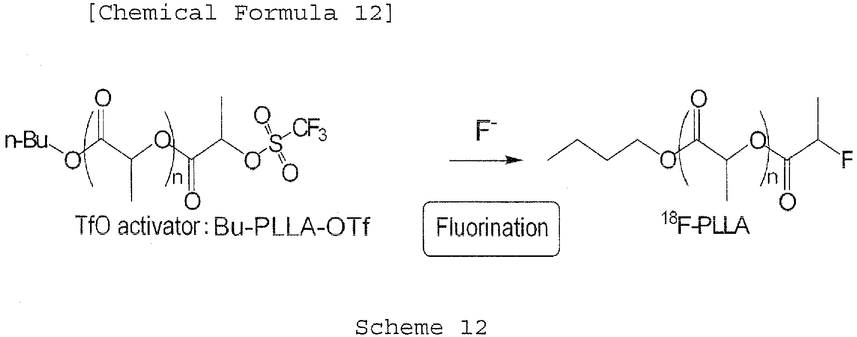

### <Example 10: Production of Molecular Probes for PET (A1/B-Based ¹⁸F-Lactosome Nanoparticles)>

In this example, as molecular probes for PET, A1/B-based ¹⁸F-lactosome nanoparticles were produced using the ¹⁸F - poly-L-lactic acid (¹⁸F-PLLA) obtained in Experimental Example 13. More specifically, A1/B-based ¹⁸F-lactosome nanoparticles were prepared in the same manner as in Example 1 except that the ¹⁸F-labeled poly-L-lactic acid (¹⁸F-PLLA) obtained in Experimental Example 13 was used as the labeled polymer B.

### <Experimental Example 14: Synthesis of Labeled Polymer B (¹⁸F-Labeled Benzoyl Poly-L-Lactic Acid (¹⁸F-BzPLLA)) >

In this experimental example, ¹⁸F-SFB was first synthesized (Scheme 13), and was then reacted with polylactic acid having a terminal amino group to synthesize ¹⁸F-labeled benzoyl polylactic acid (¹⁸F-BzPLLA) (Scheme 14).

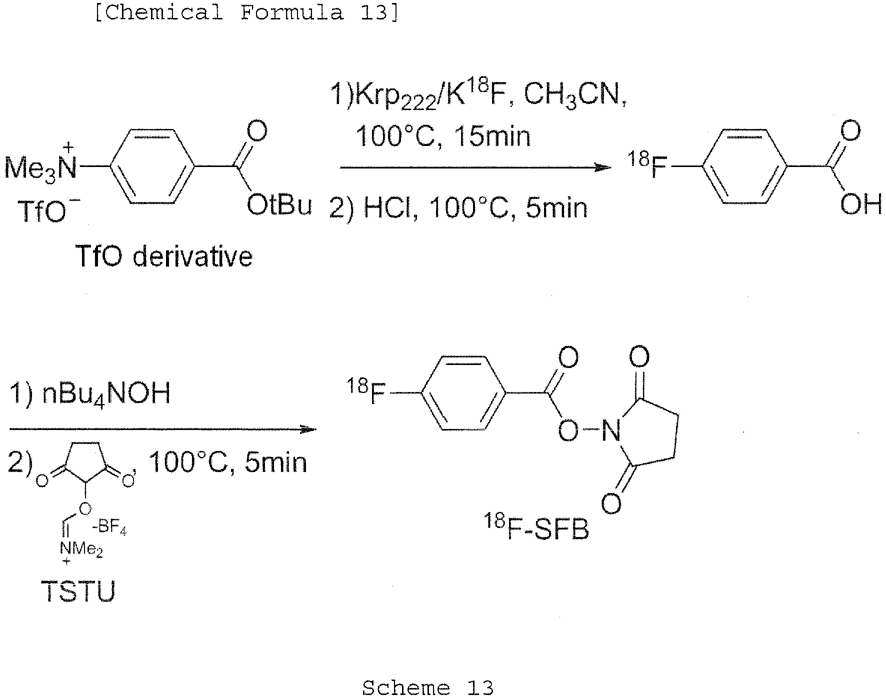

150 µL of dehydrated acetonitrile was mixed with an aqueous ¹⁸F-K₂CO₃ solution in a syringe vial, and the resulting mixture was heated at 110°C and argon gas was blown into the syringe vial to distill away the solvent. Then, 500 µL of dehydrated acetonitrile was further added, and the solvent was removed at 110°C by blowing argon gas into the syringe vial, which was repeated three times to prepare K¹⁸F/Kryptofix 222. The ¹⁸F-labeled reagent K¹⁸F/Kryptofix 222, 11.2 mCi to 13.7 mCi (414.4 MBq to 506.9 MBq)) and 1.0 mg of a TfO derivative were added to 300 µL of anhydrous acetonitrile, and the resulting mixture was heated at 100°C for 15 minutes. Then, the mixture was cooled to room temperature, and 150 µL of 1M hydrochloric acid was added thereto, and the resulting mixture was heated at 100°C for 5 minutes. Then, the resulting reaction liquid was mixed with 6 mL of distilled water, and the resulting diluted liquid was subjected to a Sep-Pak light C18 cartridge. Radioactivity loaded onto the Sep-Pak light C18 cartridge was eluted with acetonitrile, and the resulting eluate was mixed with 50 µL of a 10% tetrabutylammonium hydroxide-methanol solution and was heat-dried by blowing argon gas thereinto. The thus obtained residue was mixed with 10 mg of O-(N-succinimidyl)-N,N,N',N'-tetramethyluroniumtetrafluor oborate (TSTU) and 300 µL of acetonitrile, and was then heated at 100°C for 5 minutes to perform active ester introduction reaction. The resulting reaction liquid was diluted with 10 mL of 5 v/v% acetic acid, and the resulting diluted liquid was subjected to a Sep-Pak light C18 cartridge, and then the Sep-Pak light C18 cartridge was washed with 1 mL of distilled water. Then, radioactivity loaded onto the Sep-Pak light C18 cartridge was eluted with 300 µL of acetonitrile. The total synthesis time was 80 minutes, the radiochemical yield was 26 to 16.6% (with decay correction), and the radiochemical purity was 95%.

The result of HPLC of the eluate obtained by simple purification using a Sep-Pak C18 cartridge is shown in Fig. 20(a).

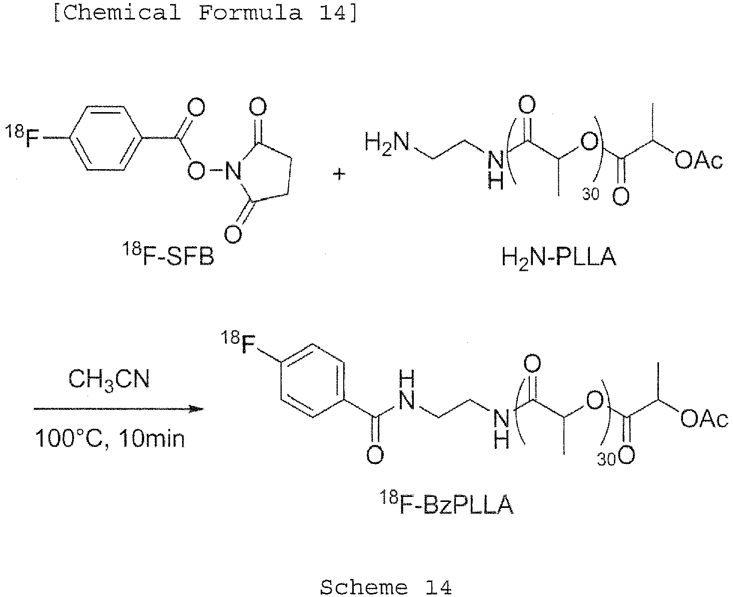

1.2 mCi of ¹⁸F-SFB was added to 300 µL of acetonitrile containing 1.0 mg of H₂N-PLLA dissolved therein, and the resulting reaction solution was heated at 100°C in an oil bath to perform labeling reaction for 10 minutes. After the completion of the reaction, the resulting reaction solution was purified by HPLC using Asahipak GF-310HQ (7.6 × 300 mm) as a column and acetonitrile as an eluent to obtain a target substance, ¹⁸F-BzPLLA. The total synthesis time was 120 minutes, the radiochemical yield was 20%, and the radiochemical purity was 100%. The result of HPLC is shown in Fig. 20(b).

### <Example 11: Preparation of Al/B-Based Lactosome Nanoparticles>

In this example, Al/B-based lactosome nanoparticles were produced using the ¹⁸F-labeled benzoyl poly-L-lactic acid (¹⁸F-BzPLLA) synthesized in Experimental Example 14 as the labeled polymer B and PLLA₃₀-PSar158 as the amphiphilic polymer A1.

A polymer film was produced using 9 mg or 3 mg of PLLA₃₀-PSar₁₅₈ (amphiphilic polymer A1) , and then 100 µCi (0.3 mL) of an acetonitrile solution of the ¹⁸F-BzPLLA (labeled polymer B) was added to the polymer film. The resulting mixture was heated at 110°C and Ar gas was blown thereinto to distill away acetonitrile. Then, the resulting residue was mixed with 2 mL of water and sonicated under heating at 50°C for 10 minutes to prepare particles.

The encapsulation of the ¹⁸F-BzPLLA (labeled polymer B) in the lactosomes was confirmed by gel filtration using Sephacryl S-100HR (inner diameter: 1.5 cm, height: 22 cm) as a column. More specifically, elution was performed using 1/15 mol/L phosphate buffer (pH 7.4), and 40 drops of eluate (about 2 cc, flow rate: about 5 seconds per drop) were collected per fraction, and radioactivity and UV absorbance of each of the fractions were measured. The result of gel-filtration elution in the case of using 9 mg of the amphiphilic polymer Al is shown in Fig. 21(a) , and the result of gel-filtration elution in the case of using 3 mg of the amphiphilic polymer Al is shown in Fig. 21(b).

As a result, 98% or more (in the case of using 9 mg of the polymer film) or 99% or more (in the case of using 3 mg of the polymer film) of radioactivity of the ¹⁸F-BzPLLA was eluted into the same fractions as that containing the lactosomes. On the other hand, radioactivity hardly remained in the column. From the result, it was confirmed that the ¹⁸F-BzPLLA was encapsulated in the lactosomes.

### <Example 12: PET Measurement Test of Cancer-bearing Mice Using Al/B-Based Lactosome Nanoparticles>

In this example, a PET measurement test of cancer-bearing mice was performed using the Al/B-based ¹⁸F-lactosome nanoparticles obtained in Example 10 as molecular probes for PET.

In addition to the cancer-bearing mice for PET measurement, cancer-bearing mice for autopsy were also prepared. Both of the cancer-bearing mice were prepared in the same manner as in Example 8.

The ¹⁸F-lactosomes were administered to both the cancer-bearing mice for PET measurement and the cancer-bearing mice for autopsy, and then each experiment was started. More specifically, the ¹⁸F-lactosomes containing about 10 MBq of radioactivity were administered per mouse for PET measurement, and the ¹⁸F-lactosomes containing about 5 MBq of radioactivity were administered per mouse for autopsy. Pet measurement (n=2) was continuously performed for 30 minutes just after administration, and was then further performed for 20 minutes after lapses of 1 hour and 10 minutes, 2 hours and 10 minutes, 3 hours and 10 minutes, 4 hours and 10 minutes, 5 hours and 10 minutes, and 6 hours and 10 minutes from administration, respectively.

The result of PET measurement is shown in Fig. 22. As shown in Fig. 22, signals were observed throughout the body of the mouse even after a lapse of 6 hours after administration. Further, specific accumulation of the ¹⁸F-lactosomes in a particular organ was not observed. It is to be noted that relatively strong signals were observed in spine. The reason for this is considered that the ¹⁸F-labeled polylactic acid having a reduced molecular weight accumulates in bones. Further, the result of PET measurement also shows that the lactosome according to the present invention has high blood retention.

The analysis of signal intensity throughout the body of the mouse was performed by autopsy, and the analytical result is shown in Fig. 23. As shown in Fig. 23, many of the ¹⁸F-lactosomes accumulated in heart, lungs, and bones, but specific accumulation in a cancer site was observed. Further, the signal intensity of blood was high. This indicates that the lactosome according to the present invention has high blood retention.

In the following examples, molecular probes for drug delivery were prepared and their anticancer activities were examined.

### <Example 13 : Anticancer Activity Test 1 against Human Cancer Cells Using A1/A2-Based Lactosome Nanoparticles>

In this example, an anticancer activity test against human cancer cells was performed using A1/A2-based lactosome nanoparticles having adriamycin encapsulated therein as molecular probes for drug delivery.

First, 11. 4 mg of adriamycin (ADM) used as an anticancer agent was dissolved in 50 mL of milliQ water, and 50 mL of chloroform was added thereto to obtain a chloroform solution. Then, 0.04 mL of a 1N aqueous NaOH solution was dropped into the chloroform solution under stirring to dehydrochlorinate the ADM, and the dehydrochlorinated ADM was extracted into a chloroform phase. The chloroform phase was collected by a separating funnel, and then an appropriate amount of sodium sulfate was added for dehydration. The sodium sulfate was removed by filtration, and drying under reduced pressure to obtain 10.6 mg of the dehydrochlorinated adriamycin (ADM/-HCl) .

Lactosomes having the ADM/-HCl encapsulated therein were prepared by forming particles, so that the ADM/-HCl mixed was used in the amount of 5 wt% with respect to that of lactosomes which contains PLLA₃₀-PSar₁₅₀ (amphiphilic polymer A1) and PLLA₃₀ (hydrophobic polymer A2) blended therein in ratio of 50 mol% with respect to that of amphiphilic polymer A1.

Encapsulation of adriamycin in the lactosomes was confirmed by separately preparing A1/A2/B-based lactosome nanoparticles having adriamycin encapsulated therein and subjecting the Al/A2/B-based lactosome nanoparticles to gel filtration. The A1/A2/B-based lactosome nanoparticles for use in confirming encapsulation of adriamycin were prepared by mixing also polylactic acid labeled with a fluorescent agent (ICG) as the labeled polymer B, at once, in addition to the above polymers A1 and A2 and the ADM/-HCl.

The result of gel filtration purification (Sepharose 4B) of the thus prepared nanoparticles for use in confirming encapsulation of adriamycin is shown in Fig. 24. Fig. 24 (a) shows the result of gel filtration of the A1/A2/B-based lactosome nanoparticles having adriamycin encapsulated therein (horizontal axis: fraction number, vertical axis: absorbance) , and Fig. 24(b) shows the absorption spectrum of fraction No. 11 (horizontal axis: wavelength, vertical axis: absorbance). As shown in Fig. 24, absorption by the fluorescent agent (ICG) and absorption by adriamycin were eluted in the same fraction. From the result, it was confirmed that adriamycin had been encapsulated in the lactosomes.

An anticancer activity test against SUIT-2 cells derived from human pancreas cancer was performed using adriamycin (ADM) as an anticancer agent and lactosomes having dehydrochlorinated adriamycin (ADM/-HCl) encapsulated therein (hereinafter, also referred to as " (ADM/-HC1) /lactosomes") . 1 × 10⁴ SUIT-2 cells/0. 1 mL were cultured in a 5% FBS-Dulbecco's modified Eagle medium on a 96-well plate at 37°C for 24 hours. Then, the ADM and (ADM/-HCl) /lactosomes was added to wells in amount of 10 µL so that final concentrations thereof were respectively in the range of 1 × 10⁻⁶ to 1 × 10⁻² mM, and then the cultivation was performed. After lapses of 24, 48, and 72 hours from the beginning of cultivation, 5 µL of a cell-counting reagent SF (manufactured by Nacalai Tesque) was added to the wells, and the wells were allowed to stand at 37° for 2 hours. Then, the absorbance at 450 nm of was measured and was compared with that of a control containing no reagent to determine cell viability. The result of the anticancer activity test is shown in Fig. 25. In Fig. 25, the horizontal axis represents the concentration of ADM (ADM conc.) and the vertical axis represents cell viability determined by comparison with a control containing no reagent.

### <Example 14: Examination of Amount of Poor-Water-Soluble Reagent Dispersed in Water by Lactosomes>

In this example, how much a poor-water-soluble anticancer agent, paclitaxel (PTX) could be dispersed in H₂O by using A1/A2-based lactosomes was examined.

PLLA₃₁ (hydrophobic polymer A2) was blended with PLLA₃₀-PSar₁₅₀ (amphiphilic polymer A1) in a ratio of 50 mol% with respect to that of the amphiphilic polymer A1 in a chloroform solution so that the total amount of the PLLA and the PLLA₃₀-PSar₁₅₀ was 3 mg, and then PTX was added so that the concentration of PTX was 0.5, 1, 2, 4, 8, 16, or 24 wt%. Then, the solvent was distilled away under reduced pressure to form a film. Then, 3 mL of milliQ water was added to the film, and the sonication was performed at 55°C for 10 minutes to convert the film into particles. The thus obtained solution containing particles was passed through a 0.2 µm filter to prepare an aqueous solution containing A1/A2-based lactosomes having paclitaxel encapsulated therein (PTX/lactosomes). The total amount of the aqueous solution containing PTX/lactosomes was freeze-dried, and then about 1 mg of the freeze-dried product was weighed and dissolved in a DMF solution containing LiBr mixed with DMF in a concentration of 10mM, so that the concentration of the PTX/lactosomes was 10 mg/mL. Then, the amount of PTX was determined by HPLC. HPLC was performed using Asahipak GF-310HQ (7.6 × 300 mm) as a gel filtration column and a solution containing LiBr mixed with DMF in a concentration of 10 mM as a solvent, and the absorbance at 270 nm was measured.

A comparative examination was performed in the same manner as described above except that 3 mg of lactosomes not containing the hydrophobic polymer A2 (lactosomes containing neither A2 nor B) were used.

Separately, PTX was singly dissolved in the above-mentioned solvent (which is a solution containing LiBr mixed with DMF in a concentration of 10 mM) so that the concentration of PTX was 0.1 mg/mLn, and lactosomes (i.e., A1/A2-based lactosomes not having PTX encapsulated therein) were singly dissolved in the above-mentioned solvent (which is a solution containing LiBr mixed with DMF in a concentration of 10 mM) so that the concentration of the lactosomes was 10 mg/ml. Then, 10 µL of the PTX solution and 10 µL of the solution containing lactosomes were analyzed. As a result, it was found that PTX was eluted at around 14.0 min (Fig. 26(b)), and the lactosomes were eluted at around 10.3 min (Fig. 26(a)).

The freeze-dried PTX/lactosomes were also dissolved in the above-mentioned solvent so that the concentration of the PTX/lactosomes was 10 mg/mL, and then 10 µL of the resulting solution was analyzed. As a result, the amphiphilic polymer constituting the PTX/lactosomes was eluted at 10.8 min and PTX was eluted at 14.2 min (Fig. 26(c)). From the result, it was confirmed that the PTX was present in the aqueous PTX/lactosome solution.

Regarding the case that 50 mol% of hydrophobic polymer A2 was contained in the lactosome (i.e., A1/A2-based lactosomes having paclitaxel encapsulated therein (PTX/lactosomes)) and the case that the hydrophobic polymer A2 was not contained in the lactosome (i.e., lactosomes containing neither A2 nor B and having paclitaxel encapsulated therein, prepared for a comparison purpose) , the detected amount of PTX detected with respective to the mixed amount of PTX is shown in Fig. 27.

As shown in Fig. 27, in the case that the hydrophobic polymer A2 was not contained in the lactosome, the detected amount of PTX was 12.8 wt%, whereas the mixed amount of PTX was 16 wt%. Further, when the mixed amount of PTX was increased to 24 wt% , resultant particles did not pass through the 0.2 µm filter.

On the other hand, in the case that 50 mol% of hydrophobic polymer A2 was contained in the lactosome, even when the mixed amount of PTX was 24 wt%, resultant particles passed through the 0.2 mm filter, and the detected amount of PTX was 18. 9 wt%. From the result, it has been confirmed that the lactosomes containing the hydrophobic polymer A2 can contain a larger amount of PTX encapsulated therein as compared to the lactosomes not containing the hydrophobic polymer A2.

### <Example 15 : Anticancer Activity Test 2 against Human Cancer Cells Using Al/A2-Based Lactosome Nanoparticles>

In this example, an anticancer activity test against human cancer cells (SUIT-2 cells derived from Human pancreas cancer) using A1/A2-based lactosomes having paclitaxel encapsulated therein as molecular probes for drug delivery.

The A1/A2-based lactosomes having paclitaxel encapsulated therein (PTX/lactsomes) were prepared in the same manner as in Example 14. In this case, the mixed amount of paclitaxel was 5 wt%.

On the other hand, the same anticancer activity test was performed using, as a commercially-available anticancer agent, paclitaxel (TAXOL® manufactured by Bristol-Myers Squibb) (PTX-i).

1 × 10⁴ SUIT-2 cells/0.1 mL were cultured in a 5% FBS-Dulbecco's modified Eagle medium on a 96-well plate at 37°C for 24 hours. Then, PTX-i or PTX/lactosomes was added to wells in amount of 10 µL so that final concentration thereof were in the range of 0.016 to 2 µM, and then cultivation was performed. After lapses of 24, 48, and 72 hours from the beginning of cultivation, each 5 of a cell-counting reagent SF (manufactured by Nacalai Tesque) was added to the wells, and the wells were allowed to stand at 37° for 2 hours. Then, the absorbance at 450 nm of each well was measured and was compared with that of a control containing no reagent to determine cell viability. The result is shown in Fig. 28. In Fig. 28, the horizontal axis represents the concentration of paclitaxel (PTX conc.) and the vertical axis represents cell viability determined by comparison with a control containing no reagent.

According to the present invention, it is possible to provide a molecular assembly which is less likely to accumulate in tissue other than cancer tissue, is highly safe for a living body, and can be prepared by a simple and safe method and whose particle size can be easily controlled. Therefore, according to the present invention, it is possible to provide a molecular probe useful for molecular imaging and to provide a molecular probe useful for drug delivery.

## Claims

1. A particulate molecular assembly comprising:
an amphiphilic block polymer A1 comprising a hydrophilic block chain having 20 or more sarcosine units and a hydrophobic block chain having 10 or more lactic acid units; and
a labeled polymer B comprising at least 10 or more lactic acid units and a labeling group,
wherein the labeling group of the polymer B is a signal group or a ligand,
wherein the signal group is selected from the group consisting of a near-infrared fluorescent group, a radioactive element-containing group, and a magnetic group,
wherein the ligand is selected from the group consisting of an antibody, an adhesion factor, and a tricarboxylic acid which can coordinate to a transition metal, and
wherein the labeling group of the polymer B is bound either directly or via a spacer group to any site in the polymer part.

2. The particulate molecular assembly according to claim 1, wherein the labeled polymer B is a labeled polylactic acid comprising 10 or more continuous lactic acid units and the iabeiing group as constituent components.

3. The particulate molecular assembly according to claim 1, wherein the labeled polymer B is a labeled amphiphilic block polymer comprising a hydrophilic block chain, a hydrophobic block chain having 10 or more continuous lactic acid units, and the labeling group.

4. The particulate molecular assembly according to any one of claims 1 to 3, which is prepared by a preparation method comprising the steps of:
preparing a solution, in a container, containing the amphiphilic block polymer A1 and the labeled polymer B in an organic solvent;
removing the organic solvent from the solution to obtain a film containing the amphiphilic block polymer A1 and the labeled polymer B on an inner wall of the container; and
adding water or an aqueous solution into the container and ultrasonic treatment is performed to convert the film into particulate molecular assembly to obtain a dispersion liquid of the particulate molecular assembly.

5. The particulate molecular assembly according to claim 4, wherein the preparation method further comprises, after the step of obtaining the dispersion liquid of the particulate molecular assembly, the step in which the dispersion liquid of the particulate molecular assembly is subjected to freeze-drying treatment.

6. The particulate molecular assembly according to claim 1, further comprising:
a hydrophobic polymer A2 having 10 or more lactic acid units.

7. The particulate molecular assembly according to claim 6, wherein the hydrophobic polymer A2 is selected from the group consisting of:
a hydrophobic polymer whose 10 or more lactic acid units are composed of L-lactic acid units;
a hydrophobic polymer whose 10 or more lactic acid units are composed of D-lactic acid units; and
a hydrophobic polymer whose 10 or more lactic acid units are composed of L-lactic units and D-lactic acid units.

8. The particulate molecular assembly according to claim 6 or 7, wherein the amphiphilic block polymer A1 and the hydrophobic polymer A2 are contained in a molar ratio of 10:1 to 1:10.

9. The particulate molecular assembly according to any one of claims 6 to 8, which is prepared by a preparation method comprising the steps of:
preparing a solution, in a container, containing the amphiphilic block polymer A1, the hydrophobic polymer A2, and the labeled polymer B in an organic solvent;
removing the organic solvent from the solution to obtain a film containing the amphiphilic block polymer A1, the hydrophobic polymer A2, and the labeled polymer B on an inner wall of the container; and
adding water or an aqueous solution into the container and ultrasonic treatment is performed to convert the film into particulate molecular assembly to obtain a dispersion liquid of the particulate molecular assembly.

10. The particulate molecular assembly according to claim 9, wherein the preparation method further comprises, after the step of obtaining the dispersion liquid of the particulate molecular assembly, the step in which the dispersion liquid of the particulate molecular assembly is subjected to freeze-drying treatment.

11. The particulate molecular assembly according to any one of claims 1 to 10, which is in the form of a micelle or a vesicle.

12. The particulate molecular assembly according to any one of claims 1 to 11, wherein the signal group is a near-infrared fluorescent group.

13. The particulate molecular assembly according to any one of claims 1 to 11, wherein the signal group is a radioactive element-containing group.

14. The particulate molecular assembly according to any one of claims 1 to 13, further comprising a drug.

15. A molecular probe for molecular imaging comprising the particulate molecular assembly according to any one of claims 1 to 14.

16. A molecular probe for drug delivery system comprising the particulate molecular assembly according to claim 14.

17. A method for preparing a particulate molecular assembly comprising the step of:
preparing, by using an amphiphilic block polymer A1 comprising a hydrophilic block chain having 20 or more sarcosine units and a hydrophobic block chain having 10 or more lactic acid units and a hydrophobic polymer A2 having 10 or more lactic acid units, a molecular assembly comprising the amphiphilic block polymer A1 and the hydrophobic polymer A2; and
controlling a particle size of the molecular assembly by adjusting an amount of the hydrophobic polymer A2 used with respect to an amount of the amphiphilic block polymer A1 used.

18. The method for preparing the particulate molecular assembly according to claim 17, wherein the hydrophobic polymer A2 is selected from the group consisting of:
a hydrophobic polymer whose 10 or more lactic acid units are composed of L-lactic acid units,
a hydrophobic polymer whose 10 or more lactic acid units are composed of D-lactic acid units; and
a hydrophobic polymer whose 10 or more lactic acid units are composed of L-lactic acid units and D-lactic acid units.

19. The method for preparing the particulate molecular assembly according to claim 17 or 18, wherein the amphiphilic block polymer A1 and the hydrophobic polymer A2 are used in a molar ratio of 10:1 to 1:10.

20. The method for preparing the particulate molecular assembly according to any one of claims 17 to 19, further comprising using a labeled polymer B comprising at least 10 or more lactic acid units and a labeling group to prepare the molecular assembly comprising the amphiphilic block polymer A1 and the hydrophobic polymer A2 and, in addition, the labeled polymer B,
wherein the labeling group of the polymer B is a signal group or a ligand,
wherein the signal group is selected from the group consisting of a near-infrared fluorescent group, a radioactive element-containing group, and a magnetic group,
wherein the ligand is selected from the group consisting of an antibody, an adhesion factor, and a tricarboxylic acid which can coordinate to a transition metal, and
wherein the labeling group of the polymer B is bound either directly or via a spacer group to any site in the polymer part.

21. The molecular probe according to claim 15 for use in a method of molecular imaging of a living body.

22. The molecular probe according to claim 16 for use in a method of drug delivery to a living body.

23. The particulate molecular assembly according to claim 14, wherein the drug is paclitaxel.

## Patentansprüche

1. Teilchenförmige molekulare Zusammenstellung, umfassend:
ein amphiphiles Blockpolymer A1, umfassend eine hydrophile Blockkette mit 20 oder mehr Sarkosineinheiten und eine hydrophobe Blockkette mit 10 oder mehr Milchsäureeinheiten; und
ein markiertes Polymer B, umfassend mindestens 10 oder mehr Milchsäureeinheiten und eine Markierungsgruppe,
wobei die Markierungsgruppe des Polymers B eine Signalgruppe oder ein Ligand ist,
wobei die Signalgruppe aus der Gruppe, bestehend aus einer Nahinfrarotfluoreszierenden Gruppe, einer Gruppe, enthaltend ein radioaktives Element, und einer magnetischen Gruppe, ausgewählt ist,
wobei der Ligand aus der Gruppe, bestehend aus einem Antikörper, einem Adhäsionsfaktor und einer Tricarbonsäure, welche an ein Übergangsmetall koordinieren kann, ausgewählt ist, und
wobei die Markierungsgruppe des Polymers B entweder direkt oder über eine Abstandshaltergruppe an eine beliebige Stelle in dem Polymerteil gebunden ist.

2. Teilchenförmige molekulare Zusammenstellung nach Anspruch 1, wobei das markierte Polymer B eine markierte Polymilchsäure, umfassend 10 oder mehr kontinuierliche Milchsäureeinheiten und die Markierungsgruppe als konstituierende Bestandteile, ist.

3. Teilchenförmige molekulare Zusammenstellung nach Anspruch 1, wobei das markierte Polymer B ein markiertes amphiphiles Blockpolymer, umfassend eine hydrophile Blockkette, eine hydrophobe Blockkette mit 10 oder mehr kontinuierlichen Milchsäureeinheiten und die Markierungsgruppe, ist.

4. Teilchenförmige molekulare Zusammenstellung nach einem der Ansprüche 1 bis 3, welche durch ein Herstellungsverfahren, umfassend die Schritte:
Herstellen einer Lösung, in einem Behälter, enthaltend das amphiphile Blockpolymer A1 und das markierte Polymer B in einem organischen Lösungsmittel;
Entfernen des organischen Lösungsmittels aus der Lösung zum Erhalten eines Films, enthaltend das amphiphile Blockpolymer A1 und das markierte Polymer B, auf einer Innenwand des Behälters; und
Zugeben von Wasser oder einer wässrigen Lösung in den Behälter und Ultraschallbehandlung wird durchgeführt zum Umwandeln des Films in eine teilchenförmige molekulare Zusammenstellung zum Erhalten einer Dispersionsflüssigkeit der teilchenförmigen molekularen Zusammenstellung, hergestellt ist.

5. Teilchenförmige molekulare Zusammenstellung nach Anspruch 4, wobei das Herstellungsverfahren weiter, nach dem Schritt des Erhaltens der Dispersionsflüssigkeit der teilchenförmigen molekularen Zusammenstellung, den Schritt, worin die Dispersionsflüssigkeit der teilchenförmigen molekularen Zusammenstellung einer Gefriertrocknungsbehandlung unterzogen wird, umfasst.

6. Teilchenförmige molekulare Zusammenstellung nach Anspruch 1, weiter umfassend:
ein hydrophobes Polymer A2 mit 10 oder mehr Milchsäureeinheiten.

7. Teilchenförmige molekulare Zusammenstellung nach Anspruch 6, wobei das hydrophobe Polymer A2 aus der Gruppe, bestehend aus:
einem hydrophoben Polymer, dessen 10 oder mehr Milchsäureeinheiten aus L-Milchsäureeinheiten aufgebaut sind;
einem hydrophoben Polymer, dessen 10 oder mehr Milchsäureeinheiten aus D-Milchsäureeinheiten aufgebaut sind; und
einem hydrophoben Polymer, dessen 10 oder mehr Milchsäureeinheiten aus L-Milchsäureeinheiten und D-Milchsäureeinheiten aufgebaut sind,
ausgewählt ist.

8. Teilchenförmige molekulare Zusammenstellung nach Anspruch 6 oder 7, wobei das amphiphile Blockpolymer A1 und das hydrophobe Polymer A2 in einem Stoffmengenverhältnis von 10:1 bis 1:10 enthalten sind.

9. Teilchenförmige molekulare Zusammenstellung nach einem der Ansprüche 6 bis 8, welche durch ein Herstellungsverfahren, umfassend die Schritte:
Herstellen einer Lösung in einem Behälter, enthaltend das amphiphile Blockpolymer A1, das hydrophobe Polymer A2 und das markierte Polymer B in einem organischen Lösungsmittel;
Entfernen des organischen Lösungsmittels aus der Lösung zum Erhalten eines Films, enthaltend das amphiphile Blockpolymer A1, das hydrophobe Polymer A2 und das markierte Polymer B, auf einer Innenwand des Behälters; und
Zugeben von Wasser oder einer wässrigen Lösung in den Behälter und Ultraschallbehandeln wird durchgeführt zum Umwandeln des Films in eine teilchenförmige molekulare Zusammenstellung zum Erhalten einer Dispersionsflüssigkeit der teilchenförmigen molekularen Zusammenstellung, hergestellt ist.

10. Teilchenförmige molekulare Zusammenstellung nach Anspruch 9, wobei das Herstellungsverfahren weiter, nach dem Schritt des Erhaltens der Dispersionsflüssigkeit der teilchenförmigen molekularen Zusammenstellung, den Schritt, worin die Dispersionsflüssigkeit der teilchenförmigen molekularen Zusammenstellung einer Gefriertrocknungsbehandlung unterzogen wird, umfasst.

11. Teilchenförmige molekulare Zusammenstellung nach einem der Ansprüche 1 bis 10, welche in der Form einer Mizelle oder eines Vesikels vorliegt.

12. Teilchenförmige molekulare Zusammenstellung nach einem der Ansprüche 1 bis 11, wobei die Signalgruppe eine Nahinfrarot-fluoreszierende Gruppe ist.

13. Teilchenförmige molekulare Zusammenstellung nach einem der Ansprüche 1 bis 11, wobei die Signalgruppe eine Gruppe, enthaltend ein radioaktives Element, ist.

14. Teilchenförmige molekulare Zusammenstellung nach einem der Ansprüche 1 bis 13, weiter umfassend ein Medikament.

15. Molekulare Sonde zur molekularen Bildgebung, umfassend die teilchenförmige molekulare Zusammenstellung nach einem der Ansprüche 1 bis 14.

16. Molekulare Sonde für ein System zur Abgabe eines Medikements, umfassend die teilchenförmige molekulare Zusammenstellung nach Anspruch 14.

17. Verfahren zur Herstellung einer teilchenförmigen molekularen Zusammenstellung, umfassend den Schritt:
Herstellen, durch Verwenden eines amphiphilen Blockpolymers A1, umfassend eine hydrophile Blockkette mit 20 oder mehr Sarcosineinheiten und eine hydrophobe Blockkette mit 10 oder mehr Milchsäureeinheiten, und eines hydrophoben Polymers A2 mit 10 oder mehr Milchsäureeinheiten, einer molekularen Zusammenstellung, umfassend das amphiphile Blockpolymer A1 und das hydrophobe Polymer A2; und
Kontrollieren einer Teilchengröße der molekularen Zusammenstellung durch Einstellen einer Menge des verwendeten hydrophoben Polymers A2, bezogen auf eine Menge des verwendeten amphiphilen Blockpolymers A1.

18. Verfahren zur Herstellung einer teilchenförmigen molekularen Zusammenstellung nach Anspruch 17, wobei das hydrophobe Polymer A2 aus der Gruppe, bestehend aus:
einem hydrophoben Polymer, dessen 10 oder mehr Milchsäureeinheiten aus L-Milchsäureeinheiten aufgebaut sind,
einem hydrophoben Polymer, dessen 10 oder mehr Milchsäureeinheiten aus D-Milchsäureeinheiten aufgebaut sind; und
einem hydrophoben Polymer, dessen 10 oder mehr Milchsäureeinheiten aus L-Milchsäureeinheiten und D-Milchsäureeinheiten aufgebaut sind,
ausgewählt ist.

19. Verfahren zur Herstellung der teilchenförmigen molekularen Zusammenstellung nach Anspruch 17 oder 18, wobei das amphiphile Blockpolymer A1 und das hydrophobe Polymer A2 in einem Stoffmengenverhältnis von 10:1 bis 1:10 verwendet werden.

20. Verfahren zur Herstellung der teilchenförmigen molekularen Zusammenstellung nach einem der Ansprüche 17 bis 19, weiter umfassend das Verwenden eines markierten Polymers B, umfassend mindestens 10 oder mehr Milchsäureeinheiten und eine Markierungsgruppe, zum Herstellen der molekularen Zusammenstellung, umfassend das amphiphile Blockpolymer A1 und das hydrophobe Polymer A2, und zusätzlich das markierte Polymer B, wobei die Markierungsgruppe des Polymers B eine Signalgruppe oder ein Ligand ist,
wobei die Signalgruppe aus der Gruppe, bestehend aus einer Nahinfrarotfluoreszierenden Gruppe, einer Gruppe, enthaltend ein radioaktives Element, und einer magnetischen Gruppe, ausgewählt ist,
wobei der Ligand aus der Gruppe, bestehend aus einem Antikörper, einem Adhäsionsfaktor und einer Tricarbonsäure, welche an ein Übergangsmetall koordinieren kann, ausgewählt ist und
wobei die Markierungsgruppe des Polymers B entweder direkt oder über eine Abstandshaltergruppe an eine beliebige Stelle in dem Polymerteil gebunden ist.

21. Molekulare Sonde nach Anspruch 15 zur Verwendung in einem Verfahren der molekularen Bildgebung eines lebendigen Körpers.

22. Molekulare Sonde nach Anspruch 16 zur Verwendung in einem Verfahren zur Abgabe eines Medikaments an einen lebendigen Körper.

23. Teilchenförmige molekulare Zusammenstellung nach Anspruch 14, wobei das Medikament Paclitaxel ist.

## Revendications

1. Ensemble particulaire moléculaire comprenant les suivants:
un polymère à bloc A1 amphiphile comprenant une chaîne à bloc hydrophile présentant 20 motifs de sarcosine ou plus et une chaîne à bloc hydrophobe présentant 10 motifs d'acide lactique ou plus; et
un polymère B marqué comprenant au moins 10 motifs d'acide lactique ou plus et un groupe de marquage,
dans lequel le groupe de marquage du polymère B est un groupe de signal ou un ligand,
dans lequel le groupe de signal est sélectionné parmi le groupe consistant en un groupe fluorescent dans l'infrarouge proche, un groupe contenant un élément radioactif et un groupe magnétique,
dans lequel le ligand est sélectionné parmi le groupe consistant en un anticorps, un facteur d'adhérence et un acide tricarboxylique qui peut assurer la coordination vers un métal de transition, et
dans lequel le groupe de marquage du polymère B est lié soit directement soit via un groupe espaceur à un site quelconque de la partie de polymère.

2. Ensemble particulaire moléculaire selon la revendication 1, dans lequel le polymère B marqué est un acide polylactique marqué comprenant 10 motifs d'acide lactique continus ou plus et le groupe de marquage comme composants constitutifs.

3. Ensemble particulaire moléculaire selon la revendication 1, dans lequel le polymère B marqué est un polymère à bloc amphiphile marqué comprenant une chaîne à bloc hydrophile, une chaîne à bloc hydrophobe présentant 10 motifs d'acide lactique continus ou plus, et le groupe de marquage.

4. Ensemble particulaire moléculaire selon l'une quelconque des revendications 1 à 3, préparé par un procédé de préparation comprenant les étapes de:
préparation d'une solution, dans un récipient, contenant le polymère à bloc A1 amphiphile et le polymère B marqué dans un solvant organique;
élimination du solvant organique de la solution pour obtenir un film contenant le polymère à bloc A1 amphiphile et le polymère B marqué sur une paroi interne du récipient; et
une addition d'eau ou d'une solution aqueuse dans le récipient et traitement par ultrasons est effectuée pour convertir le film en ensemble particulaire moléculaire pour obtenir un liquide de dispersion de l'ensemble particulaire moléculaire.

5. Ensemble particulaire moléculaire selon la revendication 4, dans lequel le procédé de préparation comprend en outre, après l'étape d'obtention du liquide de dispersion de l'ensemble particulaire moléculaire, l'étape où le liquide de dispersion de l'ensemble particulaire moléculaire est soumis à un traitement de lyophilisation.

6. Ensemble particulaire moléculaire selon la revendication 1, comprenant en outre: un polymère A2 hydrophobe présentant 10 motifs d'acide lactique ou plus.

7. Ensemble particulaire moléculaire selon la revendication 6, dans lequel le polymère A2 hydrophobe est sélectionné parmi le groupe consistant en:
un polymère hydrophobe dont 10 motifs d'acide lactique ou plus sont composés de motifs d'acide L-lactique;
un polymère hydrophobe dont 10 motifs d'acide lactique ou plus sont composés de motifs d'acide D-lactique; et
un polymère hydrophobe dont 10 motifs d'acide lactique ou plus sont composés de motifs d'acide L-lactique et de motifs d'acide D-lactique.

8. Ensemble particulaire moléculaire selon la revendication 6 ou 7, dans lequel le polymère à bloc A1 amphiphile et le polymère A2 hydrophobe sont contenus dans un rapport molaire de 10:1 à 1:10.

9. Ensemble particulaire moléculaire selon l'une quelconque des revendications 6 à 8, préparé par un procédé de préparation comprenant les étapes de:
préparation d'une solution, dans un récipient, contenant le polymère à bloc A1 amphiphile, le polymère A2 hydrophobe et le polymère B marqué dans un solvant organique;
élimination du solvant organique de la solution pour obtenir un film contenant le polymère à bloc A1 amphiphile, le polymère A2 hydrophobe et le polymère B marqué sur une paroi interne du récipient; et
une addition d'eau ou d'une solution aqueuse dans le récipient et traitement par ultrasons est effectuée pour convertir le film en ensemble particulaire moléculaire pour obtenir un liquide de dispersion de l'ensemble particulaire moléculaire.

10. Ensemble particulaire moléculaire selon la revendication 9, dans lequel le procédé de préparation comprend en outre, après l'étape d'obtention du liquide de dispersion de l'ensemble particulaire moléculaire, l'étape où le liquide de dispersion de l'ensemble particulaire moléculaire est soumis à un traitement de lyophilisation.

11. Ensemble particulaire moléculaire selon l'une quelconque des revendications 1 à 10, lequel est sous forme d'une micelle ou d'une vésicule.

12. Ensemble particulaire moléculaire selon l'une quelconque des revendications 1 à 11, dans lequel le groupe de signal est un groupe fluorescent dans l'infrarouge proche.

13. Ensemble particulaire moléculaire selon l'une quelconque des revendications 1 à 11, dans lequel le groupe de signal est un groupe contenant un élément radioactif.

14. Ensemble particulaire moléculaire selon l'une quelconque des revendications 1 à 13, comprenant en outre un médicament.

15. Sonde moléculaire pour imagerie moléculaire comprenant l'ensemble particulaire moléculaire selon l'une quelconque des revendications 1 à 14.

16. Sonde moléculaire pour système d'administration de médicament comprenant comprenant l'ensemble particulaire moléculaire selon la revendication 14.

17. Procédé de préparation d'un ensemble particulaire moléculaire comprenant les étapes de:
préparation, en utilisant un polymère à bloc A1 amphiphile comprenant une chaîne à bloc hydrophile présentant 20 motifs de sarcosine ou plus et une chaîne à bloc hydrophobe présentant 10 motifs d'acide lactique ou plus et un polymère A2 hydrophobe présentant 10 motifs d'acide lactique ou plus, un ensemble moléculaire comprenant le polymère à bloc A1 amphiphile et le polymère A2 hydrophobe; et
le contrôle d'une taille particulaire de l'ensemble moléculaire en réglant une quantité du polymère A2 hydrophobe utilisé sur base d'une quantité du polymère à bloc A1 amphiphile utilisé.

18. Procédé de préparation de l'ensemble particulaire moléculaire selon la revendication 17, dans lequel le polymère A2 hydrophobe est sélectionné parmi le groupe consistant en :
un polymère hydrophobe dont 10 motifs d'acide lactique ou plus sont composés de motifs d'acide L-lactique;
un polymère hydrophobe dont 10 motifs d'acide lactique ou plus sont composés de motifs d'acide D-lactique; et
un polymère hydrophobe dont 10 motifs d'acide lactique ou plus sont composés de motifs d'acide L-lactique et de motifs d'acide D-lactique.

19. Procédé de préparation de l'ensemble particulaire moléculaire selon la revendication 17 ou 18, dans lequel le polymère à bloc A1 amphiphile et le polymère A2 hydrophobe sont utilisés dans un rapport molaire de 10:1 à 1:10.

20. Procédé de préparation de l'ensemble particulaire moléculaire selon l'une quelconque des revendications 17 à 19, comprenant en outre l'utilisation d'un polymère B marqué comprenant au moins 10 motifs d'acide lactique ou plus et un groupe de marquage pour préparer l'ensemble moléculaire comprenant le polymère à bloc A1 amphiphile et le polymère A2 hydrophobe et en outre le polymère B marqué,
dans lequel le groupe de marquage du polymère B est un groupe de signal ou un ligand,
dans lequel le groupe de signal est sélectionné parmi le groupe consistant en un groupe fluorescent dans l'infrarouge proche, un groupe contenant un élément radioactif et un groupe magnétique,
dans lequel le ligand est sélectionné parmi le groupe consistant en un anticorps, un facteur d'adhérence et un acide tricarboxylique qui peut assurer la coordination vers un métal de transition, et
dans lequel le groupe de marquage du polymère B est lié soit directement soit via un groupe espaceur à un site quelconque de la partie de polymère.

21. Sonde moléculaire selon la revendication 15 à utiliser dans un procédé d'imagerie moléculaire d'un corps vivant.

22. Sonde moléculaire selon la revendication 16 à utiliser dans un procédé d'administration d'un médicament à un corps vivant.

23. Ensemble particulaire moléculaire selon la revendication 14, dans lequel le médicament est le paclitaxel.
